# EUROPEAN PATENT APPLICATION

(11) **EP 4 599 844 A1**
(43) Date of publication of application: **13.08.2025**
(21) Application number: 25156984.4
(22) Date of filing: 10.02.2025
(51) Int. Cl.: A61K 39/00

(54) **CLICK CHEMISTRY HLA POLYPEPTIDES**

(30) Priority: 09.02.2024 LU 103243
(71) Applicant: Immuneo Therapeutics GmbH, 14770 Brandenburg an der Havel (DE)
(72) Inventor: SCHÖNHARTING, Wolfgang, 83707 Bad Wiessee (DE); CONTRERAS, Mari Carmen Martos, 81543 München (DE); KUNZ, Meik, 92224 Amberg (DE); NOGUEIRA, Mauro, 13347 Berlin (DE); MANOOCHEHRI, Mehdi, 70193 Stuttgart (DE)
(74) Representative: Gruner, Leopold Joachim

(57) **Abstract**

The present invention relates a click chemistry HLA tumor antigen polypeptide for use as a medicament, in particular for the therapeutic and prophylactic treatment of carcinoma, particularly locally recurrent or metastatic carcinoma. The click chemistry HLA tumor antigen polypeptide comprises at least two HLA tumor antigen peptides (HTAP) corresponding to MHC class I and/or class II complexes, arranged on at least one HLA tumor antigen polypeptide (HTAPP). These HTAP are tumor-specific or tumor-associated and presented on tumor cell membranes. The HLA tumor antigen polypeptide (HTAPP) is provided as a tandem polypeptide or an overlapping tandem polypeptide, both comprising at least 2 HLA tumor antigen peptides corresponding to MHC class I and/or class II complexes.

## Description

### Technical area

The invention pertains to a click chemistry HLA tumor antigen polypeptide for use as a medicament, in particular for the therapeutic and prophylactic treatment of carcinoma, particularly locally recurrent or metastatic carcinoma. The click chemistry HLA tumor antigen polypeptide comprises HLA tumor antigen peptides (HTAP) associated with MHC class I and/or class II complexes, arranged on at least one HLA tumor antigen polypeptide (HTAPP). These HTAP are tumor-specific or tumor-associated and presented on tumor cell membranes. The HLA tumor antigen polypeptide features a scaffold amino acid sequence, with options for extended sequences, high sequence identity to native HLA tumor antigen peptides, or minimal amino acid substitutions. The click chemistry HLA tumor antigen polypeptide (HTAPP) may exist as a tandem polypeptide or an overlapping tandem polypeptide, both comprising at least 2 HLA tumor antigen peptides corresponding to MHC class I and/or class II complexes. This invention offers a versatile approach to treating carcinoma by leveraging HLA tumor antigen peptides and their various polypeptide compositions, enhancing treatment efficacy for recurrent or metastatic carcinoma and further comprises a pharmaceutical composition comprising the click chemistry HLA tumor antigen polypeptides according to the invention, a kit (or parts thereof), a method for determining an pharmaceutical composition comprising HLA tumor antigen polypeptide(s), a method for preparing a formulation according to the invention, and the use of a formulation according to the invention for the preparation of a pharmaceutical composition for the treatment of malignancies, leukemias and neoplasms.

### State of the art

Despite interdisciplinary approaches and exhaustion of classical therapies, cancers remain among the leading causes of death. In general, cancer is treated by established methods such as surgical tumor removal (resection), chemotherapy and/or radiotherapy. In particular, the development of resistance by cancer cells during chemotherapy and/or radiotherapy mostly prevents the complete removal of all cancer cells from a subject's body. Newer therapeutic concepts aim to involve the subject's own immune system in the overall therapeutic concept by using specific measures such as antibody therapy against immunosuppressors and recombinant immunoinformatics (i.e. treatment with information carriers). A prerequisite for the success of such a strategy is the recognition of tumor-specific or tumor-associated antigens or epitopes by the immune system of the subject whose effector functions (by the immune cells) are to be enhanced. Tumor cells differ biologically substantially from their non-malignant cells of origin. These differences are due to genetic alterations acquired during tumor development, which lead, among other things, to the formation of qualitatively or quantitatively altered molecular structures in the tumor cells. If such tumor-associated structures are recognized by the specific immune system of the tumor-bearing host, they are referred to as tumor-associated epitopes.

Cancer/testis antigens (CTAs) refer to a group of tumor-associated proteins expressed by tumors of various histological origins, among others (Fratta et al., Molecular Oncology, 5(2), April 2011, 164-182). In healthy adult vertebrates, expression of these proteins is restricted to male germ cells. However, in cancer, expression of these developmental antigens is often reactivated and thus can serve as a site of immune activation. Many of the CTAs are oncogenes and are involved in cellular processes such as cell growth and division, inhibition of apoptosis, and metastasis. They are often causative for oncogenesis and malignant transformation and are therefore classified as tumor antigens. The expression of CTAs in different malignancies is heterogeneous and often correlates with tumor progression, which is why they also serve as biomarkers for tumor disease progression. HLA antigen peptides of such CTAs are often presented as degradation products on the surface of tumor cells. It is known that such presentation of CTA-derived HLA antigen peptides can be used for the development of new treatment modalities combining drug treatment with anti-CTA-targeted immunotherapy.

Human leukocyte antigens (also called HLA system, HL antigens, histocompatibility antigens, *human leukocyte antigen)* are a group of human genes that are central to the function of the immune system. The HLA system is known as the major histocompatibility complex (MHC) and is found in all vertebrates.

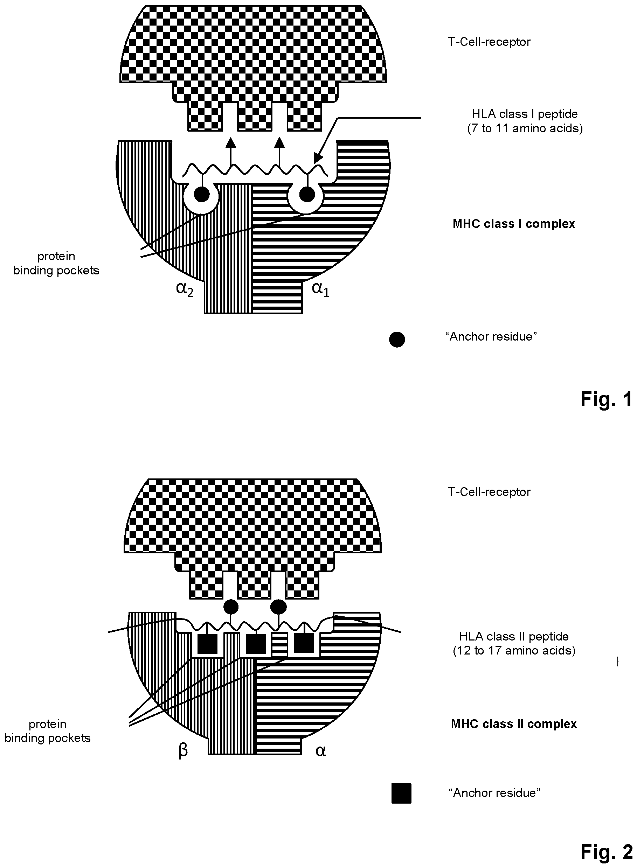

There are two types of MHCs, namely class I MHC molecules (herein also referred to as *"class I MHC complexes"* or "class *I HLA complexes*") and class II MHC molecules (herein also referred to as *"class* II *MHC complexes"* or "class II *HLA complexes*")*.* Both are found on the cell surface of all nucleated cells in the bodies of mandibular vertebrates. MHC molecules consist of two polypeptide chains, an α-chain heavy chain and a β-chain light chain (see also Figs. 1 and 2).

Class I MHC molecules are expressed on the cell surface of all nucleated cells and recognized by CD8+ T cells (also referred to as T killer cells or cytotoxic T cells). Class II MHC molecules are mainly exposed on the cell surface of antigen-presenting cells and recognized by CD4+ T cells (also referred to as T helper cells).

In blood cells, class I MHC molecules are exposed on the cell surface of platelets, but not on red blood cells. Their function is to present peptide fragments of non-self-proteins on their cell surface and transfer them out of the cell into killer T cells (also cytotoxic T cells) to trigger an immediate immune system response against a specific non-self-antigen presented using an MHC class I protein. Because MHC class I molecules present peptides derived from cytosolic proteins, the pathway of presentation of MHC class I molecules is often referred to as the cytosolic or endogenous pathway.

Here, HLA antigen peptides serve as mediators between the corresponding MHC complex presented on the cell surface and the T cell receptor. Class I HLA complexes (HLA-A, B, and C) present intracellular antigen peptide fragments (herein *"HLA antigen peptides corresponding to MHC class I complexes"* or *"type 1 HLA antigen peptides"* comprising 7 to 11, predominantly 9 amino acids - so-called nonamers - in their sequence) towards T killer cells (also cytotoxic T cells), whereas HLA class II complexes (HLA-DR, DQ and DP) present exogenously derived antigen peptides (herein *"HLA antigen peptides corresponding to MHC class II complexes"* or "HLA antigen peptides of *type* 2" comprising more than 11 amino acids, preferably 12 to 17 amino acids in their sequence) towards T helper cells. In humans, HLA antigen peptides of class I, corresponding to MHC class I, are subdivided into HLA-A, HLA-B, and HLA-C antigen peptides.

It is known from the prior art that binding of HLA antigen peptides corresponding to MHC class II complexes to the peptide binding pocket of the corresponding class II HLA complexes occurs primarily via the discrete anchor residues in amino acid positions 1, 4, 6/7, and 9 of the HLA antigen peptides (see, e.g., Sinigaglia and Hammer (1995), J. Exp. Med., 181, 449-451). Particularly preferred according to the prior art is the interaction between the peptide binding pocket motif of the class II HLA complex and the discrete anchor residues in amino acid positions 6 and 9 of the HLA antigen peptides corresponding to MHC class II complexes. The amino acid residues in amino acid positions 2, 3, 5, 7 and 8 of the respective HLA antigen peptides are thereby available for interaction with the T-cell receptor (Sant' Angelo et al. (2002), Recognition of core and flanking amino acids of MHC class II-bound peptides by the T-cell receptor, Eur J Immunol, 32(9), 2510-20).

In contrast, for HLA antigen peptides corresponding to MHC class I complexes, the amino acid positions 1, 2, and 9 have been postulated as the primary anchor residues relative to the corresponding class I HLA complex (see, for example, Binkowski et al. (2012), PLoS ONE, 7(8), e41710; Yamada (1999), Tissue Antigens, 54(4), 325-32).

A new study in the USA is currently investigating the specific efficacy of only one HLA antigen peptide in the treatment of cancer (see WO 2013/135266, Inderberg-Suso et al. (2012), Oncoimmunology., 1(5), 670-686 and Slingluff (2011), Cancer J., 17(5), 343-350). Therefore, the therapeutic spectrum is very limited and by applying only one HLA antigen peptide, the efficacy is questionable.

This is particularly true in a tumor entity such as carcinoma, which is characterized by low homogeneity (i.e., high heterogeneity) and lack of initial immunogenicity compared to other tumor entities. Clinically effective targeted immunoinformation and stimulation therapies therefore rely on a multifactorial information approach.

A major disadvantage of cancer immunotherapies is therefore that they are based on the fact that the individual mutation pattern (signatures) of the tumor of each cancer subject must be decoded. Synthetic vaccines, for example RNA-based vaccines, are then produced to match the determined profile of the mutation pattern for each individual subject (so-called vaccine production). The vaccines obtained in this way can then only be used for the individual treatment of this particular subject.

In principle, these novel vaccines in cancer immunotherapy are therefore not suitable for other subjects with the same tumor but can only be used for a single subject whose mutanoma was previously analyzed for vaccine production.

WO 2016/187508 A2 discloses a pharmaceutical composition comprising a plurality of neoantigenic peptides and a pharmaceutically acceptable carrier, wherein each neoantigenic peptide comprises a tumor-specific neoepitope capable of binding to an HLA protein in a subject and comprises a tumor-specific mutation present in a subject. In addition, the composition comprises neoantigenic peptides comprising tumor-specific mutations present in a subset of subjects in a population of subjects suffering from cancer. Disadvantageously, it is only revealed here that a small percentage of the population, namely 5%, can be covered and furthermore no statement is made as to how different amino acid mutations in related genes can be covered. Furthermore, only single HLA antigen peptides are administered, not tandem or overlapping HLA tumor antigen polypeptides.

The patent specification WO 03100432 A2 describes a method for identifying immunoreactive peptides, particularly in the context of tumor-related diseases. The method involves several key steps: (a) obtaining samples from both tumor tissue and corresponding healthy tissue, (b) determining the tumor-specific expression profile, (c) isolating and analyzing antigenic peptides from the tumor tissue sample, (d) comparing the data from steps (b) and (c), and (e) identifying peptides based on matched data. The antigenic peptides are identified as MHC ligands, and various analytical techniques such as microarray analysis, reverse transcriptase-polymerase chain reaction, and mass spectrometry are used in the process. On the downside, this approach does not provide guidance on how to screen for presented peptides on the tumor cell surface and does not disclose an approach to treat a group of subjects with different mutations or a method to combine different antigenic peptides on a single immunogenic HLA tumor antigen polypeptide.

The specification WO 2019099440 A1 describes an alternative approach to immunogenic cancer therapy utilizing multifunctional chimeric antigen receptor (CAR)-based compositions. These compositions are designed to direct immune responses, particularly against hyperproliferative disorders like cancer. The key innovation involves the incorporation of an Adapter, which grants the capacity to modulate and redirect CAR cell-mediated immune responses both in laboratory settings (in vitro) and within the body (in vivo). Notably, certain embodiments include genetic modifications in CAR cells to suppress or eliminate the expression of specific antigenic determinants. While this approach offers promise for cancer treatment, it also has potential drawbacks such as cytokine release syndrome (CRS), potentially life-threatening uncontrolled release of cytokines, off-target effects, limited target antigens and antigens expressed on the surface of CAR-T cells, and difficulties in providing a general group therapy approach.

Specification WO2022258794A2 discloses a conjugate of a sequence of a B cell epitope and at least one polypeptide comprising a sequence of a CD4+ T cell epitope, wherein the CD4+ T cell epitope comprises a region of at least 12 amino acids, by linkage via orthogonal groups. However, this specification does not teach or suggest a modular approach to combining different tumor-specific HLA tumor antigen peptides into a polypeptide that can be used in a medicament for the personalized treatment or prophylaxis of cancer.

In summary, there is a current need in cancer immunotherapy, particularly in terms of generalization to subject populations that share the same cancer type and HLA alleles, and to induce a more robust, potent, and reliable immune response with one composition. To date, there are no approaches in the prior art to efficiently solve such a problem.

### Objection of the Invention / Technical Problem

Thus, it is an object of the present invention - in contrast to fully individualized cancer immunotherapy - to provide a pharmaceutical composition for different subjects or a specifically predetermined group of subjects (i.e., for a specific group of subjects with at least one identical HLA allele) who have overlap in the mutanoma of the malignant or neoplastic tissue (carcinoma) (derivation of subject groups). Particularly preferred is a solution for a group of subjects who have the same mutanoma or at least a single amino acid mutation of a gene associated with the same mutanoma, a uniform composition approach comprising HLA antigen peptides is sought.

It is therefore an object of the present invention to provide pharmacologically active agents, as well as pharmaceutical compositions comprising such active agents, which can be used for the diagnosis, prevention and/or treatment of cancer and other diseases and disorders listed herein; and to provide methods for the diagnosis, prevention and/or treatment of such cancers involving the administration and/or use of such agents and compositions.

In particular, it is an object of the invention to provide such pharmacologically active agents, pharmaceutical compositions and/or methods that have certain advantages over the active agents, compositions and/or methods currently in use and/or known in the prior art These advantages result from the following further description.

In particular, it is an object of the invention to provide therapeutically active HLA tumor antigen peptides (HTAP) corresponding to MHC class I and/or class II complexes, arranged on at least one HLA tumor antigen polypeptide(s) (HTAPP), wherein the HLA tumor antigen peptides (HTAP) are tumor-exclusive or tumor-associated and presented on the cell membrane of the associated tumor cell, which can be used as pharmacologically active HLA tumor antigen peptides or as pharmacologically active agents, and to provide pharmaceutical compositions containing same, for the diagnosis, prevention and/or treatment of carcinomas and other diseases and disorders, in particular cancers, as set forth herein; and to provide methods for the diagnosis, prevention and/or treatment of such diseases and disorders involving the administration and/or use of such therapeutically active HLA tumor antigen peptides and compositions.

In particular, it is a specific task of the present invention to provide such HLA tumor antigen peptides suitable for prophylactic, therapeutic and/or diagnostic use in warm-blooded animals, especially in a mammal and most particularly in a human. Another obstacle that has not been addressed in the current state of the art is that amino acid mutations can occur in the same cancers and even in the same genes, but at different sites.

### Solution

To overcome the technical problems of the prior art, a preferred embodiment of the present invention comprises a pharmaceutical composition for use as a medicament, in particular for use in the therapeutic and/or prophylactic treatment of a carcinoma, particularly preferably a locally recurrent or metastatic carcinoma, in particular in a subject or group of subjects suffering from or at risk of suffering from a carcinoma, comprising a pharmacologically effective amount comprising 2 to 25 HLA tumor antigen peptides (HTAP) corresponding to MHC class I and/or class II complexes, arranged on at least one HLA tumor antigen polypeptide(s) (HTAPP), wherein the HLA tumor antigen peptides (HTAP) are tumor-exclusive or tumor-associated and presented on the cell membrane of the associated tumor cell and correspond to an amino acid sequence of a transcribed mutant gene, wherein the HLA tumor antigen polypeptide comprises a scaffold amino acid sequence, also referred to as amino acid sequence, preferably between 15 and 45, more preferably between 17 and 40 amino acids,
a) wherein the scaffold amino acid sequence comprises, in addition to HLA tumor antigen peptide(s), up to 1 to 30 amino acids (long HLA tumor antigen polypeptide); and/or
b) wherein the scaffold amino acid sequence comprises an HLA tumor antigen peptide(s) with at least 90% sequence identity similarity to the native HLA tumor antigen peptide (similarity HLA tumor antigen polypeptide); and/or
c) wherein at least one of the amino acid sequences comprises an HLA tumor antigen peptide having an amino acid sequence comprising or consisting essentially of only one amino acid substitution relative to the amino acid sequence of the native HLA tumor antigen peptide (substitution HLA tumor antigen polypeptide),
wherein an HLA tumor antigen polypeptide is a tandem polypeptide comprising or consisting of at least 2 HLA tumor antigen peptides corresponding to MHC class I and/or class II complexes; and/or wherein an HLA tumor antigen polypeptide is an overlapping tandem polypeptide comprising or consisting of at least 2 HLA tumor antigen peptides corresponding to MHC class I and/or class II complexes which overlap in their amino acid sequence.

The problems technical problems of the prior art are especially overcome by a click chemistry HLA tumor antigen polypeptide, preferably as a medicament, or particularly for use in a medicament, in particular in particular for use in the therapeutic and/or prophylactic treatment of a carcinoma, particularly preferably a locally recurrent or metastatic carcinoma, in particular in a subject or group of subjects suffering from or at risk of suffering from a carcinoma, corresponding to at least two HLA tumor antigen peptides (HTAP), preferably between 2 and 25, corresponding to MHC class I and/or class II complexes, arranged on a HLA tumor antigen polypeptide (HTAPP), wherein the HLA tumor antigen peptides (HTAP) are tumor-exclusive or tumor-associated and presented on the cell membrane of the associated tumor cell and correspond to an amino acid sequence of a transcribed mutant gene, wherein the HLA tumor antigen polypeptide comprises an amino acid sequence, also referred to as backbone amino acid sequence, which is a sequence of amino acids connected chemically by peptide bonds and/or click chemistry groups, wherein the amino acid sequence has a length of preferably between 15 and 45 amino acids,
a) wherein the amino acid sequence comprises, in addition to HLA tumor antigen peptide(s), in particular in addition to HLA antigen tumor peptides amino acid sequences, up to 1 to 30 amino acids (long HLA tumor antigen polypeptide); and/or
b) wherein the amino acid sequence comprises an HLA tumor antigen peptide(s), in particular a HLA antigen tumor peptides amino acid sequence, with at least 90 % sequence identity similarity to the native HLA tumor antigen peptide (similarity HLA tumor antigen polypeptide); and/or
c) wherein the amino acid sequence comprises an HLA tumor antigen peptide having an amino acid sequence comprising of only one amino acid substitution relative to the amino acid sequence of the native HLA tumor antigen peptide (substitution HLA tumor antigen polypeptide),
wherein
i) an HLA tumor antigen polypeptide is a tandem polypeptide comprising at least 2 HLA tumor antigen peptides, preferably between 2 to 25 **HTAPs,** corresponding to MHC class I and/or class II complexes; and/or
ii) an HLA tumor antigen polypeptide is an overlapping tandem polypeptide comprising at least 2 HLA tumor antigen peptides, preferably between 2 and 25 HTAPs, corresponding to MHC class I and/or class II complexes which overlap in their amino acid sequence,
wherein the **HTAPP** is provided as a first and a second segment wherein
- the first segment comprises a first click chemistry group bound to the first segment at the C-terminus, and
- the second segment comprises a second click chemistry group bound to the second segment at the N-terminus, wherein the click chemistry HLA tumor antigen polypeptide is provided by a click chemistry reaction.

The solution of the task further includes a method of determining a pharmaceutical composition comprising or consisting of HLA tumor antigen polypeptides according to the current invention.

According to the invention, these tasks are fulfilled by a pharmaceutical composition for use in the treatment or profilaxis of carcinomas, in particular locally recurrent or metastatic carcinomas in a subject or group of subjects suffering or suspected of suffering from carcinomas, in particular wherein the composition comprises HLA tumor antigen peptides selected from the group consisting of SEQ-ID-Nos.: 1 to 17 and/or 18 to 31 and/or 32 to 37 and/or 40 to 68. Further advantageous embodiments are given in the subclaims.

Another obstacle with the group approach to cancer immunotherapy is that the patent mutations may be in the same cancers and even in the same genes, but in different locations. An approach that has not yet been pursued in this regard is to present multimerized polypeptides on which several peptides corresponding to HLA complexes are arranged, which advantageously cover different mutated genes simultaneously. By introducing more and more mutanomes from different subjects into a bioinformatic pipeline with feedback, increasingly precise HLA tumor antigen polypeptides (HTAPP) comprising many different mutated gene segments expressed and corresponding to widespread HLA alleles can be generated and successively improved. Thus, a pharmaceutical composition of preferably 5 to 12 HTAPPs can be formed, which match at least one mutated gene and one HLA allele of a group of subjects suffering or at risk from suffering from cancer. Preferably, each single HTAPP already covers different combinations of the most frequently mutated gene segments and HLA peptides. By arranging a carefully selected number of HLA tumor antigen peptides on HLA tumor antigen polypeptides, , the exact mixture of which is tailored to the HLA alleles of the specific subject, an effective single therapy can be formed from a manageable number of HTAPPs.

In some preferred embodiments, the invention is also solved by providing an HLA tumor antigen polypeptide comprising a delivery aiding capping peptide (DACP) and by providing a click chemistry HLA tumor antigen polypeptide. The advantages of these specific embodiments of a HTAPP comprised by the pharmaceutical composition may be used interchangeably for the pharmaceutical composition and their embodiments are always also embodiments of the comprised HTAPPs and their specific variations, the HTAPP-DACP and the click chemistry HTAPPs and HTAPP-DACPs. Since the solution to the problem according to the invention always involves the provision of a HTAPP as described herein, the properties and various advantageous embodiments of the pharmaceutical composition are also disclosed for the HTAPPs comprised therein, in particular the specific embodiments of the HTAPPs comprised therein, since the effect of these is inherently linked to the composition.

### Description

According to a particularly preferred embodiment of the present invention, the pharmaceutical composition consists exclusively of a carrier liquid (preferably water, a pharmaceutically acceptable saline solution and/or pharmaceutically DMSO - such compositions of carrier liquids are known to the skilled person and are, for example, in the range around 30% DMSO and 70% DMSO). in the range of 30% DMSO and 70% water), in which a pharmacologically effective amount of 4 to 8 HLA-A tumor antigen peptides corresponding to the MHC class I complexes and 2 tumor antigen peptides corresponding to the MHC class II complexes are dissolved or suspended, and an adjuvant.

Pharmaceutical composition and suitable dosage forms for application of the pharmaceutically active HLA tumor antigen peptides are prepared according to standard procedures known in the prior art and are readily applicable to any new or improved process for their preparation.

Particularly advantageously, administering the aforementioned combination of a pharmacologically effective amount of the HLA tumor antigen peptides to the subject or group of subjects suffering from carcinoma effectively reduces the CA 15-3 level. CA 15-3 (Cancer antigen 15-3) is a so-called glycoprotein used as a specific tumor marker in carcinoma. The CA 15-3 value is a laboratory value that rises significantly above the threshold value in certain cancers, especially carcinomas. In healthy individuals, the CA 15-3 threshold is below 31 enzyme units per milliliter (< 31 U/ml). Preferably, administering the pharmacologically effective amount of tumor antigen peptides to the subject or group of subjects suffering from carcinoma reduces the CA 15-3 value below 60 U/ml, more preferably below 50 U/ml, most preferably to a range below 40 U/ml and a normal value (< 31 U/ml).

Particularly preferably, administering the pharmacologically effective amount of HLA tumor antigen peptides to the subject or group of subjects thus effectively prolongs the progression-free survival of the individual, preferably by at least 2 to 5 years.

Contrary to conventional approaches, it is a surprising finding of the present invention that tumor antigen peptides having a K_{D}-value in the range between 50 and 500 nM trigger effector cells (i.e., cytotoxic T cells), contrary to conventional *in silico binding prediction models that* have considered them to have low binding Rather, it is of particular advantage for the efficacy of the tumor antigen peptides that the HLA tumor antigen peptides used for treatment are immunogenic in the subject or group of subjects with at least one identical HLA allele, which can be determined in advance with an immunogenicity assay (e.g., by Western blot, ELISA techniques, especially by ELISPOT, preferably via interferon-gamma, interferon-alpha or interleukin (IL-2), or immunodetection with microscopic analysis).

As described herein, but without limitation to any explanation, mechanism of action, or hypothesis in the present invention, two distinct classes of HTAPPs corresponding to amino acid sequences of the invention have been determined based on their ability to enhance the interaction of class I MHC complexes and class II MHC complexes, respectively, with at least one T cell receptor (particularly in the detection method described below in embodiment 3).

These two classes of amino acid sequences of the invention are (as described below):
- "HLA tumor antigen polypeptides (HTAPP) corresponding to MHC class I and II complexes": (see particularly preferred examples in Table 1).
- "HLA tumor antigen polypeptides (HTAPP) comprising delivering aiding capping peptides (DACP) corresponding to MHC class II complexes": (see particularly preferred examples in Tables 2 to 5).

Advantageously, the use/application of the pharmaceutical composition according to the invention or the specific combination of tumor antigen peptides contained therein corresponding to the MHC class I complexes and MHC class II complexes to the subject or group of subjects with at least one identical HLA allele is not merely a passive immunization (as in the case of treatment with antibodies, e.g. Herceptin) but an active immunization (i.e. specific activation of the T cells or B helper cells via information carriers). Because of the specific activation of MHC class II molecules, which are mainly exposed on the cell surface of antigen-presenting cells, by means of the tumor antigen peptides corresponding to the MHC class II complexes, CD4+ T cells (also called T helper cells) and B cells are specifically activated.

For binding to a T cell receptor, an HLA tumor antigen peptide of the invention typically has in its amino acid sequence one or more amino acid residues or one or more segments of amino acid residues (i.e., with each "segment" comprising two or more amino acid residues located adjacent to or in close proximity to each other, i.e. in the primary or tertiary structure of the amino acid sequence) through which the amino acid sequence of the invention can bind to a T cell receptor (in particular a binding pocket thereof), the amino acid residues or portions of the amino acid residues thus forming the "anchor" for binding to a T cell receptor (also referred to herein as "anchor amino acids").

The determination of this "anchor" can be determined, for example, by *in silico* methods (e.g., the artificial neural network NNAlign used in the publicly available NetMHC-4.0) or by targeted mutation (insertions or substitutions in the amino acid sequence of HLA tumor antigen peptides).

The HLA tumor antigen peptides provided by the present invention are preferably in substantially isolated form (as defined herein) or form part of a protein or polypeptide, which may comprise or consist essentially of one or more HLA tumor antigen peptides of the invention, and which may optionally further comprise one or more pharmaceutically active HLA tumor antigen peptide(s) (all of which are optionally joined via one or more linkers in a so-called oligopeptide). For example, and without limitation, the tumor antigen peptides of the invention may be used as a binding moiety in such a protein or polypeptide, which may optionally include one or more additional amino acid sequences that may serve as a binding moiety (i.e., against one or more targets other than a T cell receptor) to provide a monovalent, multivalent, or multispecific polypeptide of the invention as described herein, respectively. Such protein or polypeptide may also be in substantially isolated form (as defined herein).

A pharmaceutical composition comprising a specific combination of HLA tumor antigen peptides corresponding to MHC class I complexes and HLA tumor antigen peptides corresponding to MHC class II complexes disclosed herein has been shown to be particularly advantageous as it is capable of specifically activating T cells as well as specifically activating B cells.

In some embodiments, the present invention also relates to a pharmaceutical composition, a kit (or parts thereof), a method for determining/identifying a pharmacologically active HLA tumor antigen peptide corresponding to class I and/or class II MHC complexes, a method for preparing a formulation according to the invention, and the use of a formulation according to the invention for the preparation of a pharmaceutical composition for the treatment of cancer, in particular /mammary carcinoma.

In particular, the polypeptides and pharmaceutical compositions of the present invention may be used for the prevention and treatment of cancers, particularly / cancers, characterized by mutation(s) (herein "amino acid substitutions") altered wild-type HLA antigen peptides corresponding to MHC class I complexes and/or corresponding to MHC class II complexes (so-called HLA tumor antigen peptides, as defined herein).

In general, the treatment of cancer of the invention of a subject or group of subjects is (are) carried out in advance by classical methods such as chemotherapy, radiotherapy and/or cancer immunotherapy.

Classical methods for the treatment of cancers of the invention are, for example, surgical tumor removal (resection), chemotherapy and/or radiation therapy, with two or even all three treatment methods frequently being applied simultaneously to a subject. Cancer immunotherapy methods are divided into active and passive immunization. In active immunization, the subject is administered substances that are intended to trigger an immune response in his or her immune system. In passive immunization, antibodies or antibody fragments are used. In adoptive immunotherapy (i.e. passive immunotherapy, as comparable to antibody treatment without direct immunomodulation), leukocytes are removed from the subject, cultured *ex vivo,* and then re-injected into the subject. If the treatment does not destroy all cells of the tumor and its metastases, further treatment of cancer is significantly hampered by the development of resistance.

Thus, the present invention also comprises a pharmaceutical composition for the treatment of cancers, as described above, following the classical methods for the treatment of cancers, namely after unsuccessful surgical tumor removal (resection), chemotherapy and/or radiotherapy.

It is a particular achievement of the inventors to have found that, for effective treatment, the HLA-A antigen peptides used according to the invention are actually presented on the cell surface of cells of the malignant tissue (in particular carcinoma) to be treated in the individual to be treated. Therefore, the pharmaceutical composition to be applied comprises HLA tumor antigen peptides presented on the surface of tumor cells of the subject's or group of subjects' carcinoma, as determined by ultra-high performance liquid chromatography (UHPCL) in conjunction with ESI mass spectrometry (MS) prior to application and assembly of the pharmaceutical composition. By such ligandome assay, unlike conventional cancer immunotherapies, the binding ability of the HLA-A antigen peptides according to the invention towards the corresponding class I and/or class II MHC complex has already been proven in advance.

According to a preferred embodiment of the invention, at least 60%, preferably at least 80%, most preferably 90%, ideally all of the HLA tumor antigen peptides contained in the pharmaceutical composition to be applied are presented on the surface of the tumor cells of the subject's or group of subjects' carcinoma as determined by methods as described herein.

It has been found by the inventors of the present invention that the HLA-A antigen peptides explicitly disclosed herein corresponding to MHC class I are particularly suitable for use in the treatment of carcinomas in subjects or a group of subjects having at least one identical HLA allele exhibiting subtype A*01 and/or A*02. That is, the HLA-A tumor antigen peptides used herein preferably bind to the corresponding MHC class I complex of subtype A*01 and/or A*02.

According to a particularly preferred embodiment, the individuals (subjects or a group of subjects) have the haplotype with the subgroup A*01:01, A*02:01, A*02:03, A*02:06, A*02:786, A*03:01, A*11:01, A*24:02, A*30:01, A*30:02, A*31:01, A*32:01, A*33:01, A*68:01, A*68:02, B*07:02, B*08:01, B*15:01, B*35:01, B*40:01, B*44:02, B*57:01, B*57:37, B*58:01, C*03:04, C*04:01, C*06:02, C*07:02, E*01:01, and E*01:03.

Particularly, according to a particularly preferred embodiment, the individuals (subjects or a group of subjects) have the haplotype with the subgroup DQA1*01:01, DQB*102:01, DQB1 *05:01, DQB1*06:02, DRB1*01:01, DRB1*15:01.

It is true that the pharmaceutical composition according to the invention could be used for the tissue-independent treatment of malignancies, neoplasms, and/or leukemias (i.e., unlike conventional preparations, the cancer subtypes do not initially play a role), so that the pharmaceutical composition can be applied across tissues for use as an anticancer drug. However, it was found that the administration/application of the specific combination of 2 to 25 HLA tumor antigen peptides corresponding to MHC class I complexes arranged on at least one HLA tumor antigen polypeptide according to the invention can be used particularly advantageously for the treatment of cancer.

According to a particularly preferred embodiment of the present invention, at least one HLA-A antigen peptide of the composition is a tumor-exclusive HLA-A antigen peptide (i.e., a cancer-testis antigen (CTA) that does not (no longer) appear beyond the immunoprivileged spermatocytes in the healthy/normal tissue of the subject/group of subjects, or a so-called neoantigen peptide), and wherein specific binding of the tumor-exclusive HLA-A antigen peptide occurs with a specific dissociation (K _{D}) in the range of 10 to 50 nM, as determined by surface plasmon resonance.

It is also an outstanding achievement of the inventors to have discovered that a pharmaceutical composition based on HLA antigen peptides is particularly effective when both T lymphocytes (T cells for short) and B lymphocytes (B cells for short) are activated by them.

T cells belong to the lymphocyte cell group and play an important role in the human immune system. T cells recognize antigens via a specific receptor, the so-called T cell receptor (TCR). However, for this to happen, the antigen must be offered by an antigen-presenting cell (APC).

Stable binding of the T cell to the antigen-presenting cell requires the participation of so-called auxiliary proteins. These include CD4 and CD8 (CD = "Cluster of Differentiation").

T cells carrying the CD4 trait are also called CD4-positive T cells or T helper cells. In normal adult blood, CD4+ T cells account for 27-57% of lymphocytes, or approximately 310-1570 cells/µl.

The group of CD8-positive (CD8+) T cells, which also includes regulatory T cells, contains the cytotoxic T cells or T killer cells. They play a special role in killing the body's own cells that are infected by viruses.

This property of cellular immune defense is critical in the present invention, as the molecular and genetic alterations of tumor cells can be recognized and lysed by this T cell population.

In contrast, B cells are the only cells capable of producing antibodies and, together with T cells, make up the crucial component of the adaptive immune system. While T cells are involved in the cell-mediated immune response, B cells are the carriers of the humoral immune response (and responsible for the formation of antibodies).

A pharmaceutical composition has been shown to comprise the tumor-exclusive or tumor-associated HLA antigen peptides whose expression level in the tumor cells is at least three times higher than in the healthy cells of the subject or the specifically determined group of subjects having at least one identical HLA allele, as determined for example by qPCR, and wherein the tumor-associated HLA tumor antigen peptides are associated with proliferation, invasiveness, angiogenesis and an increase in cytokeratin production of the carcinoma, have particularly effective pharmacological effects in the treatment of carcinomas.

Particularly preferably, each individual HLA tumor antigen polypeptide used herein for the treatment of carcinoma is present in the pharmaceutical composition at an absolute concentration (i.e., administration dose) of at least from 100 to 600 *µg,* preferably from 300 to 600 *µg.*

In the meantime, it has been shown in further experiments that pharmaceutical compositions containing an absolute concentration of > 600 µg per HLA tumor antigen peptide and/or HLA tumor antigen polypeptide, i.e. at least 700 to 1,200 *µg,* preferably from 800 to 1,200 *µg, are* particularly preferred, as this greatly intensifies the information (the activation and/or training of the immune system). This is particularly advantageous if the immune system of the subject to be treated is already weakened by pre-treatment with a standard therapy procedure (e.g. at least one operation, radiation, chemotherapy and/or hormone therapy). In addition, the absolute concentration per HLA tumor antigen peptide is preferred, as this substantially minimizes the influence of degradation of the HLA tumor antigen peptides (e.g., by ligases) after their application to the subject.

It is highly convenient that the pharmaceutical composition comprises an adjuvant which, when the composition is applied to a subject, is capable of forming a granuloma at the site of application. The advantage in the formation of a granuloma is that a depot effect can thus be achieved, whereby the HLA tumor antigen peptides are advantageously stored at the site of application in the manner of a reservoir and can be delivered to the organism of the subject over a longer period of time. Particularly advantageously, therefore, weekly applications of the pharmaceutical composition according to the invention are omitted. Preferably, the application of the pharmaceutical composition for the treatment of cancer diseases within the meaning of the invention, when used over a longer period of time, therefore only has to be carried out every 2 weeks, particularly preferably only once a month.

In this regard, the pharmaceutical composition is preferably applied subcutaneously or intradermally and, preferably substantially simultaneously at least 2, more preferably at least 3 application sites, typically at 3 to 4 application sites remote from a tumor lesion and/or the cancerous lymph node area.

The essentially simultaneous (successive) application at several application sites has the advantage that, particularly in the case of application of high absolute concentrations of the individual HLA tumor antigen polypeptides (i.e. administration dose) in the range of > 600 *µg, which* require larger application volumes (> 1 mL) for complete dissolution of the individual HLA tumor antigen polypeptides, the application of the application solution, which has only a limited shelf life after opening, is carried out as simultaneously as possible.

Preferably, the pharmaceutical composition comprising each individual HLA tumor antigen polypeptide in the composition at an absolute concentration (i.e., administration dose) of 300 to 600 µg need only be administered intradermally or subcutaneously once every 2 weeks, preferably once every 4 weeks, for a period of at least one year to a subject or a specifically identified group of subjects having at least one identical HLA allele.

It has been found that a pharmaceutical composition in which at least one HLA tumor antigen peptide has at least one mutation with respect to the wild-type HLA tumor antigen peptide (as detailed below) that results in an increase, preferably to < 500 nm, most preferably < 50 nm of the specific binding affinity to the T cell receptor (K_{D} -value) of the individual treated with HLA tumor antigen peptide compared to the wild type of HLA tumor antigen polypeptide, show particularly beneficial effects in the treatment of carcinomas.

According to a preferred embodiment of the present invention, the pharmaceutical composition is used for the treatment of cancer as monotherapy or in combination with other known therapies and/or compounds for the treatment of cancer. In this regard, the pharmaceutical composition will be administered as a first-line therapy to the subject or group of subjects with at least one identical HLA allele (so-called adjuvant monotherapy).

Alternatively, it may be provided that the subject or group of subjects to be treated with the pharmaceutical composition with at least one identical HLA allele has already received at least one standard therapy procedure (e.g., at least one surgery, radiation, chemotherapy, and/or hormone therapy) in advance.

Most preferably, the cancer to be treated is a hormone positive, HER2/neu or triple negative cancer.

### HLA tumor antigen peptide (HTAP)

The present invention also includes HLA tumor antigen peptides corresponding to MHC class I complexes or MHC class II complexes, particularly for use in treating or profiling carcinoma in a subject or group of subjects suffering or suspected of suffering from carcinoma, suffering therefrom, or for a pharmaceutical composition according to the invention, wherein the HLA tumor antigen peptides are located on an HLA tumor antigen polypeptide and thus comprise or represent a portion of amino acid sequences selected from the group consisting of those shown in SEQ-ID-Nos.: 1 to 37, 40 to 68, each representing an HLA tumor antigen polypeptide according to the invention, or having at least one mutation, preferably an amino acid substitution, with respect to one of the said amino acid sequences, or comprising a portion of the amino acid sequence 80, 81, 82, 83, 84, 85, 86, 87, 88, 89, 90, 91, 92, 93, 94, 95, 96, 97, 98 or 99% identical to SEQ-ID-Nos.: 1 to 37, 40 to 68.

Particularly preferred in this regard is the use of the foregoing HLA tumor antigen peptides in a method of treating carcinomas, particularly locally recurrent or metastatic carcinomas in a subject or group of subjects having at least one identical HLA allele, the method comprising administering/applying to the subject or group of subjects a treatment regimen comprising a pharmacologically effective amount of at least one of the foregoing HLA tumor antigen peptides.

In order for the immune system of the individual to whom the HLA tumor antigen peptides or pharmaceutical composition according to the invention is administered to still be fully functional and thus easier to train, it is advantageous if the individual has not yet received radiation, chemotherapy, and/or hormone therapy against the cancer, especially locally recurrent or metastatic cancer, and/or has not received prior adjuvant chemotherapy in recurrence for 12 months or less since the last dose of a chemotherapeutic agent.

Particularly preferably, the treatment regimen described herein, especially using at least one aforementioned HLA tumor antigen peptide of the invention, effectively prolongs progression-free survival of the individual.

### HLA-A antigen peptides and HLA-A neoantigen peptides

An "HLA-A tumor antigen peptide corresponding to MHC class I complexes" is defined herein as an "HLA tumor antigen peptide of the invention" or "amino acid sequence of the invention" (as defined herein) comprising:
a) an amino acid sequence consisting of 7 to 11 amino acids; and/or
b) a neoantigen peptide having an amino acid sequence consisting of 7 to 11 amino acids, which is
   i) is analogous to at least one HLA-A antigen peptide exposed on the cell surface of cells from malignant and/or neoplastic tissue (in particular carcinoma) of the individual to be treated, and
   ii) in its amino acid sequence at least one amino acid exchange with the wild type of this HLA-A neoantigen peptide (so-called *"HLA-A neoantigen peptide")* and
   iii) has at least a 3-fold increased specific affinity towards the T cell receptor of endogenous T cells

It is an outstanding achievement of the inventors to have recognized that HLA-A neoantigen peptides which in their amino acid sequence compared to the wild type of this HLA-A antigen peptide have at least one amino acid exchange in the amino acid positions 1 (N-terminus), 2, 7/8 and/or the C-terminus, have an at least 4-fold, particularly preferably at least 5-fold, most preferably at least 8-fold increased specific affinity (K_{D} ) towards the T cell receptor of the endogenous T cells and are therefore particularly preferably used for the composition according to the invention.

Particularly preferably, the amino acid exchange in the amino acid sequence of the HLA-A antigen peptide is a single amino acid exchange in amino acid position 1 (N-terminus), 2, 7/8 or the C-terminus.

Preferably, the amino acid exchange in the amino acid sequence of the HLA-A neoantigen relative to the wild type of this HLA-A antigen peptide is a C/Y, A/V, D/Y, E/K, P/L, N/D, or T/M exchange

Preferably, the HLA-A peptide or HLA-A neoantigen has a specific activity (K _{D}) towards the T cell receptor, as determined by any suitable detection method known to the skilled person in the prior art, of less than 100 nM, more preferably less than 75 nM or most preferably less than 50 nM, such as less than 40 nM, 35 nM, 30 nM, 25 nM or 20 nM.

Preferably, the HLA-A tumor antigen peptide or the HLA-A neoantigen is present in the pharmaceutical composition according to the invention at a concentration, as defined above, of at least 100 to 600 *µg,* alternatively preferably at an absolute concentration of > 600 µg relative to the volume of the pharmaceutical composition to be applied.

Preferably, the HLA-A tumor antigen peptides or HLA-A neoantigens are selected as defined in (b)above.

The use of compounds, constructs, proteins or polypeptides as defined according to the invention, which consist of at least two identical or different peptide sequences of HLA tumor antigen peptides and/or HLA neoantigens, has the further advantage that the longer amino acid sequences of the compounds, constructs, proteins or polypeptides result in a longer retention time in the tissue of the subject after application, whereby the compounds, constructs, proteins or polypeptides after application to the subject can be broken down, for example, by the body's own enzymes into smaller fragments (pharmaceutically active form comprising at least 7 to 11 amino acids) which have a biologically desired function in the sense of the invention. by endogenous enzymes into smaller fragments (pharmaceutically active form comprising at least 7 to 11 amino acids) which have a biologically desired function in the sense of the invention. That is, the individual fragments of the oligopeptide exhibit activity as HLA-A, HLA-B or HLA-C tumor antigen peptides and thus contribute to the activation of T cells.

In a particular embodiment, any HLA-A peptide sequence may be a humanized and/or sequence optimized sequence as further described herein.

### HLA-B antigen peptides and HLA-B neoantigen peptides

An "HLA-B antigen peptide" is defined herein as an "HLA-B antigen peptide of the invention" or "amino acid sequence of the invention" (as defined herein) comprising:
a) an amino acid sequence consisting of 12 to 17 amino acids; and/or
b) a neoantigen peptide with an amino acid sequence consisting of 12 to 17 amino acids, which is
   i) is analogous to at least one HLA-B antigen peptide exposed on the cell surface of cells from malignant and/or neoplastic tissue (in particular carcinoma) of the individual to be treated, and
   ii) in its amino acid sequence at least one amino acid exchange with the wild type of this HLA-B antigen peptide (so-called *"HLA-B neoantigen peptide")* and
   iii) has at least a 3-fold increased specific affinity towards the T cell receptor of endogenous T cells

Furthermore, the inventors have recognized that HLA-B neoantigen peptides which in their amino acid sequence have at least one amino acid exchange in amino acid positions 3, 8 and/or 10 compared to the wild type of this HLA-B antigen peptide, have an at least 4-fold, particularly preferably at least 7-fold increased specific affinity towards the T cell receptor of the body's own T cells and are therefore particularly preferred for use in the formulation according to the invention.

Particularly preferably, the amino acid exchange in the amino acid sequence of the HLA-B antigen peptide is a single amino acid exchange in amino acid position 3, 8, or 10.

Preferably, the amino acid exchange in the amino acid sequence of the HLA-B neoantigen relative to the wild-type of this HLA-B antigenic peptide is an E/A, E/K, R/W, or D/A exchange.

Preferably, the HLA-B antigen peptide or the HLA-B neoantigen peptide has a specific activity (K _{D}) towards the T cell receptor, as determined by any suitable detection method known to the skilled person in the prior art, of less than 100 nM, more preferably less than 75 nM or most preferably less than 50 nM, such as less than 40 nM, 35 nM, 30 nM, 25 nM or 20 nM.

Preferably, the HLA-B tumor antigen peptide or the HLA-B neoantigen is present in the pharmaceutical composition according to the invention at a concentration, as defined above, of at least 100 to 600 *µg,* alternatively preferably at an absolute concentration of > 600 *µg* relative to the volume of the pharmaceutical composition to be applied.

Preferably, the HLA-B peptides or HLA-B neoantigens are selected as defined above in point b)

The use of compounds, constructs, proteins or polypeptides as defined according to the invention, which consist of at least two identical or different peptide sequences of HLA tumor antigen peptides and/or HLA neoantigens, has the further advantage that the longer amino acid sequences of the compounds, constructs, proteins or polypeptides result in a longer retention time in the tissue of the subject after application, whereby the compounds, constructs, proteins or polypeptides after application to the subject can be broken down, for example, by the body's own enzymes into smaller fragments (pharmaceutically active form comprising at least 7 to 11 amino acids) which have a biologically desired function in the sense of the invention. by endogenous enzymes into smaller fragments (pharmaceutically active form comprising at least 7 to 11 amino acids) which have a biologically desired function in the sense of the invention. That is, the individual fragments of the oligopeptide exhibit activity as HLA-A, HLA-B or HLA-C tumor antigen peptides and thus contribute to the activation of T cells.

In a particular embodiment, any HLA-B peptide sequence may be a humanized and/or sequence optimized sequence as further described herein.

### HLA-C antigen peptides and HLA-C neoantigen peptides

An "HLA-C antigen peptide" is defined herein as an "HLA antigen peptide of the invention" or "amino acid sequence of the invention" (as defined herein) comprising:
an amino acid sequence consisting of 8 to 11 amino acids; and/or
a) a neoantigen peptide having an amino acid sequence consisting of 8 to 11 amino acids which is
   i) is analogous to at least one HLA-C antigen peptide exposed on the cell surface of cells from malignant and/or neoplastic tissue (in particular carcinoma) of the individual to be treated, and
   ii) in its amino acid sequence at least one amino acid exchange with the wild type of this HLA-C peptide (so-called *"HLA-C neoantigen peptide")* and
   iii) has at least a 3-fold increased specific affinity towards the T cell receptor of endogenous T cells

Furthermore, the inventors have recognized that HLA-C neoantigen peptides which in their amino acid sequence compared to the wild type of this HLA-C antigen peptide have at least one amino acid exchange in the amino acid positions 1 (N-terminus), 4 and/or the C-terminus, have an at least 4-fold, particularly preferably at least 5-fold, very particularly preferably at least 7-fold increased specific affinity towards the T cell receptor of the endogenous T cells and are therefore particularly preferably used for the formulation according to the invention.

Particularly preferably, the amino acid exchange in the amino acid sequence of the HLA-C antigen peptide is a single amino acid exchange in amino acid position 1 (N-terminus), 4, or the C-terminus.

Preferably, the amino acid exchange in the amino acid sequence of the HLA-C neoantigen peptide relative to the wild type of this HLA-C antigen peptide is a C/Y, A/P, L/F or T/M exchange

Preferably, the HLA-C antigen peptide or the HLA-C neoantigen peptide has a specific activity (K _{D}) towards the T cell receptor, as determined by any suitable detection method known to the skilled person in the prior art, of less than 100 nM, more preferably less than 75 nM or most preferably less than 50 nM, such as less than 40 nM, 35 nM, 30 nM, 25 nM or 20 nM.

Preferably, the HLA-C tumor antigen peptide or the HLA-C neoantigen is present in the pharmaceutical composition according to the invention at a concentration, as defined above, of at least 100 to 600 *µg,* alternatively preferably at an absolute concentration of > 600 µg relative to the volume of the pharmaceutical composition to be applied.

Preferably, the HLA-C antigen peptides or HLA-C neoantigen peptides are selected as defined above in point b)

The use of compounds, constructs, proteins or polypeptides as according to the invention, which consist of at least two identical or different peptide sequences of HLA tumor antigen peptides and/or HLA neoantigens, has the further advantage that the longer amino acid sequences of the compounds, constructs, proteins or polypeptides result in a longer retention time in the tissue of the subject after application, whereby the compounds, constructs, proteins or polypeptides after application to the subject can be broken down, for example, by the body's own enzymes into smaller fragments (pharmaceutically active form comprising at least 7 to 11 amino acids) which have a biologically desired function in the sense of the invention. by endogenous enzymes into smaller fragments (pharmaceutically active form comprising at least 7 to 11 amino acids) which have a biologically desired function in the sense of the invention. That is, the individual fragments of the oligopeptide exhibit activity as HLA-A, HLA-B or HLA-C tumor antigen peptides and thus contribute to the activation of T cells.

In a particular embodiment, any HLA-C antigen peptide sequence may be a humanized and/or sequence optimized sequence as further described herein.

### HLA class II antigen peptides and HLA class II neoantigen peptide

An "HLA Class II antigen peptide" is defined herein as an "HLA peptide of the invention" or "amino acid sequence of the invention" (as defined herein) comprising:
a) an amino acid sequence consisting of 13 to 20 amino acids, particularly preferably 13 to 17 amino acids; and/or
b) a neoantigen having an amino acid sequence consisting of 13 to 20 amino acids, particularly preferably 13 to 17 amino acids, which

i) is analogous to at least one class II HLA peptide exposed on the cell surface of cells from malignant and/or neoplastic tissue (in particular carcinoma) of the individual to be treated, and
ii) in its amino acid sequence at least one amino acid exchange compared to the wild type of this class II HLA peptide (so-called *"class II HLA neoantigen")* and
iii) has at least a 3-fold increased specific affinity towards the T cell receptor of endogenous T cells

Furthermore, the inventors have recognized that HLA neoantigens of class II, which in their amino acid sequence compared to the wild type of this HLA peptide of class II have at least one amino acid exchange in amino acid positions 3, 6, 10, 12, 13 and/or 14, particularly preferably in amino acid positions 12 and/or 14, have an at least 4-fold increased specific affinity towards the T cell receptor of the body's own T cells and are therefore particularly preferred for the formulation according to the invention.

Preferably, the amino acid exchange in the amino acid sequence of the class II HLA neoantigen relative to the wild type of this class II HLA peptide is an E/K, E/A, D/Y, or T/M exchange.

Preferably, the class II HLA peptide or class II HLA neoantigen has a specific activity (K_{D} ) towards the T cell receptor, as determined by any suitable detection method known to the skilled person in the prior art, of less than 100 nM, more preferably less than 75 nM or most preferably less than 50 nM, such as less than 40 nM, 35 nM, 30 nM, 25 nM or 20 nM. Preferably, the HLA tumor antigen peptide corresponding to class II MHC complexes or the HLA neoantigen corresponding to class II MHC complexes is present in the pharmaceutical composition according to the invention at a concentration, as defined above, of at least 100 to 600 *µg,* alternatively preferably at an absolute concentration of > 600 µg relative to the volume of the pharmaceutical composition to be applied.

The use of compounds, constructs, proteins or polypeptides as defined according to the invention, which consist of at least two identical or different peptide sequences of HLA tumor antigen peptides and/or HLA neoantigens, has the further advantage that the longer amino acid sequences of the compounds, constructs, proteins or polypeptides result in a longer retention time in the tissue of the subject after application, whereby the compounds, constructs, proteins or polypeptides after application to the subject can be broken down, for example, by the body's own enzymes into smaller fragments (pharmaceutically active form comprising at least 7 to 11 amino acids) which have a biologically desired function in the sense of the invention. by endogenous enzymes into smaller fragments (pharmaceutically active form comprising at least 7 to 11 amino acids) which have a biologically desired function in the sense of the invention. That is, the individual fragments of the oligopeptide exhibit activity as HLA-A, HLA-B or HLA-C tumor antigen peptides and thus contribute to the activation of T cells.

In a particular embodiment, any class II HLA peptide sequence may be a humanized and/or sequence optimized sequence as further described herein.

The use of at least one HLA tumor antigen peptides corresponding to class II MHC complexes comprised in at least one HLA tumor antigen polypeptide for the treatment of cancer according to the invention has the significant advantage that B cells are also activated in a targeted manner in the subject to be treated.

The use of HLA tumor antigen peptides of class II according to the invention has the further advantage that HLA antigen peptides, which have longer amino acid sequences than HLA tumor antigen peptides of class I, can be broken down by the body's own enzymes, for example, into smaller fragments (comprising at least 7 to 11 amino acids) after application to the test person, which have an activity as HLA-A, HLA-B or HLA-C antigen peptides and thus contribute to the activation of T cells.

"Fragment" means a portion of a polypeptide that preferably contains at least 10%, 20%, 30%, 40%, 50%, 60%, 70%, 80%, 90%, 95% or more of the total length of the reference polypeptide. A fragment may contain 7, 8, 9, 10, 11, 12, 13, 14, 15, or more amino acids.

In particular, amino acid sequences and polypeptides of the invention are preferred as defined in the claims and those in which: at least one HLA tumor antigen peptide is analogous to at least one HLA tumor antigen peptide exposed on the cell surface of cells from malignant and/or neoplastic tissue of the individual to be treated, which peptide has in its amino acid sequence at least one amino acid exchange compared to the wild type of said HLA tumor antigen peptide (i.e., HLA neoantigen peptide).HLA neoantigen peptide) and, compared to the wild type of this HLA tumor antigen peptide, has at least a 3-fold increased specific affinity towards the T cell receptor of the endogenous T cells.

### HLA tumor antigen polypeptide (HTAPP)

It is an outstanding achievement of the inventors to have found that a pharmaceutical composition containing more than 3, preferably between 5 and 25 such HLA tumor antigen peptides is particularly effective. The pharmaceutical composition according to the invention therefore preferably contains at least 5, very preferably at least 8, most preferably 10 of the HLA tumor antigen peptides corresponding to class I and/or class II MHC complexes arranged on HLA tumor antigen polypeptides, in particular those selected from the list consisting of SEQ-ID-Nos.: 1 to 37, 40 to 68. A further outstanding achievement of the inventors is to have found that, in particular in vitro and in vivo, the potency of HLA tumor antigen peptides can be particularly enhanced by arranging them on tandem or overlapping HLA tumor antigen polypeptides, preferably the potency, measured e.g. as [nspots/pM] via an ELISpot method, of these HLA tumor antigen polypeptides being 1-10, particularly preferably 1. 5-5 times higher than that of individual HLA tumor antigen peptides. e.g. as [nspots/pM] via an ELISpot method, of these HLA tumor antigen polypeptides is 1-10, particularly preferably 1. 5-5 times higher than that of the individual HLA tumor antigen peptides associated with the HLA tumor antigen polypeptide.

It is a great achievement of the inventors to have found that multimerization of HLA tumor antigen peptides (HTAP) corresponding to MHC class I and/or class II complexes by their arrangement on at least one HLA tumor antigen polypeptide (HTAPP), wherein the HLA tumor antigen peptides (HTAP) are tumor-exclusive or tumor-associated and are presented on the cell membrane of the associated tumor cell and correspond to an amino acid sequence of a transcribed mutant gene, the in vitro and in vivo effect of such HLA tumor antigen polypeptides is greatly enhanced compared to the single related HLA tumor antigen peptides. Moreover, a single HLA tumor antigen polypeptide may advantageously correspond to at least one, preferably at least two, more preferably at least three, most preferably at least 5, alternatively preferably at least 6, most preferably at least 8 HLA tumor antigens that correspond to MHC I or II class complexes, and as such may be predisposed for use in a subject or group of subjects, preferably a group of subjects.

Preferably, a monovalent amino acid sequence (or a polypeptide comprising only one amino acid sequence of the invention) used in the invention is such a one as to bind to a T cell receptor of endogenous T cells with a 3-fold increased specific affinity compared to the corresponding wild-type HLA peptide sequence.

It should be noted that "may specifically bind to" and "specifically binds to" are used interchangeably herein and refer to the ability to bind specifically to the corresponding specified entity.

It is important in connection with the present invention that the diagnosis of the cancer(s) is made in advance by the treating physician.

It is possible and preferred to combine amino acid sequences belonging to different classes of HLA tumor antigen peptides used in the invention in a single HLA tumor antigen polypeptide of the invention. In particular, it has been demonstrated that the combination of HLA-A, HLA-B and/or HLA-C tumor antigen peptides and/or corresponding neoantigen peptides in a single polypeptide of the invention have unique binding properties.

By one skilled in the art, the specific activity (K_{D} ) of the polypeptides of the invention comprising more than one component of the amino acid sequence of the HLA-A, HLA-B and/or HLA-C tumor antigen peptides and/or neoantigen peptides corresponding to the MHC complexes of class I and/or II can be determined according to one of the detection methods described above/below, wherein the compounds, constructs, proteins or polypeptides of the invention preferably have a specific activity similar to the specific activity of each of their components, i.e. a specific activity similar to the specific activity of each of the (individual components of the) amino acid sequences of class I and/or II contained in the compounds, constructs, proteins or polypeptides of the invention. Some specific, but not limiting, examples of the above preferred compounds, constructs, proteins or polypeptides are compounds, constructs, proteins or polypeptides that either.

Some specific, but not limiting, examples of such compounds, constructs, proteins or polypeptides of the invention are set forth, for example, in Tables 1-6 or are apparent to those skilled in the art based on the present disclosure.

According to another preferred embodiment of the present invention, the HLA tumor antigen polypeptide is present (in each case) as a single-membered amino acid sequence, i.e. not as an element of compounds, constructs, proteins or polypeptides consisting of at least two identical or different amino acid sequences for HLA tumor antigen peptides/neoantigens, in which the HLA tumor antigen peptides/neoantigens are connected to each other by suitable linkers.

Preferably, the HLA tumor antigen peptides of the invention exhibit specific activity towards T cell receptors (T cells are used for this purpose), which can be determined using any suitable detection method known to those skilled in the art, such as EliSpot AlphaScreen detection methods (as described herein) or cell-based detection methods (as described herein). Preferably, the blocking activity is determined using a cell-based detection method.

In particular, the compounds, constructs, proteins or polypeptides of the invention comprising an amino acid sequence of an HLA tumor antigen peptide of the invention and belonging to MHC class I (as defined herein) preferably have a specific affinity of 10 to 50 nM towards the corresponding T cell receptor.

It is also within the scope of the present invention that an amino acid sequence of the invention may bind to two or more class I and/or class II MHC complexes, epitopes, components, domains or subunits of a class I and/or class II MHC complex. In such a case, the MHC complexes, epitopes, components, domains or subunits of an MHC complex to which the amino acid sequences and/or polypeptides of the invention bind may be substantially the same or different (and in the latter case, the amino acid sequences and polypeptides of the invention may bind to such different complexes, epitopes, components, domains or subunits of a class I and/or II MHC complex, including combinations thereof, with an affinity and/or specificity that may be the same or different).

It is also expected that the polypeptides of the invention will generally bind to all naturally occurring or synthetic analogs, variants, mutants, components and fragments of a class I and/or class II MHC complex, respectively. Also in such a case, the amino acid sequences and polypeptides of the invention may bind to such analogs, variants, mutants, alleles, components and fragments with an affinity and/or specificity equal to or different from the affinity and specificity with which the amino acid sequences of the invention bind to the wild types of the class I and/or II MHC complex, respectively.

It is also within the scope of the present invention that the amino acid sequences and polypeptides of the invention bind to some analogs, variants or mutants of a class I and/or class II MHC complex, but not others.

In a specific, but not limiting, embodiment of the present invention, compounds, constructs, proteins or polypeptides comprising an HLA tumor antigen peptide of the invention may have an increased half-life in serum compared to the amino acid sequence from which they were derived. For example, an amino acid sequence of the present invention may be linked (chemically or otherwise) to one or more groups or moieties that extend half-life (such as PEG) such that they are a derivative of an amino acid sequence of the invention with increased half-life.

In general, the compounds or polypeptides of the invention with increased half-life preferably have a half-life that is at least 1.5 times, preferably at least 2 times, such as at least 5 times, for example at least 10 times or more than 20 times higher than the half-life of the corresponding amino acid sequence of the invention per se. For example, the compounds or polypeptides of the invention with increased half-life have a half-life that is more than 1 hour, preferably more than 2 hours, more preferably more than 6 hours, such as more than 12 hours or even more than 24, 48 or 72 hours higher compared to the corresponding amino acid sequence of the invention per se.

In general, when an HLA tumor antigen peptide of the invention (or a compound, construct or polypeptide comprising the same) is intended for administration to a subject (for example, for therapeutic and/or diagnostic purposes, as defined herein), it is preferably either an amino acid sequence that does not naturally occur in the subject; or, if naturally occurring in the subject, it is in substantially isolated and at the same time concentrated form (as defined herein).

In addition, it will also be apparent to those skilled in the art that, for pharmaceutical use, the HLA tumor antigen peptides of the invention (and the compounds, constructs and polypeptides comprising the same) will be directed against a human T cell receptor including combinations thereof as defined in the claims; wherein, for veterinary purposes, the polypeptides of the invention are preferentially directed against a T cell receptor including combinations thereof (as defined in the claims) from the species to be treated or are at least cross-reactive towards a T cell receptor including combinations thereof from the species to be treated.

Th cancer to be treated is preferably a hormone positive, HER2/neu or triple negative cancer.

### Detailed description and definitions

In the present description and claims, the following terms are defined as follows:
In the context of the present invention, the features of the invention designated "comprising" are intended to be understood to include the more limited description of "consisting of" or "consisting essentially of" the same features of the present invention.

The term "and/or" is used to specifically disclose the two features or components together or separately. Therefore, the term "and/or" as used, for example, in the phrase "I and/or II" in the present disclosure includes "I and II", "I or II", "I" and "II".

A pharmaceutical composition is to be understood herein as a so-called informatic that can be administered to a subject or a group of subjects with at least one identical HLA allele and which contains the combination of HLA tumor antigen peptides arranged on HLA tumor antigen polypeptides according to the invention in the concentration disclosed herein, wherein an HLA tumor antigen polypeptides and its comprised HLA tumor antigen peptide(s) represents an information carrier. This means that the arrangement of amino acids in the amino acid sequence of the HLA tumor antigen polypeptides ("code") induces a sequence-specific activation of the immune system, in particular of T cells (by HLA tumor antigen peptides corresponding to MHC class I complexes) and/or B cells (by HLA tumor antigen peptides corresponding to MHC class II complexes). Thus, the *in vitro* or *in vivo* loading of MHC class I complexes or MHC class II complexes of the tumor cells with the HLA tumor antigen peptides of the pharmaceutical composition according to the invention having an identical or slightly modified amino acid sequence with HLA tumor antigen peptides and their corresponding HLA tumor antigen polypeptides, respectively, renders contacting T cells with said HLA tumor antigen peptides tumor cells sensitive to lysis of the tumor cells of the tissue or tissue resection by specific cytotoxic or specifically activated T lymphocytes.

However, the primary objective of the present invention is not to load tumor cells with HLA tumor antigen peptides in *vitro* or *in vivo,* but to induce specific activation and training of the immune system, in particular of T cells and B cells against tumor cells. For this reason, the pharmaceutical composition according to the invention is preferably applied subcutaneously or intradermally and, preferably substantially simultaneously (i.e., successively) at least 2, particularly preferably at least 3, sites of application remote from a tumor lesion and/or the cancerous lymph node area. This has the particular advantage that the immune system or the T cells, respectively, which recognize the applied HLA tumor antigen peptides, process this information applied in the form of tumor antigen peptides and consequently specifically recognize and lyse tumor cells presenting these HLA tumor antigen peptides on their surface.

Therefore, the method according to the invention has the advantage that in case the signals emitted by the tumor towards the immune system are too weak (passive) or the tumor actively secretes substances that promote its growth (stimulate macrophages) (active e.g. TREX or BD1 ramp up), the immune system, in particular the T cells can be trained to the presence of the tumor even if the signals emitted by the tumor are too weak. The signals emitted by the tumor are too weak when the presentation of HLA tumor antigen peptides on the cell surface of the tumor cells is low or decreases in response to cytotoxic T cell attacks that have occurred (escapemechanism).

For the purposes of the present invention, an HLA tumor antigen peptide, also sometimes simply referred to as HLA antigen peptide, is an HLA tumor antigen peptide if it is a tumor-exclusive HLA antigen peptide (i.e. expressed and/or exposed exclusively by tumor cells; a cancer testis antigen (CTA) that is no longer present in the healthy/normal tissue of the adult subject/group of subjects; or a so-called neoantigen peptide) or a tumor-associated HLA antigen peptide. Analogously, an HLA antigen polypeptide is an HLA tumor antigen polypeptide if at least one, preferably two HLA tumor antigen peptides are arranged on it, preferably executed as a tandem or overlapping HLA tumor antigen polypeptide.

*"Tumor-exclusive HLA antigen peptides"* are mutated HLA antigen peptides resulting, for example, from a mutation of a gene, where the mutation of the gene is causative for tumor growth and/or is related to oncogenesis. They are also referred to as "tumor-exclusive antigen(s)", short TEA(s) or as "tumor-specific antigen(s)", short TSA(s). The mutated gene product in the tumor is specific for the individual subject or a certain group of subjects with at least one identical HLA allele.

*"Tumor-associated HLA antigen peptides"* include non-mutated HLA antigen peptides expressed in the adult stage only in some tissues of the subject or a specific group of subjects with at least one identical HLA allele, but also in tumor cells. They are also referred to as "tumor-associated antigen(s)" or TAA(s). The immunogenicity of tumor-associated HLA antigen peptides is usually low, since their presence in healthy cells can produce immunological tolerance (immune tolerance). Nevertheless, the induction of a strong immune response against HLA antigen peptides that are merely tumor-associated carries the risk of an autoimmune response. In some preferred embodiments, targeting TAAs may be advantageous if they are preferably overexpressed by at least a factor of 3, more preferably by at least a factor of 5, especially if they are associated with tumor growth and can complement a strong immune response.

*"Tumor-exclusive HLA antigen polypeptides"* comprise at least one mutated HLA antigen peptides resulting, for example, from a mutation of a gene, where the mutation of the gene is causative for tumor growth and/or is related to oncogenesis. The mutated gene product in the tumor is specific for the individual subject or a certain group of subjects with at least one identical HLA allele.

*"Tumor-associated HLA antigen polypeptides"* comprise at least a non-mutated HLA antigen peptides expressed in the adult stage only in some tissues of the subject or a specific group of subjects with at least one identical HLA allele, but also in tumor cells. The immunogenicity of tumor-associated HLA antigen peptides is usually low, since their presence in healthy cells can produce immunological tolerance (immune tolerance). Nevertheless, the induction of a strong immune response against HLA antigen peptides that are merely tumor-associated carries the risk of an autoimmune response. In some preferred embodiments, targeting TAAs with Tumor-associated HLA antigen polypeptides may be advantageous if they are preferably overexpressed by at least a factor of 3, more preferably by at least a factor of 5, especially if they are associated with tumor growth and can complement a strong immune response.

A chimeric human leukocyte antigen (HLA) allele, also known as a fusion HLA allele, is an allele created by combining or fusing sequences from two different HLA alleles. This can occur naturally during the process of meiosis. In the context of the present invention, these chimeric HLA alleles can be considered as two different HLA alleles, since they also occur individually in subjects. This has the advantage that an HLA tumor antigen polypeptide comprising a chimeric HLA tumor antigen peptide corresponding to a chimeric HLA allele is a tandem peptide with all the advantages set forth herein.

A "tandem polypeptide", also referred to as "tandem HLA tumor antigen polypeptide" or "tandem polypeptide of the invention" or simply "tandem peptide" comprises or consist of at least 2 HLA tumor antigen peptides according to the invention corresponding to MHC class I and/or class II complexes. The individual HLA tumor antigen peptides may be directly connected or bound by an amino acid linker. A linker is preferably between 3 and 5 amino acids in length, most preferably selected from GPGPG, AAY or AYY. This allows for better flexibility of the peptide backbone and allows for cleaving of a HTAPP into fragments, which may be processed into MHC class I and/or II complex inside of a cell. Providing the fragments in the form of a tandem **HTAPP** instead of a mixture of smaller fragments comprising the **HTAPP** is more effective and represents an improvement over the prior art. The process of creating tandem peptides may be referred to as linking, which may include the use of a linker sequence or also directly fusing the amino acid sequences of the HLA tumor antigen peptides and/or HLA tumor antigen polypeptides together.

Furthermore, in a preferred embodiment of the present invention, a tandem polypeptide comprising at least two HLA tumor antigen peptides linked by a linker, preferably selected from AAY or AYY, can be readily cleaved, preferably enzymatically. This allows the HLA tumor antigen peptide to be precisely matched to an MHC class I and/or class II complex and, at the same time, has the advantage that a single polypeptide can be used to simultaneously target different epitopes of a mutant cell, preferably a cancer cell.

An "overlapping tandem polypeptide", also referred to as "overlapping tandem HLA tumor antigen polypeptide" or "overlapping tandem polypeptide of the invention" or simply "overlapping peptide" comprises or consist of at least 2 HLA tumor antigen peptides according to the invention corresponding to MHC class I and/or class II complexes, which overlap in their amino acid sequence. Overlap in the context of the inventions means that the individual amino acid sequences of at least two different HLA tumor antigen peptides share a partial consecutive sequence of their amino acid sequence. Preferably, this overlap consists of a single amino acid, more preferably two amino acids, most preferably at least three amino acids, but never exceeds the total length of either peptide minus one amino acid to ensure that the amino acid sequence of the peptides cannot be congruent. This advantageously allows to effectively multimerize an HLA tumor antigen polypeptide and increase the density of matching HLA alleles and mutated genes, thus allowing an efficient polypeptide formation with a length of preferably between 15 and 45, preferably between 17 and 40 amino acids, while reducing the risk of disadvantages associated with longer amino acid chains, especially peptide folding, tertiary structure interactions, increased risk of enzymatic or chemical cleavage and degradation.

A "delivery aiding capping peptide" (DACP) in the sense of the current invention is a peptide, i.e., a short amino acid sequence, preferably between 10 and 20 amino acids in length, that can be directly or indirectly (via a linker) attached to the C- or N-terminus of an HLA tumor antigen polypeptide according to the present invention. Advantageously, this peptide increases the ability of **HTAPP** to enter or penetrate the cell, allowing it to enter the cell more easily, which is critical due to the presentation mechanism of MHC class I and II complexes on the surface of a cancerous cell: MHC class I molecules are central to cellular immune defense by presenting intracellular antigenic peptides on the surface of almost all nucleated cells. This pathway begins with the degradation of intracellular proteins by the proteasome. The resulting peptides are then transported to the endoplasmic reticulum (ER) via the transporter associated with antigen processing (TAP). In the ER, these peptides are loaded onto MHC class I molecules, which are then transported to the cell surface via the Golgi apparatus. Upon presentation, these complexes can be recognized by CD8+ cytotoxic T cells, leading to the destruction of cells that have abnormal or pathogen-derived peptides, such as those from tumor cells or virus-infected cells. MHC class II molecules are mainly expressed on the surface of antigen-presenting cells and play a critical role in the immune system by presenting extracellular antigenic peptides to CD4+ helper T cells. These molecules scavenge exogenous antigens that are taken up into the cell by endocytosis and then processed in endosomes. MHC class II molecules synthesized in the ER are transported to endosomes, where the invariant chain (II) occupying the peptide-binding groove is replaced by antigenic peptides with the help of HLA-DM. The peptide-MHC class II complexes are then expressed on the cell surface, where they are recognized by T helper cells and trigger an adaptive immune response in which other immune cells are activated. Therefore, it is highly desirable, especially for HLA class I complexes, to be able to efficiently enter the intracellular space of a cancer cell to effectively present the epitope. They can then be transported by a transporter associated with antigen processing (TAP) to the endoplasmic reticulum (ER), where they are encoded on the corresponding MHC class I complexes.

An immunogenic HLA tumor antigen peptide is also referred to herein as an "epitope".

According to a preferred embodiment of the present invention, MHC complexes corresponding to tumor-exclusive or tumor-associated HLA antigen peptides are associated with proliferation, invasiveness, angiogenesis, and an increase in cytokeratin production of carcinoma. The skilled person is aware of relevant databases and literature listing these effects (e.g., the National Center for Biotechnology Information (NCBI) databases).

The HLA tumor antigen polypeptide(s) (HTAPPs) and HTAPP-DACPs of the present invention, alongside the Tumor-Associated Antigens (TAAs) and Tumor-Exclusive Antigens (TEAs), preferably the peptides disclosed in sequences SEQ.-ID-No.: 1 to 481, especially preferably selected from the list comprising SEQ.-ID-No.: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, and 481, may be employed either individually or in various combinations within immunogenic therapies for the treatment and/or prophylaxis in a subject or group of subjects suffering from or at risk from suffering from cancer. These therapies are preferably directed towards immunotherapeutic approaches, including but not limited to Chimeric Antigen Receptor T-cell (CAR-T) therapies, RNA/DNA-based pharmaceuticals and/or vaccines and/or pharmaceutical compositions, and peptide pharmaceuticals and/or vaccinations and/or pharmaceutical compositions. Furthermore, the invention encompasses the disclosure of peptide sequences along with their corresponding RNA and DNA nucleotide sequences, which are responsible for transcribing the aforementioned peptides.

The at least one HLA tumor antigen polypeptide within the scope of the present invention is particularly formulated for subcutaneous administration. The term/concept "composition" therefore refers to the provision of at least one HLA tumor antigen peptide and an adjuvant in a pharmaceutical formulation that allows good applicability and includes solutions, in particular injection solutions and infusion solutions, concentrates for the preparation of injection and infusion preparations, powders for the preparation of injection and infusion preparations and subcutaneous implants.

Pharmaceutical compositions are prepared by dissolving or suspending the determined HLA tumor antigen peptides in a carrier liquid (i.e., a pharmacologically acceptable vehicle), optionally with the addition of other excipients such as wetting agents, dyes, permeation enhancers, resorption enhancers, preservatives, antioxidants, light stabilizers.

The carrier fluid is preferably selected from the group consisting of Sodium Chloride Injection Solution, Ringers Injection Solution, Isotonic Dextrose, Sterile Water, Dextrose Solution, Lactated Ringers Injection Solution, Distilled Water, or mixtures thereof, for local injection.

It is particularly advantageous for good solubility of the determined HLA tumor antigen polypeptides if their amino acid sequence has the lowest possible number of hydrophobic amino acids.

Preferably, dimethyl sulfoxide (DMSO), ethoxyethylene diglycol, ethanol, phosphatidylcholines, propylene glycol dipelargonates (DPPG), or glycolysed ethoxylated glycerides are suitable permeation promoters.

According to a preferred embodiment of the present invention, the pharmaceutical composition comprises water, a pharmaceutically acceptable saline solution and/or DMSO. For example, pharmaceutical compositions comprising a mixture of about 30% DMSO and 70% water are suitable for application. Another preferred mixture comprises 25% DMSO and 75% water. A higher water content is desirable because DMSO, while largely nontoxic, is associated with side effects such as unpleasant odor and may mediate the entry of other toxins into the body.

The term *"class* I *HLA tumor antigen peptide (corresponding to MHC complexes)"* as used herein refers to a peptide sequence that is bound to or immunogenic for the class I MHC complex (HLA complex in humans). The class I HLA protein complex is used for antigen presentation on the cell surface and comprises a heavy chain with 3 domains (α1, α2, and α3) and the β2-microglobulin (β2M).

The term *"class II HLA tumor antigen peptide (corresponding to MHC complexes)"* refers to a polypeptide sequence that is bound or immunogenic to the class II MHC complex (in humans, the HLA complex). The class II HLA protein complex is used for antigen presentation on the cell surface and consists of two chains of nearly equal size, an α-chain and a non-covalently bound β-chain, each chain having two extracellular domains (α1 and α2 and β1 and β2).

As such, the polypeptides and pharmaceutical compositions of the present invention (as defined herein) may be used for use in the prevention and treatment of cancer (also referred to herein as "cancer of the invention" or as "carcinoma", or to as "malignant tissue", or to as "cancerous tissue"). In general, the "cancer of the invention" may be defined as diseases and disorders that can be appropriately prevented and/or treated by appropriate administration of either a tumor antigen peptide or a pharmaceutical composition of the invention (and more particularly, a pharmaceutically effective amount thereof) to a subject (i.e., a person having the disease or disorder, or at least one symptom thereof, and/or who is at risk of acquiring or developing such disease or disorder).

Preferably, a cancer of the invention may be selected but is not limited to the list comprising breast cancer, lung cancer, prostate cancer, colon cancer, stomach cancer, liver cancer, cervical cancer, bladder cancer, non-Hodgkin's lymphoma, skin cancer, thyroid cancer, kidney cancer, ovarian cancer, pancreatic cancer, and esophageal cancer.

For the purposes of the present invention, a "peptide sequence" (e.g., an HLA tumor antigen peptide or an HLA tumor antigen peptide arranged on an HLA tumor antigen polypeptide) having a "native sequence" comprises a peptide sequence having the same (i.e., unmodified) amino acid sequence as a naturally occurring peptide sequence in the subject. Such a peptide sequence with a "native sequence" can be isolated from nature or produced recombinantly or synthetically. In particular, the term peptide sequence having a "native sequence" includes naturally occurring truncated or secreted forms of the peptide sequence (e.g., an extracellular domain sequence), naturally occurring variants (e.g., alternatively spliced forms), and naturally occurring allelic variants of the peptide sequence.

In this context, the pharmaceutical composition is preferably applied subcutaneously ("under the skin"), or intradermally ("into the skin"), or intramuscularly ("into the muscle"). The application may be intramuscular as an injection into the thigh (vastus lateralis muscle), preferably into the upper arm (deltoid muscle).

As further described herein, the amino acid sequences used in the invention are single variable HLA antigen peptide domains ("HLAs" or "HLA complex"). A single variable HLA antigen peptide domain is (as further defined herein) a region within the amino acid sequence of a protein that can be distinguished from its surrounding sequence based on defined characteristics.

Amino acid sequences or regions within the amino acid sequence of a protein of the invention that are HLAs are also referred to herein as *"HLAs of the invention."* Some preferred examples of single variable HLA tumor antigen peptide domains suitable for use in the invention are apparent from the further description herein and include, in particular, HLA-A, HLA-B and HLA-C antigen peptides corresponding to MHC class I complexes and HLA-DR, DQ and DP antigen peptides corresponding to MHC class II complexes.

Such neoantigen peptides (i.e., HLA tumor antigen peptides expressed and/or exposed exclusively by tumor cells) that have less than 100% sequence identity or similarity to the native HLA tumor antigen peptide are preferably characterized by an amino acid substitution in the amino acid sequence for the purposes of the present invention.

The following terms are used to describe the sequence relationships between two or more amino acid sequences or polypeptide sequences: "reference sequence," "amino acid exchange," "sequence identity," "percentage of sequence identity," and "substantial identity."

In the context of the present invention, the term "amino acid substitution" or amino acid exchange refers to the substitution of one amino acid for another amino acid within the amino acid sequence of the HLA antigen peptide to be synthesized relative to the wild type of such HLA tumor antigen peptide (i.e., native HLA antigen peptide). "Sequence identity," "percentage of sequence identity," or identity or similarity with respect to such amino acid sequence is defined herein as the percentage of amino acid residues in the amino acid sequence of the polypeptide that is identical (i.e., same residue) or similar (i.e., amino acid residue from the same group based on common side chain characteristics, see below) to the amino acid sequence of the wild type.

According to a preferred embodiment of the present invention, the amino acid substitution for the HLA tumor antigen peptide corresponding to the class I and/or class II MHC complexes comprises at least one substitution at any position within the amino acid sequence relative to the wild type of that HLA peptide.

According to another preferred embodiment of the present invention, the amino acid exchange for the HLA tumor antigen peptide corresponding to class I and/or class II MHC complexes comprises at least one of a D/Y, a C/Y, an A/V, a T/M, an E/A, or a D/A exchange at any position within the amino acid sequence relative to the wild type of said HLA peptide.

The amino acids used herein are abbreviated according to the generally accepted single letter code of the IUPAC Nomenclature Commission. Where two amino acids are separated by a hyphen (/), this indicates that at a specific amino acid position in the amino acid sequence concerned, the wild-type amino acid (left side of the hyphen) has been replaced by another amino acid (right side of the hyphen).

To determine/derive preferred anchor positions and a preferred specific amino acid exchange in the amino acid sequence of HLA tumor antigen peptides, *in silico* modeling methods are particularly suitable, such as by means of the algorithm NetMHC 4.0 (http://www.cbs.dtu.dk/services/NetMHC/) based on the publications by Andreatta and Nielsen (Bioinformatics (2016) Feb 15;32(4):511-7) and Nielsen et al. (Protein Sci., (2003) 12:1007-17).

The term "cancer" pertains to a complex group of diseases characterized by uncontrolled cell growth and proliferation, which can originate in various tissues and organs throughout the body. Cancer may be incited by a myriad of factors, whether sourced externally, such as through exposure to carcinogens, or driven by internal genetic mutations or malfunctions, leading to the development and progression of malignant tumors. In humans, "cancer" broadly encompasses a spectrum of conditions that induce pain, dysfunction, and distress, both physically and emotionally, to the individual impacted, and potentially imposes social and psychological burdens upon those in close association with them. This all-encompassing perspective on cancer may, at times, also embrace a variety of facets related to the disease, such as secondary symptoms, anomalies in physiological structures and functions, and implications of specific cancer treatments or therapies. In this context, cancer refers to but is not limited to, cancers that predominantly threaten individuals, namely: Lung, Breast, Colorectal, Prostate, Stomach, Liver, Cervical, Thyroid, Bladder, Non-Hodgkin Lymphoma, Kidney, Leukemia, Pancreatic, Ovarian, and Esophageal cancers. Importantly, it is recognized that confronting and managing cancer can significantly reshape an individual's life perspective and personality due to the profound challenges and adjustments invariably associated with diagnosis, treatment, and survivorship.

In this context, the term "mutanoma" is a neologism of "mutation" and "carcinoma" and is intended to emphasize the central role of genetic mutations in the formation and development of neoplasms. As such, a mutanoma is the sum of genetic mutations in a subject or group of subjects who have cancer or are at risk of developing cancer.

For purposes of comparing two or more amino acid sequences, the percentage of "sequence identity" between a first amino acid sequence and a second amino acid sequence may be calculated or determined by dividing the number of amino acids in the first amino acid sequence that are identical to amino acids at corresponding positions in the second amino acid sequence, by [the total number of amino acids in the first amino acid sequence] and multiplying by [100%], wherein each deletion, insertion, substitution or addition of an amino acid in the second amino acid sequence - compared to the first amino acid sequence - is considered as a difference to a single amino acid (position).The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The amino acids used herein are abbreviated according to the generally accepted 15 one-letter code of the IUPAC Nomenclature Commission. Where two amino acids are separated by a hyphen (/), this indicates that at a specific amino acid position in the amino acid sequence concerned, the wild-type amino acid (left side of the hyphen) has been replaced by another amino acid (right side of the hyphen). In the present invention, the amino acids (IUPAC one-letter code) S, T, C, N, Q, Y are considered hydrophilic acids; the amino acids (IUPAC one-letter code) A, F, G, I, L, M, P, V, W are considered hydrophobic acids; the amino acids (IUPAC one-letter code) D, E, are considered acidic; the amino acids (IUPAC one-letter code) R, K, H are considered basic acids.

The terms "immunization" or "vaccination" describe the process of administering an antigen to an individual with the purpose of inducing an immune response, for example, for therapeutic or prophylactic reasons.

An HLA tumor antigen polypeptide is "immunogenic" if the tumor cells expose on their cell surface at least one corresponding MHC class I complex and/or at least one corresponding MHC class II complex that recognizes and binds to the HLA tumor antigen peptide, i.e. the HLA tumor antigen peptide exhibits a high specific activity towards this MHC class I complex and/or MHC class II complex. The immunogenicity of the HLA tumor antigen peptide can be determined by Western blot, ELISA techniques, in particular by ELISPOT or immunodetection with microscopic analysis.

The HLA tumor antigen peptides of the present invention are preferentially immunogenic and are therefore also referred to as immunogenic HLA tumor antigen peptides ("epitopes"). The immunogenicity of the HLA tumor antigen peptides can be determined by suitable detection methods. Such methods are known to the skilled person and/or described herein.

The HLA tumor antigen polypeptides of the present invention are preferentially immunogenic and are therefore also referred to as immunogenic HLA tumor antigen peptides ("epitopes"). The immunogenicity of the HLA tumor antigen polypeptides can be determined by suitable detection methods. Such methods are known to the skilled person and/or described herein.

The term "specific affinity" or "specific binding affinity" of the HLA tumor antigen peptide towards the T cell receptor (TCR) refers to the specific and reversible binding of the HLA peptide to the TCR of the endogenous T cells. This "specific affinity" according to the present invention is expressed in moles via the dissociation constant (K _{D}) determined by ligand binding assays.

Alternatively, the "specific affinity" of the HLA tumor antigen peptide can be determined by *in silico* methods.

According to a preferred embodiment of the present invention, at least one HLA tumor antigen peptide is a tumor-exclusive HLA tumor antigen peptide (i.e., a cancer-testis antigen (CTA) that no longer occurs in the healthy/normal tissue of the subject/group of subjects or a so-called neoantigen peptide), and wherein the specific binding affinity of the tumor-exclusive HLA tumor antigen peptide is determined against the corresponding MHC class I complex with a K_{D} in the range of 10 to 50 nM, as determined by surface plasmon resonance.

According to a preferred embodiment of the present invention, at least one HLA tumor antigen polypeptide is a tumor-exclusive HLA tumor antigen polypeptide (i.e., a cancer-testis antigen (CTA) that no longer occurs in the healthy/normal tissue of the subject/group of subjects or a so-called neoantigen peptide), and wherein the specific binding affinity of the tumor-exclusive HLA tumor antigen polypeptide is determined against the corresponding MHC class I and/or II complex with a K_{D} in the range of 10 to 50 nM, as determined by surface plasmon resonance.

Based on conventional *in silico binding prediction models* (modeling), tumor antigen peptides with a K_{D} of <50 nM are generally considered strong-binding and those with a K_{D} between 50 and 500 nM are considered weak-binding tumor antigen peptides. However, *in the in vivo determinations* according to the invention, it has now been shown that the K_{D} value is not necessarily important. Surprisingly, it was found that tumor antigen peptides that had a K_{D} value in the range of 50 to 500 nM triggered effector cells (i.e., cytotoxic T cells) contrary to conventional *in silico binding prediction models that* considered them to have low binding. Thus, it is essential for tumor antigen peptide efficacy that the tumor antigen peptides are immunogenic.

The term "active substance enhancer/adjuvant" refers to an adjuvant which triggers and/or enhances the effect of the HLA peptides in the first place. In principle, all commonly used adjuvants known to the skilled person are suitable for the production of a formulation according to the invention.

The term "individual" (also referred to herein as "subject" or "patient") is used interchangeably with the term "subject" to mean any mammal that is being treated for an abnormal physiological condition or has been diagnosed with a disease.

The terms "individual" and "subject" as used in the present invention include mammals, such as a rodent, a carnivore, a cloven-hoofed animal, an odd-toed ungulate, or a primate. In particularly preferred embodiments, the subject is a human.

Where reference is made herein to a "subject group" or a "group of subjects" or a "patient group", reference is always made to a group of individuals, preferably human beings, who all have at least one, preferably at least two, most preferably at least three identical HLA allele(s).

It is permissible that subjects to be treated with the pharmaceutical composition have received a standard therapy procedure (e.g., at least one surgery, radiation, chemotherapy, and/or hormone therapy).

The terms "individual" and "subject" as used in the present invention include mammals, such as a rodent, a cloven-hoofed animal, an odd-toed ungulate, or a primate. In particularly preferred embodiments, the subject is a human. Unless otherwise stated, the terms "individual" and "subject" do not denote a particular age, and thus encompass adults, elderlies, children, and newborns. In some embodiments, the term "subject" includes humans of age of at least 50, at least 55, at least 60, at least 65, at least 70, or older. In some embodiments, the term "subject" includes humans of age of at least 65, such as 65 to 80, 65 to 75, or 65 to 70.

However, the pharmaceutical composition may also be administered as a first-line therapy to the subject or specifically identified group of subjects with at least one identical HLA allele.

According to a preferred embodiment of the present invention, the individual has not yet received chemotherapy for locally recurrent or metastatic cancer and/or has not received prior adjuvant chemotherapy in recurrence for 12 months or less since the last dose.

Particularly preferably, the individual has the haplotype with subgroup A*01 or A*02.

A "haplotype" (an abbreviation for "haploid genotype") is the sum of the composition of all specific alleles (= specific fingerprint) of a subject and denotes a variant of a nucleotide sequence on one and the same chromosome in the genome of a living being. A specific haplotype can be individual-, population- or species-specific.

For the purposes of this invention, the transcriptome comprises the sum of all genes transcribed at a given time in a cell, i.e. transcribed from the DNA sequence into mRNA sequences, i.e. the totality of all as well as the quantification of the individual mRNA molecules produced in a cell. However, the creation of the transcriptome does not yet allow any statement about the "correctness" of the transcribed mRNA sequences.

In this context, the term "building a transcriptome" as used in the present disclosure refers to the analysis of the transcriptome as the sum of all genes transcribed in a cell at a given time point preferably by quantitative real-time (RT)-PCR followed by DNA microarray or subsequent DNA sequencing. Typically, constructing the transcriptome of the tissue sample provided in step (a) of the determination procedure of the invention comprises acquiring more than 40,000 coding DNA sequence (raw data).

In genetics, the exome is the totality of the exons of an organism, i.e. all sections that potentially code for proteins. In humans, the exome comprises about 23,000 genes with approximately 50 million nucleobases. *Whole* exome *sequencing* (WES) examines all exons, i.e. the sections coding for proteins in the genome of a tissue section (i.e. healthy tissue or tumor tissue of a test person). Genetic diagnostics focuses on these 1-2% of the human genome, where 85% of known disease-causing mutations are found.

Accordingly, exome analysis involves sequencing the exome of the subject (and other relatives, if applicable), evaluating the sequence data, and summarizing the results in a medical report. This diagnostic procedure is the method of choice to find the cause of the disease, especially in subjects with complex or unspecific symptoms and a diagnosis that has often remained unexplained for years.

Compared to *Whole Exome Sequencing* (WES), in which all protein-coding regions of the approximately 23,000 known genes are enriched and sequenced, *Clinical Exome Sequencing* (CES) enriches a subset of the exome. In WES, the focus is on determining disease-associated genes described in the *Human Gene Mutation Database* (HGMD).

For the purposes of the present invention, the proteome refers to the totality of all proteins of at least one cell in a malignant or neoplastic tissue/tissue section or a cell compartment thereof, under precisely defined conditions and at a specific point in time. The proteome of a cell can be determined by proteome sequencing and is linked to the genome of that cell via the transcriptome.

Immunotherapies are based on deciphering the individual mutation pattern (signature) of the tumor of each cancer subject. Based on the profile of the mutation pattern, synthetic vaccines, for example RNA-based vaccines, are produced for each individual subject according to conventional treatment approaches (i.e. vaccine production or production of the informaticum according to the invention). These are subsequently used for the individual treatment of the subject.

Basically, these novel vaccines are no good for other subjects with the same tumor, but can only be used for the respective subject whose mutanoma has been previously analyzed for vaccine production or production of the informaticum of the invention. Therefore, it is an outstanding achievement of the inventors to have recognized that different subjects have overlap in the mutanoma of the malignant or neoplastic tissue/tissue resection thereof. Advantageously, different subjects can be divided into uniform subject groups such that at least 50% of the HLA peptides of the formulation according to the invention is compatible with the mutanoma of the subject group.

The totality of all HLA tumor antigen peptides presented on the cell surface via MHC molecules is referred to as the (HLA) ligandome for the purposes of the invention. It is assumed that more than 10⁵ HLA molecules are expressed on the cell surface and the number of identical HLA peptides presented can vary from a few to up to 10,000 copies per cell. Consequently, approximately 10,000 different HLA peptides are presented on a cell in varying proportions.

The ligandome is influenced by various physiological, intrinsic as well as pathological (e.g. cancer or necrosis) factors such as cell type or tissue type, infection or transformation of the cell, or simply the current state of the cell, which depends on nutrient situation or external stress factors, resulting in changes in the HLA peptides presented.

At the beginning of the analysis of the HLA ligandome, for example, Edman degradation can be used to gain first insights into the presented peptides. On the one hand, it is possible in this way to determine the general peptide motif of an allele via pool sequencing, and on the other hand, individual peptide sequences can already be determined via the analysis of individual *reversed phase high performance liquid chromatography* (RP-HPLC) fractions.

Alternatively or complementarily, the analysis of the ligandome can be performed by using modern mass spectrometers in proteomics, with which it is possible to unambiguously determine the sequences of many individual ligands. Two methods are used for the ionization of the peptides or proteins required for this purpose: Electrospray ionization (ESI) and *matrix-assisted laser desorption*/*ionization* (MALDI). In ESI, coupling with an RP-HPLC system is common.

However, as mass spectrometers have become more sensitive, capillary electrophoresis (CE) has also been used as an analytical separation method.

In order to achieve a higher sample throughput and increased sensitivity in ligandome analysis, the so-called UHPLC systems *(ultra high performance liquid chromatography)* can be used. These HPLC systems use only 2 µm diameter materials as packing material for separation columns, resulting in improved speed, efficiency and chromatographic separation.

In order to achieve a higher sample throughput and increased sensitivity in ligandome analysis, the so-called UHPLC systems (ultra-high performance liquid chromatography) can be used. These HPLC systems use only 2.2 to 1.7 µm diameter materials as packing material for separation columns, resulting in improved speed, efficiency, and chromatographic separation.

In ESI mass spectrometry, the direct coupling of HPLC and ESI interface allows on-line separation of the sample, which, in combination with an autosampler, enables a fully automated measurement procedure. Because of the continuous solvent flow from the HPLC, samples can be measured in a relatively short time. ESI mass spectrometry uses a wide range of instruments for analysis such as quadrupole time-of-flight mass spectrometers, linear quadrupole ion traps, triple quadrupoles, or ion trap-orbitrap hybrid systems. This advantageously allows the identification of hundreds of HLA peptides in one measurement.

IFNy ELISpot analysis (for protocol see A. Lalvani, R. Brookes, S. Hambleton, W. J. Britton, A. V.S. Hill, A. J. McMichael, J Exp Med 1997; 186 (6): 859-865) was performed ex vivo (without further in vitro culturing for expansion) using PBMCs depleted of CD4+ and enriched for CD8+ T cells (CD8+ effectors), or depleted of CD8+ and enriched for CD4+ T cells (CD4+ effectors). Tests were performed in duplicate and with a positive and negative control, Multiscreen filter plates (Merck Millipore) pre-coated with IFNγ-specific antibodies (ELISpotPro kit, Mabtech). 267.000 Cells were used per plate. Plates were scanned using an AID Classic Robot ELISPOT Reader and analyzed by AID ELISPOT 7.0 software (AID Autoimmun Diagnostika). Antigen Peptides (except negative and positive control group) were done in duplicates, spot counts were taken as mean values of each duplicate.

The term "deriving a ranking" in the context of the present invention refers to determining/selecting the quantity and affinity of HLA peptides exposed on the cell surface of the cells of the harvested tissue (or a tissue section thereof).

Using the previous analyses, a cumulative ranking for the HLA tumor antigen peptides with respect to protein quality and specific affinity (K_{D} ) towards the T cell receptor of the endogenous T cells is derived. Thereby, with regard to protein quality, especially content-related factors for tumor progression, such as invasiveness, angiogenesis, but also escape mechanisms of the tumor against immune attack are evaluated.

In another embodiment, the highest-ranked sequence possibilities may be further qualified by their existence in a database of possible HLA tumor antigen peptides with a high specific affinity to the endogenous T cell receptors (as defined herein) predicted from sequence data, particularly one that is restricted to the organism/proband from which the HLA tumor antigen peptide was obtained. In another embodiment, the highest ranked sequence possibilities may be further qualified by the separation coordinates of the HLA tumor antigen peptide (e.g., isoelectric point and molecular weight of a protein) and/or its monomer composition.

To provide (tumor) antigen peptides, synthetic or isolated HLA tumor antigen peptides derived from the cumulative ranking can in principle be used for the preparation of the (drug) formulations of the present invention and for the preparation of the pharmaceutical composition (as so-called informatics).

However, it is a good idea to use synthetic HLA peptides. Processes for the synthetic production of peptides are known to the skilled person. Examples of such production methods are the Merrifield solid-phase peptide synthesis, the Bailey peptide synthesis and the *N-carboxylic anhydride method.*

A further object of the present invention is also (pharmaceutical-) formulations in different dosage forms which contain the combination of active ingredients according to the invention and optionally further active ingredients and/or excipients.

Preferred drug formulations are tablets, chewable tablets, chewing gums, coated tablets, capsules, drops, juices, syrups, suppositories, transmucal therapeutic systems, transdermal therapeutic systems, solutions, injections, emulsions, suspensions, easily reconstitutable dry preparations, powders or sprays. Particularly preferred drug formulations are injections or solutions.

Alternatively, the drug formulation is present in a suitable application device, preferably as a lyophilizate in a syringe, which allows *in situ reconstitution* with a pharmaceutically acceptable solution (e.g., saline).

Preferably, the (drug) formulations according to the invention are suitable for oral, intravenous, intramuscular, subcutaneous, intrathecal, epidural, buccal, sublingual, pulmonary, rectal, transdermal, nasal or intracerebroventricular administration, with (drug) formulations for subcutaneous or intravenous administration being particularly preferred.

Methods known in the prior art for the preparation of pharmaceutical compositions or dosage forms are found in, for example, "Remington's Pharmaceutical Sciences". Pharmaceutical compositions for parenteral administration may contain, for example, excipients, sterile water, or saline, polyalkylene glycols, such as polyethylene glycols, oils of vegetable origin, or hydrogenated naphthalenes. Biocompatible, biodegradable lactide polymer, lactide/glycolide copolymers, or polyoxyethylene-polyoxypropylene copolymers can be used to control the release of the compounds. Other potentially useful parenteral delivery systems for the therapeutic anti-prion compounds include ethylene vinyl acetate copolymer particles, osmotic pumps, implantable infusion systems, and liposomes.

In the context of the current invention, artificial intelligence (AI), also referred to machine learning, network, deep learning network, weighted network, neural network, is defined as any machine learning (ML), deep learning (DL), or other model specifically tailored to run and optimize the selection of HLA tumor antigen peptides. Especially, these models are adept at comparing, evaluating, weighting, and transforming a multitude of parameters associated with amino acid sequences and their biochemical interactions.

Machine Learning, also referred to as neural network, or machine learning model, for the purposes of this invention, means any algorithm that can use machine learning to build a model based on sample data, referred to as training data, to make predictions or decisions. This algorithm does not have to be explicitly programmed for these circumstances but builds its model exclusively on the training data set.

In a preferred embodiment of the present invention, traditional machine learning models, such as support vector machines, random forests, and gradient boosting machines, are utilized. These models offer the advantage of being interpretable and relatively swift in their training phase. Their strength lies in their ability to perform exceptionally well on structured data, making them less susceptible to overfitting when the dataset size is limited. In an especially preferred embodiment a random forest is used, which has the advantage of having feature importance included directly into the model.

Preferably within the scope of the present invention, deep learning models, including Convolutional Neural Networks and Recurrent Neural Networks, are employed for tasks involving unstructured data. These models are particularly proficient in handling vast datasets, such as images or sequences, and have the innate capability to autonomously extract salient features from the data.

In a preferred embodiment, transformer-based models like BERT, BART, and LAMA are incorporated. These models, especially effective for sequence data, can discern intricate patterns and relationships within the data when pre-trained on extensive corpora. Their potential is further amplified when fine-tuned on domain-specific data.

Preferably within the scope of the present invention, a hybrid approach is adopted that amalgamates the strengths of different architectures. For instance, a fusion of Convolutional Neural Networks with Recurrent Neural Networks can be employed to extract and process features.

In a preferred embodiment of the present invention, ensemble models, which harness the power of multiple models through techniques like bagging or boosting, are utilized. These models offer a robust solution by aggregating predictions, enhancing accuracy, and reducing overfitting.

The present invention comprises a pharmaceutical composition for use as a medicament, in particular for use in the therapeutic and/or prophylactic treatment of a carcinoma, particularly preferably a locally recurrent or metastatic carcinoma, especially preferably a carcinoma selected from the list comprising pancreatic cancer (PaCa), liver metastasis (LMCa); bone cancer metastasis (BMCa), prostate cancer (PrCa), Breast Cancer (BrCa), Uterine Cancer (UCa), Brain Cancer (BrnCa), Bladder Cancer (BICa), Thymus Cancer (ThCa), Biliary Tract Cancer (BiTCa), Skin Cancer (SkCa), Thyroid Cancer (ThdCa), Lung Cancer (LuCa), Colorectal Cancer (CoCa), Esophageal Cancer (EsCa), Stomach Cancer (StCa), Head and Neck Cancer (HNCa), in particular in a subject or group of subjects suffering from or at risk of suffering from a carcinoma, comprising a pharmacologically effective amount comprising 2 to 25 HLA tumor antigen peptides (HTAP) corresponding to MHC class I and/or class II complexes, preferably a number HTAP in the numerical range obtained by combining any two of the following end point values: 2, 3, 4, 5, 6, 7, 8, 9, 10, 11, 12, 13, 14, 15, 16, 17, 18, 19, 20, 21, 22, 23, 24, 25, arranged on at least one, preferably between 2 to 7 HLA tumor antigen polypeptide(s) (HTAPP), wherein the HLA tumor antigen peptides (HTAP) are tumor-exclusive or tumor-associated and presented on the cell membrane of the exclusive and/or associated tumor cell and correspond to an amino acid sequence of a transcribed mutant gene, wherein the HLA tumor antigen polypeptide comprises a scaffold amino acid sequence, preferably between 15 and 45, more preferably between 17 and 40 amino acids, alternatively preferred the amino acids of the scaffold is in the numerical range obtained by combining any two of the following end point values: 15, 16, 17, 19, 21, 23, 25, 27, 29, 31, 33, 35, 37, 39, 41, 43, and 45,
a) wherein the scaffold amino acid sequence comprises or consists of, in addition to HLA tumor antigen peptide(s), up to 1 to 30 amino acids, (long HLA tumor antigen polypeptide); and/or
b) wherein the scaffold amino acid sequence comprises or consists of an HLA tumor antigen peptide(s) with at least 90% sequence identity similarity to the native HLA tumor antigen peptide (similarity HLA tumor antigen polypeptide); and/or
c) wherein at least one of the amino acid sequences comprises or consists of an HLA tumor antigen peptide having an amino acid sequence comprising or consisting essentially of only one amino acid substitution relative to the amino acid sequence of the native HLA tumor antigen peptide (substitution HLA tumor antigen polypeptide),
wherein
i) an HLA tumor antigen polypeptide is a tandem polypeptide comprising or consisting of at least 2, preferably between 2 and 10, HLA tumor antigen peptides corresponding to MHC class I and/or class II complexes; and/or
ii) an HLA tumor antigen polypeptide is an overlapping tandem polypeptide comprising or consisting of at least 2, preferably between 2 and 10, HLA tumor antigen peptides corresponding to MHC class I and/or class II complexes which overlap in their amino acid sequence.

In a preferred embodiment of the pharmaceutical composition for the treatment of carcinoma, the scaffold amino acid sequence within the HLA tumor antigen polypeptide(s) (HTAPP) introduces unique variations tailored to specific needs.

The invention further discloses a long HLA tumor antigen polypeptide (a), wherein the scaffold amino acid sequence in this embodiment comprises up to 1 to 30 amino acids in addition to the HLA tumor antigen peptide(s). This longer sequence could increase the stability of the polypeptide and allow more efficient delivery to amino acid sequences present on carcinoma cells. The presence of additional amino acids in the scaffold, also referred to as backbone, which refers to the amino acid sequence, could also increase the affinity of the polypeptide for its corresponding MHC class I and/or II complex, improving specificity and reducing potential off-target effects.

The invention also encompasses a similar HLA tumor antigen polypeptide. In a preferred embodiment, the scaffold amino acid sequence comprises HLA tumor antigen peptide(s) that have at least 90% sequence identity similarity to the native HLA tumor antigen peptide. By maintaining such a high degree of similarity to the native peptide, this composition could better mimic the natural interactions in the body. This would likely enhance the therapeutic effect by ensuring that the immune system recognizes and efficiently attacks tumor cells. In addition, these modifications may increase binding affinity, allow for the inclusion of additional HLA alleles, or reduce unwanted intra- or intermolecular interactions.

The invention also includes an embodiment relating to (c) a substitution HLA tumor antigen polypeptide in which the amino acid sequence of the HLA tumor antigen peptide has a minor modification: an amino acid sequence that either comprises, or consists essentially of, only one amino acid substitution compared to the native HLA tumor antigen peptide. This single substitution can significantly alter the properties of the peptide, potentially increasing its binding affinity or altering its immunogenic properties. This could result in the pharmaceutical composition eliciting a stronger and more targeted immune response against carcinoma cells.

The tailored designs of the scaffold amino acid sequences in these embodiments offer specific advantages when incorporated into pharmaceutical compositions for the treatment of carcinoma. By manipulating the structure and sequence of the HLA tumor antigen polypeptide(s), the therapeutic potential of the drug can be optimized to ensure efficient targeting and elimination of carcinoma cells. Variations - from extended sequences to minor substitutions - provide the opportunity to tailor treatment to the type of carcinoma and genetic makeup of the subject or group of subjects. However, these adaptations, which are feasible and obvious to the person skilled in the art when carrying out the method according to the invention with little effort, advantageously allow for lower side effects, the inclusion of new HLA alleles and cancer-related mutated genes of a subject or group of subjects included lead to higher pharmaceutical composition efficacy and better treatment results.

A scaffold amino acid sequence in the sense of the present invention is a sequence of amino acids linked by an amide bond, also referred to as a peptide sequence. Preferably, a scaffold amino acid sequence is linear. Preferably, the scaffold amino acid sequence of an HTAPP comprises between 15 and 45, more preferably between 17 and 40 amino acids to reduce the risk of protein folding and degradation that may increase with peptide size.

In an especially preferred embodiment of the current invention, the pharmaceutical composition may be used for the treatment of any cancer which has at least one mutation in a gene expressed on the surface of the mutant cell (epitope) or a gene which is overexpressed at least by factor three, more preferably factor 5 in relation to native genes in healthy cells. The type of cancer that can be treated by the composition according to the invention can preferably be selected from (but is by no means limited to) the list consisting of: pancreatic cancer (PaCa), liver metastasis (LMCa); bone cancer metastasis (BMCa), prostate cancer (PrCa), Breast Cancer (BrCa), Uterine Cancer (UCa), Brain Cancer (BrnCa), Bladder Cancer (BICa), Thymus Cancer (ThCa), Biliary Tract Cancer (BiTCa), Skin Cancer (SkCa), Thyroid Cancer (ThdCa), Lung Cancer (LuCa), Colorectal Cancer (CoCa), Esophageal Cancer (EsCa), Stomach Cancer (StCa), Head and Neck Cancer (HNCa). More preferably, the type of cancer that can be treated by the composition according to the invention can be selected from (but is by no means limited to) the list consisting of: Breast Cancer, Prostate Cancer, Pancreatic Cancer, Kidney Cancer, Testicular Cancer, and Osteosarcoma (Bone Cancer).

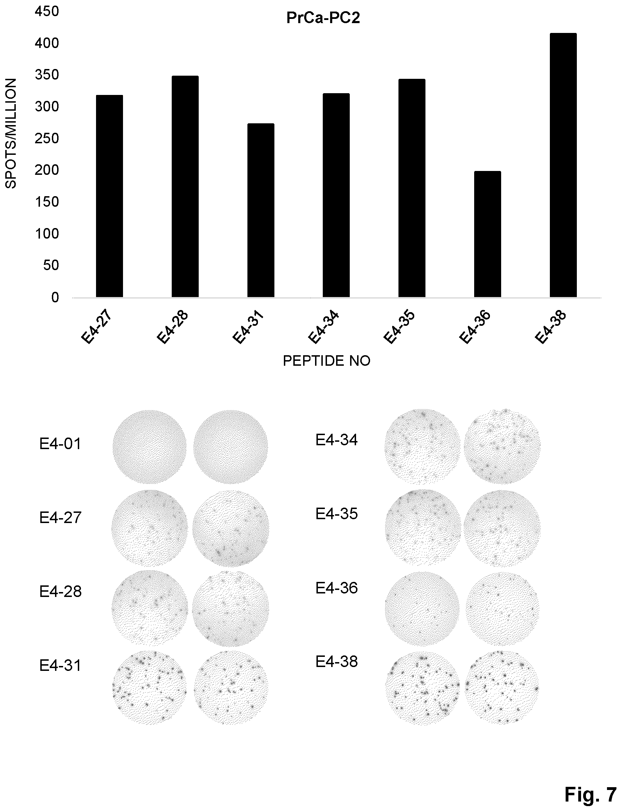

Advantageously, the pharmaceutical composition comprises only linked tandem polypeptides in a particularly preferred embodiment, the efficacy of the immune response can be optimized with respect to potentially exposed MHC class I and/or II complexes carrying the HLA tumor antigen peptides, corresponding to genes associated with a carcinoma and the HLA alleles of a subject, thereby eliciting a stronger and more responsive immune response, as exemplified by some of the preferred HTAPPs of the invention in the examples, particularly in Fig. 7A and Fig. 7B, is shown. This has the additional technical advantage, that fusion, chimeric or combination of genes related to the carcinoma can be targeted at once. The drastic effect of the combination is especially well documented in figure 7B, were the linking of the two HLA tumor antigen polypeptides SEQ-ID-No.: 77 and SEQ-ID-No.: 78 to the tandem peptide SEQ-ID-No.: 8 led to an increase in Elispot count of more than 237% in relation to the more immunogenic single HTAP which shows a synergistic effect of this step.

In a particularly preferred embodiment, the tandem polypeptide may comprise one HTAP linked to another HTAP, which are each related to different HLA alleles and different genes associated or exclusive for a specific cancer type. This allows for more diverse targeting. In another preferred embodiment, at least one HTAPPs can be linked with a HTAP or another HTAPP together to form a tandem polypeptide in the sense of the present invention, preferably combining different genes and HLA peptides. Advantageously, this can be used in a group approach to target a broad range of different mutations in genes commonly associated with a particular type of cancer and associated with an HLA ligandome of a broad range of subjects, wherein at least one HLA allele in each subject is matched to the pharmaceutical composition comprising tandem HTAPPs as defined in the present invention.

The effect of the process of linking multiple HLA tumor antigen peptides on a single tandem HLA tumor antigen polypeptide, preferably by a AAY or AYY linker, is preferably at least a 10% boost in immune response, as determined by in vivo or in ex vivo Elispot Sport count difference, more preferably at least 20%, particularly preferably at least 30%, most preferably at least 50%.

Advantageously, the pharmaceutical composition comprises only overlapping tandem polypeptides in a particularly preferred embodiment, the efficacy of the immune response can be optimized with respect to potentially exposed MHC class I and II complexes carrying the HLA tumor antigen peptides, corresponding to genes associated with a carcinoma and the HLA alleles of a subject, thereby eliciting a stronger and more responsive immune response, as exemplified by some of the preferred HTAPPs of the invention in the examples, particularly in **Fig. 7A** and **Fig. 7B**, is shown. The effect of the multimerization process of overlapping multiple HLA tumor antigen peptides on a single HLA tumor antigen polypeptide is preferably at least a 10% boost in immune response, as determined by in vivo or in ex vivo Elispot Sport count difference, more preferably at least 20%, particularly preferably at least 30%, most preferably at least 50%.

In a particularly preferred embodiment, the pharmaceutical composition comprises at least one overlapping tandem polypeptide and may further utilize HTAPs. This can be used in cases where a subject or group of subjects has a very specific mutation or set of mutations associated with a carcinoma that can be selectively targeted by using a tailored set of additional short HTAPs that are less expensive to synthesize and can be made on a laboratory scale if needed.

In a particularly preferred embodiment, an overlapping tandem peptide comprises at least two, more preferably at least three, most preferably at least five different HLA tumor antigen peptides which are overlapping in their amino acid sequences. This creates a stronger immune response, since the absolute amount of peptides is limited per injection by practical means and side effects, therefore including more potential immunogenic sequences on a single polypeptide is highly advantageous.

A further clinical gain in the use of the pharmaceutical composition in the treatment of a subject or of a subject or group of subjects having at least one identical HLA allele can be achieved when the pharmaceutical composition, further, comprises a pharmacologically effective amount of at least one HLA-A tumor antigen peptide, preferably 1, 2, 3, 4 or 5 HLA-AB tumor antigen peptide(s) corresponding to MHC class I complexes and/or of at least one HLA-B tumor antigen peptide, preferably 1, 2, 3, 4 or 5 HLA-B tumor antigen peptide(s) corresponding to MHC class I complexes and/or at least one HLA-C tumor antigen peptide, preferably 1, 2, 3, 4 or 5 HLA-C tumor antigen peptide(s) corresponding to MHC class I complexes. It is understood that also the HLA-B tumor antigen peptide(s) or HLA-C tumor antigen peptide(s) corresponding to the specific haplotype of the subject or group of subjects having at least one identical HLA allele are selected for treatment or prophylaxis. In an especially preferred embodiment, an HLA tumor antigen polypeptide of the invention comprises at least one HLA-A and HLA-B tumor antigen peptide. In an especially preferred embodiment, an HLA tumor antigen polypeptide of the invention comprises at least one HLA class I and class II HLA tumor antigen peptide.

According to a preferred embodiment of the present invention, the pharmaceutical composition according to the invention comprises 2-25, alternatively preferably at least 4, 6, 7, 8, 9, 10, 12, 14, 16, 18 ,20 or 25 HLA tumor antigen peptides corresponding to MHC class I complexes and/or MHC class II complexes arranged on at least one, preferably 2, more preferably 3, particularly preferably 4, especially preferably 5, most preferably 7 HLA tumor antigen polypeptide(s), and/or at least one HLA tumor antigen peptide which is analogous to at least one HLA tumor antigen peptide exposed on the cell surface of cells from malignant and/or neoplastic tissue (in particular carcinoma) of the individual to be treated, which in its amino acid sequence has at least one amino acid exchange with respect to the wild type of this HLA antigen peptide (so-called neoantigen peptide) and at least a 3-fold increased specific affinity towards the T cell receptor of the body's own T cells (correspondingly measured and/or expressed as KD value). Particularly good success was achieved with pharmaceutical compositions having at least 12, 14, 16, 18, 20 HLA tumor antigen peptides corresponding to MHC class I complexes and/or MHC class II complexes arranged on HLA tumor antigen polypeptides.

Furthermore, in some preferred embodiments of the pharmaceutical composition according to the disclosure, a tandem polypeptide comprises at least one HLA tumor antigen peptide corresponding to MHC class I complexes and at least one HLA tumor antigen peptide corresponding to MHC class II complexes, and/or wherein the overlapping tandem polypeptide comprises at least one HLA tumor antigen peptide corresponding to MHC class I complexes and at least one HLA tumor antigen peptide corresponding to MHC class II complexes.

In a further advantageous embodiment, the inventors have found that short HLA tumor antigen peptides, for example HLA class I, are less effective than multimerized HTAPPs containing the said HTAPs. This may be attributed to decomposition, since a single cleaving of a HTAP leads to loss of the information, while the fragments of a tandem or overlapping tandem HTAPP may be still immunogenic.

Furthermore, it is an outstanding achievement to have found that by overlapping HTAPs onto HTAPPs, rather than simply coupling the HTAPs to fragments, production costs can be dramatically reduced. The production cost and yield of a peptide scales with the power of the amino acids it contains, as each coupling step must be multiplied by the associated yield (on the order of 95-99% in modern solid-state synthesis). Thus, a tandem polypeptide quickly becomes uneconomical to produce.

Preferably, the pharmaceutical composition for use as disclosed herein comprises an HLA tumor antigen polypeptide corresponding to HLA tumor antigen peptides corresponding to MHC class I and class II complexes selected from the group consisting of the amino acid sequences set forth in SEQ ID Nos: 1 to 17, 69 and 76. Preferably these sequences are used to treat a subject with a prostate, bone metastasis or pancreas cancer, as indicated by the cancer type in table 1. Advantageously, the working principle of compositions according to the current invention have been demonstrated.

In a preferred embodiment, the pharmaceutical composition for use as disclosed herein comprises an HLA tumor antigen polypeptide corresponding to MHC class I and class II complexes which are selected from the group consisting of the amino acid sequences set forth in SEQ-ID-Nos.: 1 to 17, 69, 76 and have at least one amino acid substitution relative to said amino acid sequences. This advantageously allows for adaptation for other mutations encountered or to match additional genes or HLA alleles.

In a preferred embodiment, the pharmaceutical composition for use as disclosed herein comprises an HLA tumor antigen polypeptide corresponding to MHC class I and class II complexes which are selected from the group consisting of the amino acid sequences set forth in SEQ-ID-Nos.: 1 to 17, 69, 76 and have at least 90%, more preferably 95% sequence identity to these sequences. This advantageously allows for adaptation for other mutations encountered or to match additional genes or HLA alleles.

In some preferred embodiments the pharmaceutical composition comprises at least one HTAPP, wherein the HLA tumor antigen polypeptide comprises, in addition to the HLA tumor antigen peptides, a delivery aiding capping peptide (DACP) attached to a terminal part of its scaffold amino acid sequence, wherein the DACP is preferably selected from the list consisting of penetratin, TAT, R9-TAT, DVP3, and DVP6 (DACP-1 to DACP-5). These peptides are also known as CPPs, which are DACPs in the spirit of the current invention, if they are chemically bound terminally to a HTAPP directly or by a linker. In a preferred embodiment the DACP is directly attached, especially chemically bound, to the HTAPP, in another preferred embodiment the DACP is attached to the HTAPP by a linker. In some preferred embodiments, the linker is easily enzymatically or chemically cleavable, thus enabling a decomposition in the cytosol by enzymes, for example the proteasome.

In the context of protein synthesis within a cell, MHC class I molecules typically present endogenous antigens - those that originate from the cell, such as abnormal or cancerous proteins - while MHC class II molecules present exogenous antigens that have been taken up by the cell. These processes are mediated by HLA alleles, which are highly polymorphic genes encoding MHC molecules. The presentation of HLA tumor antigen peptides by MHC molecules is an essential prerequisite for the activation of T cells, which are central to the immune system's ability to recognize and destroy cancer cells in accordance with the current invention. By coupling HLA antigen polypeptides with DACPs, they can be better transported into the cell interior, where the sequences can associate with MHC complexes either fragmented or whole. It is an outstanding achievement of the inventors to have found out that in particular tandem or overlapping HLA tumor antigen polypeptides linked to DACP (HTAPP-DACP) elicit a particularly strong ex and in vivo immunoreaction.

In a preferred embodiment of the invention, the use of a DACP enhances this mechanism of immune surveillance. As a short amino acid sequence, preferably between 10 and 20 amino acids, the DACP can bind directly or indirectly (via a linker) to the HLA tumor antigen polypeptide. The DACP facilitates the entry or penetration of the HTAPP into the cell. This may be enabled because of the high content in basic polar amino acid residues in the amino acid sequence of the DACP sequence. This increased cellular uptake is particularly beneficial because MHC molecules must present tumor-derived peptides on the cell surface to elicit an immune response, but especially for HLA class I antigen peptides corresponding to MHC class I complexes, incorporation into the cancer cell cytostome must occur before an immune response is elicited. By increasing the ability of HTAPPs to enter cells, DACPs enhance the presentation efficiency of MHC class I and/or II complexes.

The presentation of tumor antigens by MHC molecules on the surface of cancer cells is then recognized by T cells. For MHC class I, this leads to the activation of cytotoxic T cells that can directly kill tumor cells. For MHC class II, interaction with T helper cells leads to recruitment and activation of other immune cells, further enhancing the immune response against the tumor. By combining HTAPP with DACP, the invention can enhance the immunogenicity of tumor antigens, resulting in a more robust and effective immune response that specifically targets cancer cells.

While cell penetrating agents are known in the prior art, the use of DACPs, preferably (but not limited to) penetratin (SEQ ID NO: DACP-1), in the context of multimerized HLA tumor antigen polypeptides in a pharmaceutical composition is novel and represents an outstanding achievement of the inventors. By combining multiple synergistic effects to enhance the immunogenic effect of the HLA antigen peptides, the overlap or tandemization of HTAPs on larger HTAPPs allows for multiple fragments that can be associated with different genes and different HLA alleles and therefore can be presented on a variety of MHC complexes, in conjunction with the delivery mechanism that allows for extremely precise and effective targeting of carcinomas in a subject or group of subjects.

In a particularly preferred embodiment, the HLA tumor antigen polypeptide (HTAPP) comprising a delivery aiding capping peptide (DACP) has a scaffold amino acid sequence length between 30 and 60 amino acids, more preferably between 31 and 55 amino acids. This enables the technical realization of the composition through relatively low-cost peptide synthesis.

In a particularly preferred embodiment, the HTAPPs are no longer than 70 amino acids, preferably no longer than 60 amino acids, because the inventors have found that elongation beyond a certain point leads to undesirable tertiary structural interactions between the amino acids of the scaffold amino acid sequence. This may lead to a reduced effectiveness of the delivery and reduce the efficiency of the immunization, furthermore, increasing the cost and lowering the yield of the peptides.

In some preferred embodiments of the pharmaceutical composition according the invention for use as recited herein, wherein the HLA tumor antigen polypeptide comprises a amphipathic delivery aiding capping peptide (DACP), wherein the delivery aiding capping peptide comprises a positively charged and amphipathic sequence with a total percentage of 33% to 89% of arginine (R)and lysine (K) residues and preferably a high content of amphipathic phenylalanine (F), and tryptophan (W) residues, most preferably selected from the group consisting of the amino acid sequences set forth in SEQ IDs DACP-1 to DACP-25.

The inventors found that basic polar amino acids are associated with good cell-modifying properties because they can interact with the polar phospholipid portion of a eukaryotic cell membrane. The percentages of amino acids comprising the delivery aiding capping peptide (DACP) add up to 100%, in particular the addition of the percentages of 10 to 30% arginine (R), preferably 11 to 25% arginine (R) residues and a percentage of 10 to 30% lysine (K) residues, preferably 14 to 25% lysine (K) residues and preferably a high content of amphipathic phenylalanine (F) and tryptophan (W) residues adds up to 100%. Further advantageous compositions are listed in Table 3.

In some alternatively preferred embodiments, delivery aiding capping peptides (DACP) comprising or consisting of up to 100% arginine R and/or lysine K amino acids are especially preferred. In embodiments related to arginine, these may be cyclic, which may increase the cell penetrating capabilities of these capping peptides.

In a preferred embodiment of the pharmaceutical composition according to the present invention for use in the treatment or prophylaxis of cancer the HLA tumor antigen polypeptide(s) (HTAPP) comprising a delivery aiding capping peptide (DACP) is/are selected from the HLA tumor antigen polypeptide(s) consisting of the group of amino acid sequences set forth in SEQ-ID-Nos.: 1 to 17, 69 and 76 and the delivery aiding capping peptide (DACP) is selected from the group consisting of DACP-1 to DACP-25, more preferably DACP-1 to DACP-5, most preferably DACP-1.

In another preferred embodiment of the pharmaceutical composition according to the present invention for use in the treatment or prophylaxis of cancer the HLA tumor antigen polypeptide(s) (HTAPP) comprising a delivery aiding capping peptide (DACP) is/are selected from the group consisting of the amino acid sequences set forth in in SEQ-ID-Nos: 18 to 37, 40 to 68, 70, 71, 81 and 84, preferably 18 to 31, 32 to 36, 40, 43 to 44, 47 to 54, 57 to 64, 67 to 68 and 70, 71, 81, 84. These sequences are immunogenic and tested, particularly preferentially in the context of metastatic cancer, even more preferentially in the treatment of prostate and pancreatic cancer in the context of the genes indicated in Tables 2, 4, 5, and 6.

In some preferred embodiments of the pharmaceutical composition according to the present invention for use in the HLA tumor antigen polypeptide(s) (HTAPP) comprising a delivery aiding capping peptide (DACP) is/are selected from the group consisting of the amino acid sequences set forth in in SEQ-ID-Nos.: 18 to 37, 40 to 68, 70, 71, 81 and 84, preferably 18 to 31, 32 to 36, 40, 43 to 44, 47 to 54, 57 to 64, 67 to 68 and 70, 71, 81, 84, and have at least one amino acid substitution relative to said amino acid sequences.

In some preferred embodiments of the pharmaceutical composition according to the current invention, the HLA tumor antigen peptides (HTAP) corresponding to MHC class I and/or class II complexes correspond to an amino acid sequence of a transcribed mutant gene exclusive and/or associated with a carcinoma with at least one amino acid substitution compared to the native gene, wherein said gene is preferably selected from the list consisting of ABCA2, ABLIM1, ADAM8, ADAM8-NUP50, AFP, AFF2, ASIP, BRAF, CACNA2D1, CAMK1, CAN13, CCL28, CD33, CEACAM5, CEMIP2, COL10A1, CST1, CTBP2, D2HGDH, DENND4, DHRS2, DIP2A, DIP2C, DLX, DLX1, DLX1-FOLH1, EGFR, ELL3, ESR1, EXT2, EXT2-CACNA2D1, FA83A, FBXO2, FBXW7, FOLH1, GNAS, GRB14, GRIPAP1, HIVEP2, KDM3A, KIAA1804, KIT, KLK3, KRAS, LAMC1, LRRC8A, LRRC9, LUZP2, MAGEC1, MAP3K9, MMP1, MMP11, MTOR, NASP, NGAL, OPA1, PCDHGA11, PCSK1, PCM1, PLEC, PLD3, PLK1, PPFIA1-SHANK2, PRKAG1, PRR21, POTEG, POTEH, PTEN, PYGO2, RBBP6, RGS12, RIF1, RPAP1, S10A7, S10A8, S100A7, SALL3, SAMD9L, SEMA4D, SEMA4D-SEMAD4D, SEHL2, SHANK2, SHS, SLC30A8, , SMG8, SMC4, STIM1, STK11IP, STK40, STON2, STS, TDRD1, TMEFF2, TMPRSS2-ERG, TNC, TP53, TP53BP2, TRANK1, TRAP1, U2AF1, UHRF1, ZC3H12A, ZC3H12C and ZDHHC18, particularly preferably selected from the list consisting of ABLIM1, ADAM8-NUP50, BRAF, CEACAM5, CEMIP2, COL10A1, CST1, DLX1-FOLH1, EGFR, EXT2-CACNA2D1, FBXW7, GNAS, KLK3, KDM3A, KRAS, LUZP2, MMP1, MMP11, PIK3CA, PLD3, POTEG, POTEH, SEMA4D-SEMAD4D, , SMC4, TMEFF2, TMPRSS2-ERG, TP53, TNC-LAMC1 and U2AF1. These genes or combinations of these genes in the form of chimeras or fusion genes have been associated with cancer, preferably prostate, breast, pancreatic, liver, and bone cancers and their associated metastases. The use of chimeric or fusion genes is particularly preferred, especially when separated by a linker, preferably AAY or AYY or another easily cleavable sequence, since stand fragments are immunogenic and correspond to MHC complexes.

In a preferred embodiment, the pharmaceutical composition for the treatment or prophylaxis of cancer in a subject or a group of subjects the administering the pharmacologically effective amount of the tumor antigen peptides to the subject or group of subjects suffering from carcinoma is effective to reduce a tumor marker level, preferably the tumor marker is selected from the list consisting of CEA, PSA, CA 125, CA 19-9, AFP, hCG, HER2/neu, BRCA1, BRCA2, EGFR, CA 15-3, CA 27.29.

It is particularly preferred that the tumor marker targeted by this composition is selected from a list that includes CEA, PSA, CA 125, CA 19-9, AFP, hCG, HER2/neu, BRCA1, BRCA2, EGFR, CA 15-3, and CA 27.29. Each of these tumor markers is indicative of specific types of carcinomas:
CEA (Carcinoembryonic Antigen) is often associated with colorectal cancer, but it can also be elevated in other cancers such as pancreatic, stomach, breast, and lung cancer. In a preferred embodiment, the composition targets CEA to treat colorectal cancer.

PSA (Prostate-Specific Antigen) is a marker for prostate cancer. Preferred is a composition that specifically targets PSA for the treatment of prostate cancer.

CA 125 is primarily associated with ovarian cancer. Particularly preferred is a composition that targets CA 125 for ovarian cancer treatment.

CA 19-9 is commonly linked with pancreatic and liver cancer. In a preferred embodiment, the composition is formulated to reduce CA 19-9 levels, offering potential therapeutic effects for pancreatic or liver cancer patients.

AFP (Alpha-fetoprotein) is a marker for liver cancer and certain types of testicular cancers. Preferred is a composition that targets AFP for these cancer types.

hCG (Human Chorionic Gonadotropin) can be indicative of germ cell tumors. In a preferred embodiment, the composition targets hCG for the treatment of germ cell tumors.

HER2/neu is associated with aggressive forms of breast cancer. Particularly preferred is a composition that targets HER2/neu for breast cancer treatment.

BRCA1 and BRCA2 are genes associated with a higher risk of breast and ovarian cancer. In a preferred embodiment, the composition is formulated to target mutations in these genes, offering potential therapeutic effects for patients with these genetic predispositions.

EGFR (Epidermal Growth Factor Receptor) is linked with lung, colorectal, and pancreatic cancers. Preferred is a composition that specifically targets EGFR for the treatment of these cancer types.

CA 15-3 and CA 27.29 are both markers for breast cancer. In a preferred embodiment, the composition targets either CA 15-3 or CA 27.29 for the treatment of breast cancer.

The technical advantage of this composition lies in its ability to target a broad spectrum of tumor markers, offering a versatile approach to cancer treatment. Alternatively, the composition can be formulated to specifically target one or a subset of these tumor markers, allowing for specialized treatment options based on the specific cancer type. The units of the tumor marker levels can be determined using standard oncological diagnostic methods, ensuring accurate monitoring of treatment efficacy.

In another preferred embodiment of the pharmaceutical composition the HLA tumor antigen polypeptides corresponding to HLA tumor antigen peptides corresponding to MHC class I and class II complexes, respectively, are immunogenic HLA tumor antigen polypeptides as determined by an immunogenicity assay, in particular by Western blot, ELISA techniques, ELISPOT or immunodetection with microscopic analysis; preferably by at least a factor of 2, even more preferably by at least a factor of 3 of the response of a corresponding blind test. This ensures the immunogenic effect of the HTAPPs in accordance with the current invention.

Preferably, the HLA tumor antigen peptides of the composition of the invention are presented on the surface of the tumor cells of the carcinoma of at least one subject, preferably a group of subjects, as determined by ultra-high performance liquid chromatography (UHPCL) in combination with ESI mass spectrometry (MS). These methods allow rapid identification of the appropriate MHC class I and II complexes corresponding to HTAPs on HTAPPs. The immune response is directly related to the number of MHC complexes presented on the cell surface, which are recognized by T cells that can then induce apoptosis of the targeted tumor cell.

In an especially preferred embodiment of the pharmaceutical composition according to the current invention for use therapeutic agent or prophylactic agent against a tumor in a subject or group of subject suffering or at risk from suffering from cancer, at least one HLA tumor antigen polypeptide is selected to match at least one, more preferably at least two, most preferably at least 5 HLA tumor antigen peptides that are presented on the surface of the tumor cells of the subject's or group of subjects' carcinoma as determined by ultra-high performance liquid chromatography (UHPCL) in conjunction with ESI mass spectrometry (MS). By targeting several different MHC class I and II complexes, a composition can be created that is robust and tolerates slight changes in genes of the same cancer type in different patients or different HLA alleles if at least one HLA allele and one gene mutation are the same with respect to the cancer type.

Preferably the expression level of at least one HLA tumor antigen peptide in the tumor cells is at least three times higher than in the healthy cells of the subject or group of subjects having at least one identical HLA allele as determined by qPCR, and whereas the HLA tumor antigen peptides are associated with proliferation, invasiveness, angiogenesis, and an increase in cytokeratin production in carcinoma. This ensures a specific targeting of the tumor cell and reduces the possibility of an autoimmune response.

In an especially preferred embodiment of the invention, the pharmaceutical composition comprises:
- at least one HLA tumor antigen peptide is a tumor exclusive HLA tumor antigen peptide, and
- specific binding of the tumor-exclusive HLA tumor antigen peptide determined against the corresponding MHC class I complex with a K_{D} in the range of 10 to 50 nM occurs as determined by surface plasmon resonance.

In a preferred embodiment of the invention, the pharmaceutical composition comprises an the pharmacologically effective amount of each individual HLA tumor antigen polypeptide in the composition in an absolute concentration (i.e., administration dose) ranges from 100 to 1000 µg. In a more preferred embodiment, the individual dose is 300 µg per antigen polypeptide. In a particularly preferred embodiment, the total dose of all HLA tumor antigen polypeptides in the composition of the invention is between 600 to 10000 µg, more preferably between 1000 and 6000 µg. This ensures that a strong immunogenic response is generated while the side effects of the application are minimized.

In some embodiments of the pharmaceutical composition according to the current invention, the composition comprises an adjuvant which, when the composition is applied to a subject, is capable of forming a granuloma at the site of application. This allows to store the HLA tumor antigen polypeptides at the site of the application an releases them into the organism of a subject over an extended period of time, preferably between 1 and 14 days.

In a particularly preferred embodiment, the use of Montanide ISA 51 VG has proven to be suitable. After application of the (drug-) formulation to the test person, this forms a so-called granuloma which advantageously stores the HLA tumor antigen peptides in the form of a reservoir at the site of application and releases them into the organism of the test person over a longer period of time. Particularly advantageously, therefore, weekly applications of the (drug-) formulation according to the invention are omitted. Preferably, the application of the (drug-) formulation for the treatment of cancer diseases in the sense of the invention, when used over a longer period of time, therefore only has -to be -carried out every 2 weeks, particularly preferably only once a month.

It has been found that the application of the pharmaceutical composition according to the present invention for the treatment or prophylaxis of carcinoma subcutaneously or intradermally and, preferably substantially simultaneously at t least 2 sites of application, preferably at least 4 sites of application, remote from a tumor lesion and/or the cancerous lymph node area is advantageous. This ensures a controlled release of the HLA tumor antigen polypeptides in a controlled manner.

In some preferred embodiments of the pharmaceutical composition at least one HLA tumor antigen peptide has at least one mutation relative to the wild-type HLA tumor antigen peptide (the mutanoma) that results in an increase in affinity for the T cell receptor of the individual treated with HLA tumor antigen peptide compared to the wild-type HLA tumor antigen peptide. This enhances the affinity for MHC class I and class II complexes significantly, thus ensuring a more robust immune answer. Preferred examples of this amino acid exchange can be found in the tables 1,2 and 4 to 6. Particularly preferably, the HLA tumor antigen peptide with a mutation is arranged on an HLA tumor antigen polypeptide.

The pharmaceutical composition according to the present invention is suitable for the treatment or prophylaxis of almost all types of cancer, provided that at least two HLA antigen peptides can be matched with at least one, preferably two, HLA alleles and genes associated with cancer cells and presented on their epitope. Therefore, in some preferred embodiments the pharmaceutical composition is used for the treatment of carcinoma as monotherapy or in combination with other known therapies and/or compounds for the treatment of carcinoma, preferably is selected from the list consisting of breast cancer, lung cancer, prostate cancer, colon cancer, stomach cancer, liver cancer, cervical cancer, bladder cancer, non-Hodgkin's lymphoma, skin cancer, thyroid cancer, kidney cancer, ovarian cancer, pancreatic cancer, and esophageal cancer.

In some preferred embodiments of the invention, the subjects to be treated with the pharmaceutical composition have received a standard therapy procedure (e.g., at least one of surgery, radiation, chemotherapy, and/or hormone therapy). A common problem in cancer is relapse and the inability to remove the entire cancer. Pharmaceutical composition is a mild and specific therapy and can therefore complement existing invasive or drug-based therapies, especially in metastatic cancers.

In some preferred embodiments the pharmaceutical composition is administered as a first-line therapy to the subject or group of subjects having at least one identical HLA allele. This allows a more cost effective and broader application of the immune therapy.

In another preferred embodiment the pharmaceutical composition according to the present invention for the treatment or prophylaxis of carcinoma is administered to a group of subjects having at least one, preferably two, more preferably three, most preferably five identical allele and at least one HLA tumor antigen polypeptide(s) presented on the cell membrane of the associated tumor cell corresponding to an amino acid sequence of a tumor antigen polypeptide as disclosed herein. The group therapeutic approach allows broader access to this novel and inventive approach, which is hampered by the cost of determining and synthesizing an individualized therapy. By identifying common HLA alleles associated with specific cancers, a composition according to the present invention can be applied to a larger group of subjects. The shared allele is preferably selected from the list comprising of A*01:01, A*02:01, A*02:03, A*02:06, A*02:786, A*03:01, A*11:01, A*24:02, A*30:01, A*30:02, A*31:01, A*32:01, A*33:01, A*68:01, A*68:02, B*07:02, B*08:01, B*15:01, B*35:01, B*40:01, B*44:02, B*57:01, B*57:37, B*58:01, C*03:04, C*04:01, C*06:02, C*07:02, DQA1*01:01, DQB*102:01, DQB1*05:01, DQB1*06:02, DRB1*01:01, DRB1*15:01, E*01:01, and E*01:03. These Alleles are represented by the preferred sequences disclosed in tables 1,2 and 4 to 6.

In a preferred embodiment related to the administration of the composition to a group of subjects sharing at least one common HLA allele, the pharmaceutical composition comprising each individual HLA tumor antigen peptide and/or HLA tumor antigen polypeptide in the pharmaceutical composition at an absolute concentration (i.e., administration dose) of 100 to 1000 µg is administered intradermally or subcutaneously once every 2 weeks for a period of at least one year to a subject or group of subjects having at least one identical HLA allele. This provides an effective treatment suitable for broad-spectrum use.

It has been an outstanding achievement of the inventors to have found a way to increase the efficacy of HLA antigen-based immunotherapeutic agents by including tandem and overlapping tandem polypeptides that exhibit a dramatically enhanced immunogenic response. Therefore, although it is advantageous to use a composition of HLA tumor antigen polypeptides for the reasons disclosed herein, in some preferred embodiments, a single HLA tumor antigen polypeptide may be used as an agent for the treatment of carcinoma or for a pharmaceutical composition according to the present invention. In a preferred embodiment of the current invention, an HLA tumor antigen polypeptide corresponding to HLA tumor antigen polypeptides corresponding to MHC class I complexes or MHC class II complexes is used for the treatment of carcinomas or for a pharmaceutical composition according to the current invention, in particular for use in the therapeutic and/or prophylactic treatment of a carcinoma, particularly preferably a locally recurrent or metastatic carcinoma, in particular in a subject or group of subjects suffering from or at risk of suffering from a carcinoma, comprising at least two tumor antigen peptides (HTAP) corresponding to MHC class I and/or class II complexes, arranged on at least one HLA tumor antigen polypeptide(s) (HTAPP), wherein the HLA tumor antigen peptides (HTAP) are tumor-exclusive or tumor-associated and presented on the cell membrane of the associated tumor cell and correspond to an amino acid sequence of a transcribed mutant gene,
wherein the HLA tumor antigen polypeptide comprises a scaffold amino acid sequence, preferably between 15 and 45, more preferably between 17 and 40 amino acids,
   a) wherein the scaffold amino acid sequence comprises, in addition to HLA tumor antigen peptide(s), up to 1 to 30 amino acids (long HLA tumor antigen polypeptide); and/or
   b) wherein the scaffold amino acid sequence comprises an HLA tumor antigen peptide(s) with at least 90% sequence identity similarity to the native HLA tumor antigen peptide (similarity HLA tumor antigen polypeptide); and/or
   c) wherein at least one of the amino acid sequences comprises an HLA tumor antigen peptide having an amino acid sequence comprising or consisting essentially of only one amino acid substitution relative to the amino acid sequence of the native HLA tumor antigen peptide (substitution HLA tumor antigen polypeptide),
wherein
an HLA tumor antigen polypeptide is a tandem polypeptide comprising or consisting of at least 2 HLA tumor antigen peptides corresponding to MHC class I and/or class II complexes; and/or
an HLA tumor antigen polypeptide is an overlapping tandem polypeptide comprising or consisting of at least 2 HLA tumor antigen peptides corresponding to MHC class I and/or class II complexes which overlap in their amino acid sequence,
wherein the HLA tumor antigen polypeptide is preferably selected from the group consisting of the amino acid sequences set forth in SEQ-ID-Nos.: 1 to 17, 69 and 76. These represent immunogenic sequences, as determined by Elispot and case studies, therefore demonstrating the effectiveness of the HTAPPs as disclosed herein. The use of single HTAPPs is associated with lesser cost and easier manufacturing, therefore allowing a simpler access to this new therapeutic approach.

In an especially preferred embodiment the HLA tumor antigen polypeptide comprises a delivery aiding capping peptide (DACP) and is preferably selected from the group consisting of the amino acid sequences set forth in in SEQ-ID-Nos: 18 to 37, 40 to 68, 70, 71, 81 and 84, more preferably 18 to 31, 32 to 36, 40, 43 to 44, 47 to 54, 57 to 64, 67 to 68, 70, 71, 81 and 84.

Preferably the HLA tumor antigen polypeptide is used in the treatment of carcinomas, particularly locally recurrent or metastatic carcinomas in an individual, the method comprising administering to the individual a treatment regimen comprising a pharmacologically effective amount of the HLA tumor antigen peptide.

Preferably the HTAPP is used when the individual has not yet received radiation, chemotherapy, and/or hormone therapy for the cancer, particularly locally recurrent or metastatic cancer, and/or has not received prior adjuvant chemotherapy in recurrence for 12 months or less since the last dose of a chemotherapeutic agent.

Advantageously, the HTAPP can be used when the treatment regimen is effective to prolong progression-free survival of the individual by at least 2 to 5 years. This allows for prophylaxis of recurrence and minimizes the risk of autoimmune response, as the HTAPP can be tailored to known metastatic or recurrent cancers, while treating a later stage cancer does require a stronger immune response which is typically associated with a composition in accordance with the current invention.

The HLA tumor antigen peptides are preferably immunogenic in the subject or group of subjects, as determined by means of an immunogenicity assay, in particular by Western blot, ELISA techniques, in particular by ELISPOT, AFM or immunodetection with microscopic analysis. This ensures that HTAPPs are effective when used as a single therapeutic agent.

### Group therapy and general peptide pool individualization approach

In the context of group therapeutics, one embodiment of the present invention also includes an innovative approach to adaptable cancer immunotherapies. This approach leverages multimerized HLA tumor antigen polypeptides (HTAPPs) that are uniquely designed to contain at least two tandem or overlapping HLA tumor antigen peptides. These peptides are selected to correspond to MHC class I and/or class II complexes and to match the amino acid sequence of mutant genes commonly found in cancers with at least one shared mutated gene across a group of subjects with at least one common HLA allele. The concept underlying this embodiment is to bridge the gap between common mutations observed in various cancers and the predominant HLA tumor antigens detected in the HLA ligandome of a large group of subjects or potential subjects. The peptides in accordance with the current invention

In a preferred embodiment of the present invention, relating to a pharmaceutical composition for group therapy, multimerized HLA tumor antigen polypeptides on which multiple peptides corresponding to HLA complexes are arranged provides advantageous coverage of different mutant genes in a group of subjects simultaneously. By introducing more and more mutanomes from different subjects into a bioinformatic pipeline with feedback, increasingly precise HLA tumor antigen polypeptides (HTAPPs) can be generated and successively improved, expressing many different mutant gene segments and corresponding to widespread HLA alleles. Thus, a growing pool of HTAPPs can be generated, which can then be easily tailored to a specific subject. Preferably, a single HTAPP already covers different combinations of the most commonly mutated gene segments and HLA alleles in a group of subjects. By combining a number of these peptides, the precise mixture of which is tailored to the HLA alleles of the particular subject, an effective single therapy can be formed from a manageable number of HTAPPs. Preferably, databases with the most frequent mutated gene sequences, e.g. COSMIC (Catalogue Of Somatic Mutations In Cancer), TCGA (The Cancer Genome Atlas), ClinVar, ExAC (Exome Aggregation Consortium), gnomAD (Genome Aggregation Database), ICGC (International Cancer Genome Consortium), dbSNP, HGMD (Human Gene Mutation Database), cBioPortal, OncoKB, CIViC (Clinical Interpretation of Variants in Cancer) are used, which together with the most frequent alleles of a group that has cancer or is at risk of developing cancer, preferably cover a broad part of the population, preferably 90%, more preferably 95%, most preferably 98%. That allows advantageously to share the treatment of a whole group of people suffering from cancer who have at least one HLA allele and a gene mutation related to the cancer and presented on the surface of the cancer cells.

### Group HTAPP pool approach

In an alternative embodiment, the invention aims to create a reservoir of therapeutic options, also referred to as pool or pharmaceutical composition pool, that can be tailored to both individual and group needs. Hereby, a pharmaceutical composition pool contains a set of HTAPPs, for example, a pool of 5 to 100, more preferably a pool of 10 to 75, most preferably, a pool of 10 to 50. Each HTAPP in this pool is designed to target a multitude of specific mutation-HLA tumor antigen pairing, preferably at least 1 to 10, more preferably 2-8, thereby providing broad coverage of potential therapeutic targets. However, given the heterogeneity of cancer and the unique genetic nature of subjects, the invention also introduces a method for refining this broad approach. Based on a subject's specific HLA ligandome and the gene mutations identified in their tumor, a subset of the pool of these HTAPPs, preferably 2 to 10, more preferably 3 to 7, can be selected. This curated subset of the pool is then used to generate a therapeutic composition that, while tailored to the subject's unique genetic landscape, still benefits from the generalized approach of the broader HTAPP group.

In a preferred approach, in a method of determining a pharmaceutical composition comprising or consisting of HLA tumor antigen polypeptides according to the current invention, this pool approach is integrated into step III) of the pharmaceutical composition determination and creation, where the specific HLA ligandome and tumor mutations of a specific subject are first processed in the bioinformatic feedback loop pipeline, to further increase the specificity and scope of the models contained in it.

The technical implications of this innovative approach are, first, that by maintaining a broad set of HTAPPs, the invention ensures that a wide range of mutations and HLA ligandomes can be addressed, increasing the potential applicability of the therapy to a diverse subject population. Second, the ability to refine and select a subset of HTAPPs based on individual subject data ensures that the therapy remains personalized, potentially increasing its efficacy.

This dual approach, combining the advantages of both broad-spectrum and personalized therapies, offers a novel paradigm in cancer immunotherapy. It not only streamlines the therapeutic process by reducing the need for entirely bespoke treatments but also enhances the potential success rate by ensuring that the therapy is closely aligned with the subject's unique genetic and immunological profile. In summary, this embodiment of the invention represents a new approach to cancer immunotherapy not yet found in the prior art that combines the advantages of broad-spectrum treatments, e.g., regulatory hurdles, cost, applicability, and resource intensity, with the precision of personalized medicine.

The present invention further comprises a process for preparing a pharmaceutical composition or (drug-) formulation according to the invention-, wherein the process comprises the following steps:
(a) Determining at least a pharmacologically effective amount comprising 2 to 25 HLA tumor antigen peptides arranged on at least one HLA tumor antigen polypeptide(s) corresponding to MHC class I and/or class II complexes exposed on the cell surface of cells from a carcinoma of the subject or group of subjects to be treated with at least one identical HLA allele by the determination method of the invention.
(b) synthesizing at least one HLA tumor antigen polypeptide(s) comprising the 2 to 25 HLA tumor antigen peptides corresponding to MHC class I and/or class II complexes determined in step a); and
(c) Preparing the pharmaceutical composition according to the invention comprising at least the 2 to 25 HLA tumor antigen peptides corresponding to MHC class I and/or class II complexes, arranged on at least one HLA tumor antigen polypeptide(s) synthesized in step b), and an adjuvant as defined herein.

The present invention also comprises the use of the pharmaceutical composition or (drug-) formulation according to the invention for the preparation of a medicament or a combination preparation for the treatment of malignancies, leukemias and neoplasms, in particular cancer.

The synthesis of the HTAPPs comprising the HTAP(s) according to the process for preparing a pharmaceutical composition of the invention comprises a solid state synthesis. Preferably, the peptides may be prepared by standard procedures in accordance with GMP standards. The peptides (HTAPPs) may be prepared by any peptide synthesis and subsequent purification and verification known to those skilled in the art, preferably via an Fmoc (9-fluorenylmethoxycarbonyl) SPPS (solid-phase peptide synthesis) with reversed-phase high-performance liquid chromatography (RP-HPLC) purification and HPLC HRMS control of the peptides obtained.

Also encompassed by the invention is a pharmaceutical composition comprising an HLA peptide as defined in the invention and a pharmaceutically acceptable excipient.

The invention also relates to an HLA tumor antigen peptide or a neoantigen peptide of the invention for use in the preparation of a formulation (such as, without limitation, a pharmaceutical formulation, as further described herein) for the treatment of cancer, either in *vitro* (e.g. in an *in vitro* or cellular detection method) or *in vivo (such as, for example, in* a unicellular or multicellular organism and more particularly in a mammal and more particularly in a human being, such as, for example, a human being at risk of developing or suffering from a cancer of the inventions).

The present invention further comprises a Combination drug for use in the treatment of carcinoma with simultaneous, separate, or sequential administration,
comprising the following two separate preparations (a) and (b):
(a) a first preparation comprising, together with a pharmaceutically acceptable carrier or diluent, a pharmacologically effective amount comprising 2 to 25 HLA tumor antigen peptides corresponding to MHC class I and/or class II complexes, arranged on at least one HLA tumor antigen polypeptide(s) according to the invention, and optionally determined by the method of the invention , and
(b) a second preparation comprising, together with a pharmaceutically acceptable carrier or diluent, an anticancer agent selected from the group consisting of anticancer alkylating agents, anticancer antimetabolites, anticancer antibiotics, herbal anticancer agents, platinum-coordinated anticancer complex compounds, anticancer camptothecin derivatives, anticancer tyrosine kinase inhibitors, monoclonal antibodies, interferons, interleukins, biological response modifiers, and other anticancer agents, or a pharmaceutically acceptable salt thereof.

In a preferred embodiment the second preparation is a monoclonal antibody, in particular against an immunosuppressive protein selected from the group consisting of CTLA4, GM-CFS, TReg, EpCam, IDO, MIC, PDL1, Fas and PD1-L, TRAIL.

The combination preparation according to the invention is particularly suitable for use in the treatment or profilaxis of carcinomas, in particular locally recurrent or metastatic mammary carcinomas in a subject or group of subjects who have or are suspected of having a carcinoma.

Particularly preferably, the first preparation of the combination preparation according to the invention is administered subcutaneously.

Particularly good experience has been made in the administration of the pharmaceutical composition according to the invention (first preparation) in combination with the administration of herbal anticancer drugs, in particular artesunate and/or curcumin as the second preparation.

Nevertheless, the combined administration of the pharmaceutical composition according to the invention (first preparation) in combination with a monoclonal antibody, in particular against immunosuppressive proteins selected from the group consisting of CTLA4 (herein e.g. Ipilimumab - Yervoy^{®}), PDL1 (herein e.g. Nivolumab - Opdivo), PD1-L, also EpCam, IDO, MIC, Fas and TRAIL; specifically: as estrogen inhibitor for hormone positive subjects from the group of aromatase inhibitors - (herein e.g. Letrozole and/or the estrogen blocker Fulvestrant); as antibody against HER2 positive subjects: Herceptin (TDM-1) as a second preparation.

In a particularly preferred embodiment, the drug combination may be used in conjunction with other cancer therapies. These may include, but are not limited to, surgical intervention, radiotherapy, hormone therapy, chemotherapy, or other individual therapies. For purposes of the present invention, a surgical procedure involves the removal of tumor masses or affected tissues from the body. Following such surgery, the drug may be administered to target remaining tumor cells to increase the overall effectiveness of the treatment. Radiation therapy in the context of the present invention uses high energy radiation to damage or kill cancer cells. Following radiation therapy, the pharmaceutical composition may be administered to target potentially radioresistant tumor cells, providing a comprehensive treatment approach. Hormone therapy, as conceived in the present invention, is used for cancers that are sensitive to hormones and is aimed at stopping the production of, or blocking the action of, hormones that promote these cancers. When combined with hormonal therapy, the pharmaceutical composition can provide a dual approach that targets both the hormonal aspect and specific HLA tumor antigens. Finally, chemotherapy, as understood in the context of the present invention, uses drugs to kill or inhibit rapidly dividing cancer cells. After chemotherapy, the introduction of drugs may target residual or resistant tumor cells to increase the overall efficacy of treatment. In one embodiment, the pharmaceutical composition according to the invention comprising HLA tumor antigen polypeptides is used as a sole therapy. By focusing the specific tumor genes through the HLA antigen peptides, the technical effect is that the therapy has fewer off-target effects and thus potentially fewer side effects than some other therapies, particularly much more non-specific conventional chemotherapies. The technical advantage of this approach is its precision, which may provide subjects with a better tolerated treatment with fewer systemic complications.

In another embodiment, the pharmaceutical composition is administered after a surgical procedure. In this case, after the majority of the tumor mass has been physically removed, the immunotherapy acts on any residual or microscopic tumor cells that may have remained. The technical advantage of this combined approach is a potential reduction in cancer recurrence rates, allowing for a two-pronged attack on cancer.

In another embodiment, the pharmaceutical composition is used after radiation therapy. After radiation has damaged or killed many tumor cells, immunotherapy can then target surviving cells that may be more resistant to radiation. The technical advantage here is to target radiation-resistant populations, aiming for more comprehensive tumor cell eradication and potentially preventing or delaying relapse.

In another preferred embodiment, where the pharmaceutical composition of the invention is administered after chemotherapy, the combination aims to increase overall efficacy. The chemotherapy may reduce the overall tumor burden, and the subsequent immunotherapeutic approach may target residual or resistant cells. The technical advantage of this approach is the potential synergy between the cytotoxic effects of chemotherapy and the targeted immune response elicited by HLA tumor antigen polypeptide-based therapy, which may result in increased overall survival and improved quality of life for subjects.

Also disclosed is a business method comprising marketing 2 to 25 HLA tumor antigen peptides corresponding to MHC class I and/or class II complexes, arranged on at least one HLA tumor antigen polypeptide(s) for the treatment of cancer in a human to effectively reduce a tumor marker score of a subject or group of subjects having at least one identical HLA allele, in particular to increase progression-free survival or reduce the likelihood of cancer recurrence or increase subject survival. In some embodiments, the marketing is followed by treatment of the subject with the combination of HLA tumor antigen peptides.

### Content factors for tumor progression

It is also important for the ranking to identify factors that play a significant role in tumor progression (i.e., the increase in size and/or metastasis of tumors). Characteristic of tumor progression is an increased growth rate, as well as increased invasiveness of the tumor.

Invasiveness refers to the extent of tissue-penetrating growth of a malignant tumor from its site of origin into adjacent tissue structures.

Angiogenesis describes the formation of new blood vessels from an existing vascular system and is a component of both physiological processes (e.g. embryogenesis, wound healing) and pathological processes (e.g. diabetic retinopathy, chronic polyarthritis, tumor growth). It has been known for a long time that new vessel formation (angiogenesis) occurs in cancer, which is referred to as tumor angiogenesis. Tumors are composed of cells that, like all other cells in the body, require nutrients and oxygen. In fact, because cancer cells divide frequently, their need is particularly high. And that is why a tumor needs its own blood vessels.

When a tumor develops, it initially does not yet have its own blood vessels. Its growth is therefore severely restricted. Without its own blood vessels, it does not grow larger than 1 to 2 millimeters. The formation of metastases also interacts with tumor angiogenesis, because tumor cells must reach surrounding blood vessels to do so. Only then can they be transported to distant regions of the body and form metastases there.

For example, it is known that there is an interdependence between class I HLA and integrin β to stimulate signal transduction and cell proliferation. In this regard, integrin β-mediated cell migration depends on its interaction with class I HLA molecules (Zhang and Reed, Hum Immunol. 2012 Dec, 73(12), 1239-1244).

An HLA peptide of the invention, or a composition or formulation containing the same, may be used for modulating a class I and/or class II HLA complex, including combinations thereof, either in *vitro* (e.g., in an in vitro or cellular detection method) or *in vivo* (e.g. in a unicellular or in a multicellular organism and in particular in a mammal and more particularly in a human being, such as a human being at risk of developing or suffering from a cancer of the invention).

In the context of the present invention, "modulating" or "modulating" substantially means increasing the specific affinity of T cell towards a tumor-exclusive or tumor-associated MHC class I complex or MHC class II complex, respectively, of the malignant and/or neoplastic tissue, as measured by an appropriate *in vitro,* cellular or *in vivo detection method* (such as those referred to herein). In particular, "modulate" or "modulate" means to increase the specific affinity of endogenous T cells of the subject or group of subjects, respectively, towards a tumor-exclusive or tumor-associated MHC class I complex or MHC class II complex of the malignant and/or neoplastic tissue, by at least 1 %, preferably at least 5%, such as 10% or at least 25%, for example by at least 50%, at least 60%, at least 70%, at least 80% or 90% or more compared to the affinity of T cells towards a tumor-exclusive or tumor-associated MHC class I complex or MHC class II complex of the malignant and/or neoplastic tissue in the same detection method under the same conditions but without the presence or prior application of the HLA tumor antigen peptides of the pharmaceutical composition of the invention (as defined herein).

In a particular embodiment, the invention comprises a method, wherein the expression profile of at least five, preferably at least six, most preferably at least 6 marker genes is determined. As mentioned above, the marker genes are also defined by variants, this expression profile is compared to the expression profile of a "reference". For example, a reference can be the expression profile of healthy tissue (e.g., intestinal tissue or tissue of the liver, lung, etc.). Tissue from the affected individual (proband) can be used as "healthy tissue" here, whereby this tissue is known not to be proliferatively altered or even metastatic. Appropriate examples are shown in the experimental part of the invention. However, data from tissues of foreign individuals, preferably healthy individuals, can also be used as a "reference" or "reference value".

As set forth herein, according to the invention, in the method for the detection of a carcinoma, at least 6, but preferably at least 8, and most preferably at least 10 of the HLA tumor antigen peptides shown herein corresponding to MHC class I complexes or MHC class I complexes are to be determined.

### Click Chemistry Approach

The invention also relates to a click chemistry HLA tumor antigen polypeptide corresponding to a HTAPP according to the current invention, wherein the HTAPP is provided as a first and a second segment, each independently preferably corresponding to HTAPs, wherein
- the first segment comprises a first click chemistry group bound to the first segment, preferably via a linker group, at the C-terminus, and
- the second segment comprises a second click chemistry group bound to the second segment preferably via a linker group, at the N-terminus,
wherein the click chemistry HLA tumor antigen polypeptide is provided by a click chemistry reaction before being used in a pharmaceutical composition as disclosed herein. This has the technical advantage that the HTAPPs can be separated into smaller, more cost-effective and easier to synthesize peptide segments and the introduction of linker groups which may enhance the solubility of the peptides while also being able to be cleaved inside of a cell. This combines the advantages of the HTAPPs as described herein, especially the higher pharmacological efficiency compared to short HTAP peptides with easier production and provision. Further advantages may include higher stability for storage of the segments and the possibility to match different first and second segments to create new click chemistry HTAPPs in the spirit of the current invention. In addition, the production yield could be higher with this approach because industrial peptide synthesis, e.g. using stationary phases such as solid phase peptide synthesis (Merrifield synthesis), gives a lower overall yield with increasing peptide length, since the overall yield is calculated by y^{length}, where length is the reaction steps corresponding to the amino acid length of a peptide and y is the yield of each individual step, which is typically in the range of 95 to 99%. Thus, a 20 amino acid long peptide has an overall yield of 54% (97% yield per step), and a 40 amino acid long peptide only has a yield of 29% (97% yield per step), so providing a HTAPP as a click chemistry HTAPP can significantly improve the overall yield.

Two antagonistic click chemistry groups, such as the first and the second click chemistry groups may be referred to as click chemistry group pair or simply as click chemistry pair.

In an alternatively preferred embodiment of the invention a click chemistry HLA tumor antigen polypeptide corresponding to a HTAPP-DACP according to the current invention is disclosed, wherein the HTAPP-DACP is provided as a first, a second segment, each independently preferably corresponding to HTAPs, and a third segment, corresponding to a DACP, preferably DACP-1 to DACP-25, wherein
- the first segment comprises a first click chemistry group bound to the first segment, preferably via a linker group, at the C-terminus, also denoted as R¹, and
- the second segment comprises a second click chemistry group bound to the second segment, preferably via a linker group, at the N-terminus, also denoted as R², as well as a third click chemistry group bound to the second segment, preferably via a linker group, at the C-terminus, also denoted as R³, and
- the third segment comprises a fourth click chemistry group bound to the third segment, preferably via a linker group, at the *N*-terminus, referred also denoted as R⁴,
wherein the first and the second click chemistry group are antagonistic to each other and the third and the fourth click chemistry group are antagonistic to each other and orthogonal to the first and second click chemistry group, and wherein the click chemistry HLA tumor antigen polypeptide is provided by a click chemistry reaction before being used in a pharmaceutical composition as disclosed herein. This allows for a step-wise assembly of HTAPP-DACP peptides according to the current invention and a modular approach, where a library of different building blocks can be used to assemble customized combinations of peptides which match the tumor gene and HLA alleles of a specific patient and/or group of patients specifically by combining different HTAP blocks and different DACP to form HTAPP-DACPs in the spirit of the current invention. In addition, the production yield could be higher with this approach because industrial peptide synthesis, e.g. using stationary phases such as solid phase peptide synthesis (Merrifield synthesis), gives a lower overall yield with increasing peptide length, so providing a HTAPP-DACP as a click chemistry HTAPP-DACP can significantly improve the overall yield.

In some preferred embodiments of the current invention, the HTAPPs may be separated into smaller, in general pharmacologically less effective fragments, also referred to as segments when bound to a linker and a click chemistry group according to the current invention. The fragments match MHC class I and/or II complexes and HLA alleles as well as tumor genes. Examples of separation points can be points where no or only some, such as 1 or 2 HTAPs overlap on the HTAPPs or where tandem HTAPPs are connected directly or by a linker, e.g. AYY. The segments comprising the fragments may correspond to HTAPs and/or smaller HTAPPs. These segments then are coupled with two distinctive groups R¹ and R², which each independently represent click chemistry groups bound to a segment preferably via a linker group according to the current invention, wherein the linker group for example comprises AYY, AAY, GPGPG, which may be cleaved enzymatically or chemically inside of a cell, or 8-amino-3,6-dioxaoctanoic acid (PEG₂) and elongated PEGₙ (2 ≤ n ≤ 10), which increase the flexibility of the peptide chain and may improve the solubility of the segment. The click chemistry groups, in particular the first and the second and/or the third and the fourth click chemistry group, are antagonistic to each other, i.e. they react under the conditions of the click chemistry reaction with high yields, preferably more than 90%, and high selectivity, preferably more than 80%, the selectivity referring to the percentage of the click chemistry reactions in the total reactions (comprising the click chemistry reactions and all side reactions of the first and second and/or the third and fourth click chemistry groups involved).

The technical advantage of this approach lies in the modular design, allowing for the HTAPPs to be segmented into smaller, more easily synthesized peptides, enhancing solubility through linker groups and enabling a tailored assembly for specific pharmacological targets. The segments are designed to match MHC class I and/or II complexes, HLA alleles, and tumor genes, with separation points strategically selected to optimize pharmacological efficacy and versatility in design.

Orthogonal in the context of the click chemistry groups according to the invention means that click chemistry group pairs which are antagonistic to each other are not antagonistic to each other with click chemistry groups from other pairs, i.e. they do not react with an orthogonal click chemistry group under the conditions of click chemistry. Preferred examples are the *N*-maleimide and cysteine pair to the azide and alkyne pair, or the maleimide and cysteine pair to the tetrazine and alkene pair, or the azide and alkyne pair to the tetrazine and alkene pair. Not reacting in this context means that the reaction constant k of a click chemistry group with an orthogonal group is at least 15, preferably at least 5 orders of magnitude lower than the reaction rate of the same group with an antagonistic group. This configuration allows the first and second groups of click chemistry to be antagonistic, as well as the third and fourth groups, with the latter pair also being orthogonal to the first and second groups, allowing a versatile approach to peptide assembly. This orthogonal property means that the respective groups do not react under the defined conditions of click chemistry, ensuring a targeted and efficient assembly process.

In some embodiments, a DACP can also be linked with a linker group and a first or second click chemistry group and reacted with a HTAPP modified on the C-terminus with an antagonistic first or second click chemistry group and optionally a linker group. This has the advantage that different DACP, preferably DACP-1 to DACP-25, can be attached to a specific HTAPP according to the invention. The attachment of a DACP allows a medium increase in ELIspot-response of about 30% compared to unmodified HTAPPs according to the current invention.

### Click chemistry groups

According to a preferred embodiment of the click chemistry HTAPPs comprised by the current invention, the first and second and/or third and fourth click chemistry groups are selected in pairs from the list comprising or consisting of N-maleimide and cysteine group, azide and alkyne group, tetrazine and alkene group.

The chemical structure of some preferred antagonistic click chemistry groups is shown below. A preferred pair of antagonistic click chemistry groups comprises N-maleimide, preferably attached to the C-terminus of a peptide sequence corresponding to a fragment of a HTAPP, reacting in a click chemistry reaction, in particular in a Brønsted- or Lewis acid-catalyzed Michael addition (1, 4), with an HS group of a cysteine preferably bound to the N-terminus of a peptide sequence corresponding to a fragment of a HTAPP, or alternatively preferably with a non-terminal cysteine comprising the backbone of a HTAPP peptide fragment. The cysteine may itself be bound to a small peptide (1 to 5 amino acids) in addition to the linker according to some embodiments. This has the technical effect that a highly selective reaction is formed and at the same time a covalent bond is formed between the first and second segments, resulting in a HTAPP as defined by the present invention.

### Michael-addition-pairs:

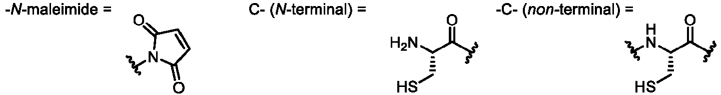

A further preferred pair of antagonistic click chemistry groups comprises an azide, preferably in the form of a terminally bound modified amino acid, most preferably selected from the group containing 3-azido-L-alanine (AzA), 4-azido-L-homoalanine (AzHA), 4-azido-L-phenylalanine (AzF), 6-azido-L-lysine (AzK), 4-azido-L-proline (AzP) or protected derivatives thereof, and an alkyne, preferably in the form of a terminally bound modified amino acid and/or modified amino acid, most preferably selected from the group containing L-homopropargylglycine (HPrG), L-propargylglycine (PrG), L-propargyltyrosine (PrY, CAS: 610794-20-2), or protected derivatives thereof, or a DBCO- group bound to a peptide, for example DBCO-amine (DBCOam, CAS 1255942-06-3) or dibenzocyclooctyne-acid (DBCOac, CAS: 1353016-70-2). These antagonistic click chemistry groups enable a selective and highly efficient Cu(I)-catalyzed or even uncatalyzed [3+2] cycloaddition yielding stable 1,2,3-triazole products. This enables selective binding of first and second segments according to the invention to obtain stable click chemistry HTAPPs according to the present invention. DBCO groups can react with azide without the need for room temperature copper catalysis, allowing for easier processing, and can be advantageously attached to the N-terminus (DBCOac) or C-terminus (DBCOam) of a peptide or a linker according to the present invention.

### [3+2] cycloaddition pairs:

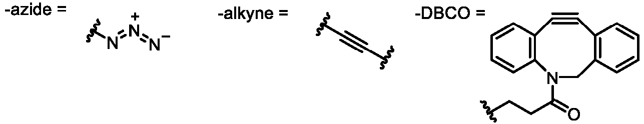

A further preferred pair of antagonistic click chemistry groups comprises an alkene, preferably a trans-cyclooctene (TCO), (E)-cyclooct-4-en-1-yl carbonyl- (TCOC) or norbornene group, bound to an amino acid (for example by the means of a amide bond formation with a TCO-NHS ester (CAS: 1191901-33-3) or a TCO-amine (CAS: 1800507-94-1)); and an tetrazine, preferably bound to an amino acid or as modified amino acid, most preferably selected from the list comprising L-Phe(4-MeTz) (TzF, CAS: 1698038-85-5) or (Me)Tz-butanoic acid (TzBa) CAS: 1923268-81-8). These antagonistic click chemistry groups enable a selective and highly efficient inverse electron demand Diels Alder (IEDDA) reaction ([4+2] CA) followed by a retro-DA reaction to eliminate nitrogen gas, leading to an irreversible covalent bond between the first and second segment of the click chemistry HTAPP. This reaction can be conducted with high selectivity and in aqueous media.

### [4+2] cycloaddition (Diels-Alder) pairs:

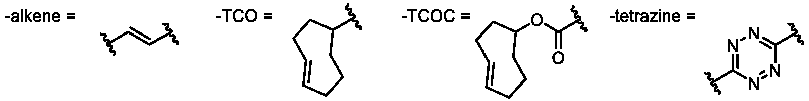

### Linker

According to some preferred embodiments of the invention, the antagonistic chick chemistry groups may be bound to a linker, which may be a short amino acid sequence and/or a small molecule, preferably with a molar mass M between 40 and 500 g/mol, more preferably between 40 and 400 g/mol, which increases the flexibility of the click chemistry group and increases the solubility of the segments in water, which is important for high yields under click chemistry reaction conditions. In some preferred embodiments the linkers are chosen in a manner which allows the segments to be separated (cleaved) under biochemical conditions inside of a cell in order to allow the separated segments to be cleaved, in these embodiments the cleavers are preferably amino acid sequences which are known to be cleaved inside of cells, most preferably AAY, AYY and GPGPG. The linker group comprised by the first or second segment is preferably selected independently from the list comprising PEGₙ (2 ≤ n ≤ 10), AAY, AYY, GPGPG, more preferably AAY and PEG₂ and comprises preferably at least one cleavable linker, preferably chosen from AAY, AYY, and GPGPG. This has the advantage of providing an enzymatically and/or chemically cleavable separating spot, where the click chemistry HLA tumor antigen polypeptide can be cleaved inside a cell to be processed into the corresponding MHC class I and/or class II complexes as described herein.

The following shows the chemical structure of the PEGₙ-Linker:

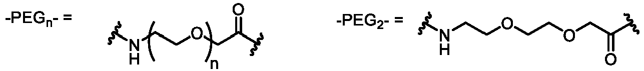

In some further preferred embodiments, the length of the PEG₂ linker may be elongated by incorporating further oxyethylene groups, also referred to as PEGₙ, wherein n = 2 corresponds to PEG₂ and elongated PEG₂ Linkers may include between n = 2 and n = 15, more preferably between n = 2 and n = 10 oxyethylene groups, such as 2, 3, 4, 5, 6, 7, 8, 9, 10 oxyethylene groups. This enables greater flexibility and solubility of the segments in water and can be used to increase the yield of the reaction. One key advantage of PEGₙ linkers is the ability to attach the linker booth to the N-terminus and/or the C-terminus of a peptide, such as a HTAPP fragment. In addition, the linker is already used in a peptide-based therapeutic, semaglutide, and has thus shown to be biocompatible.

In some most preferred embodiments, linkers may be combined, for example a AAY and/or GPGP cleavable linker with a PEGₙ linker. This enables higher solubility in water or water/cosolvents and at the same time provides a cleavage site where the HTAPP or HTAPP-DACP fragment is released and can be processed into an MHC class I and/or MHC class II complex. Exemplary configurations might be HTAP¹-AAY-PEG₂-click chemistry group or click chemistry group-PEG2-AAY-HTAP².

Some further, exemplary combinations of first and second click chemistry groups and/or the third and fourth click chemistry groups bound to the segment via a linker group at the C- or the N- Terminus are shown below. The line indicates the binding site at which the linker is bound to the HTAPP fragment and thus forms the first or second segment. Each row represents a pair of antagonistic click chemistry groups and each forms a single exemplary embodiment of the invention.

### Michael-addition-pairs:

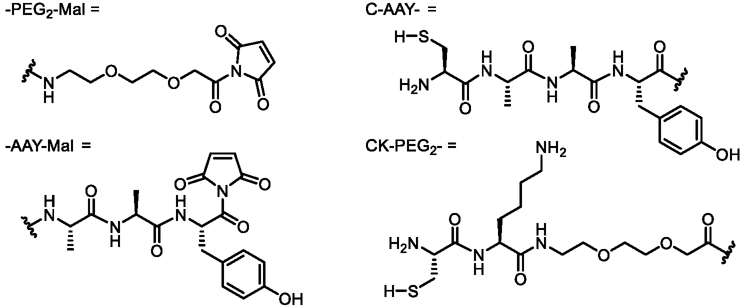

### [3+2] cycloaddition-pairs

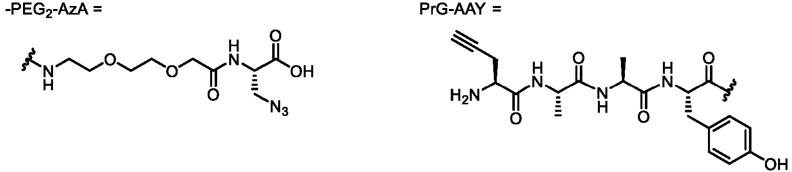

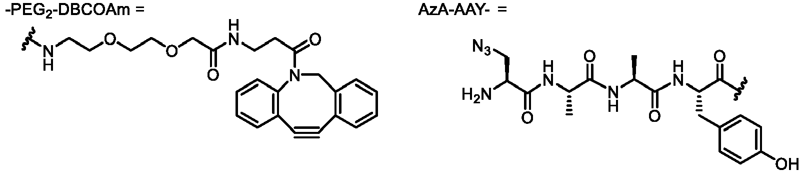

AzA refers to 3-azido-L-alanine and PrG to L-propargylglycine and DBCOAm to DBCO-amine.

### [2+2] cycloaddition-pairs

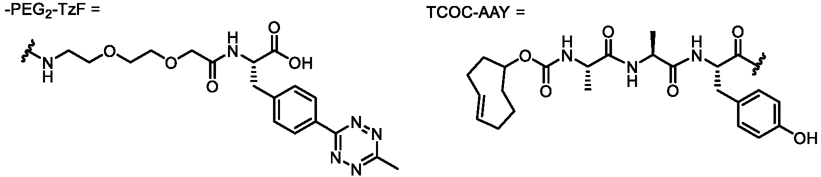

TzF refers to L-Phe(4-MeTz) and TCOC- to (E)-cyclooct-4-en-1-yl carbonyl-, the coupling product of the reaction of a terminal NH₂ group of the AAY- linker with a TCO-NHS ester (CAS: 1191901-33-3).

### Terminal Group

In some preferred embodiments of the current invention, the click chemistry HLA tumor antigen polypeptide comprises a chemically bound terminal group, wherein the HTAPP is provided as a first, a second segment, each independently preferably corresponding to HTAPs, and a third segment, corresponding to the terminal group, wherein
- the first segment comprises a first click chemistry group bound to the first segment, preferably via a linker group, at the C-terminus, also denoted as R¹, and
- the second segment comprises a second click chemistry group bound to the second segment, preferably via a linker group, at the N-terminus, also denoted as R², as well as a third click chemistry group bound to the second segment, preferably via a linker group, at the C-terminus, also denoted as R³, and
- the third segment comprises a fourth click chemistry group bound to the third segment, preferably via a linker group, at the *N*-terminus, referred also denoted as R⁴,
wherein the first and the second click chemistry group are antagonistic to each other and the third and the fourth click chemistry group are antagonistic to each other and orthogonal to the first and second click chemistry group, and wherein the click chemistry HLA tumor antigen polypeptide is provided by a click chemistry reaction, and wherein the terminal group is a lipide, preferably selected from the group comprising or consisting of saturated fatty acids, modified fatty acids, unsaturated fatty acids and their diacylglycerides.

A terminal group in the spirit of the current invention is defined as a low-molecular-weight chemical compound having a molar mass in a range from approximately 50 to 1500 g/mol, preferably from about 50 to 1250 g/mol, and most preferably from about 50 to 1000 g/mol, which is selected to enable improved delivery and physio-chemical properties of the peptide Such a terminal group is selected to enhance the delivery of the polypeptide as well as to facilitate targeting to specific tissues or cells. The terminal group is selected from the group consisting of or comprising saturated fatty acids, modified fatty acids, unsaturated fatty acids. Here, saturated fatty acids are defined as natural or synthetic carboxylic acids having an aliphatic chain, wherein the chain is preferably linear and saturated. Unsaturated fatty acids are similarly defined, except that one or more carbon-carbon single bonds are substituted by double bonds, which may be in either the E (trans) or Z (cis) configuration. Modified fatty acids, on the other hand, are carboxylic acids that have been chemically altered through processes such as oxidation, reduction, epoxidation, or dihydroxylation to introduce one or more functional groups. Modiefied fatty acids especially also comprise or consist of saturated or unsaturated fatty acids with modified terminal carboxyl-group, which is chemically bound to either a modified amino acid having a click chemistry group as disclosed herein or directly modified to comprise a click chemistry group antagonistic to the click chemistry group of the click chemistry HTAPP to be modified with the terminal group. The saturated, unsaturated and modified fatty acids of the current invention may be provided in the form of their diesters with the glycerin triol, which are referred to as diacylglycerides in the spirit of the current invention. This diacylglyceride has a free hydroxy group, which may be used to chemically bin a click chemistry group in the spirit of the current invention, either directly in the form of a modified amino acid or via a linker.

The diacylglyceride can be bound to a click chemistry group or a linker with a secondary hydroxy group of the glycerin, which in the spirit of this invention is referred to as "GlycS-", or via a primary alcohol, which is referred to as "GlycP-", as depicted with two general unsaturated acids CH₃(CH₂)ₙCOOH and CH₃(CH₂)ₘCOOH, wherein n or m are independently between 6 and 30, below:

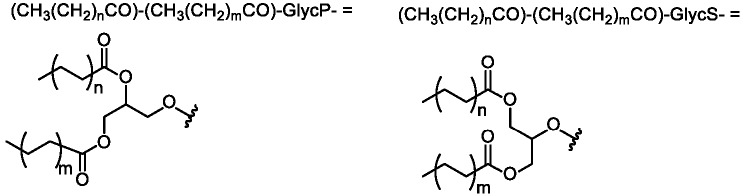

The terminal groups may be bound to modified amino acids. Some exemplary structures of first and second click chemistry groups and/or the third and fourth click chemistry groups bound to an generalized unsaturated fatty acid CH₃(CH₂)ₙCOOH or a diacylglyceride CH₃(CH₂)ₙ)-(CH₃(CH₂)ₘ)-Glyc- wherein n or m are independently between 6 and 30, and via a peptide bond are shown below:
**Michael-addition terminal groups:** 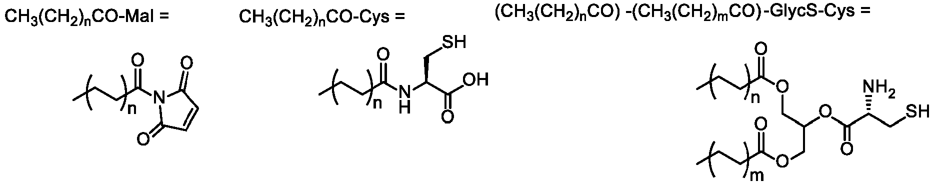
[3+2] cycloaddition terminal groups:

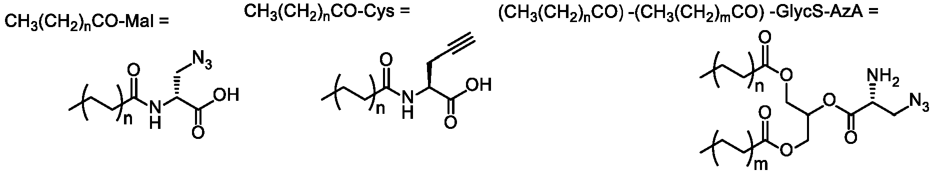

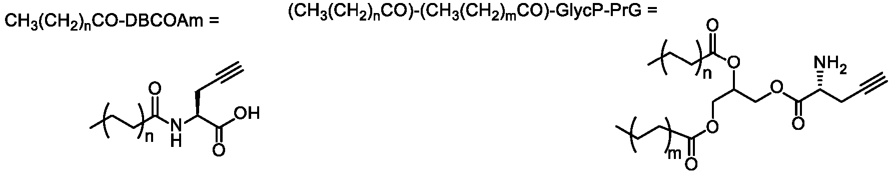

Preferably, saturated fatty acids have the general structural formula CH₃(CH₂)ₙCOOH, wherein n is between 6 and 30, preferably between 6 and 24, and are preferably selected from the list comprising or consisting of caprylic acid, capric acid, lauric acid, myristic acid, palmitic acid, stearic acid, arachidic acid, behenic acid, lignoceric acid, cerotic acid. These naturally occurring fatty acids are biocompatible and are accessible in high quality. Unsaturated amino acids are preferably derived from these saturated fatty acids and have at least one and up to the stoichiometric possible amount of unsaturated carbon-carbon bonds.

In another embodiment, the terminal group comprises a PEG sequence, which is known to increase the water solubility and circulation half-life of the polypeptide, thereby reducing immunogenicity and improving pharmacokinetic properties. In each case, the terminal group is conjugated to the antigen polypeptide via a suitable linker group. This linker group provides a chemical bridge that preserves the reactivity of the click chemistry functional groups while maintaining a spatial separation between the biologically active domain and the terminal group.

The incorporation of the terminal group into the click chemistry HLA tumor antigen polypeptide serves not only to enhance the pharmacokinetic profile of the polypeptide but also to provide improved targeting capability. By judiciously selecting the chemical nature of the terminal group, the present invention achieves an optimal balance between effective antigen presentation and efficient delivery to the desired cellular targets.

### Click chemistry reaction conditions

In some preferred embodiments of the click chemistry HLA tumor antigen polypeptide according to the current invention, the first click chemistry group is preferably a N-maleimide group and the click chemistry nucleophile is a, preferably terminally, cysteine HS- group. These end groups advantageously enable the covalent binding of the two segments to form a pharmacologically active click chemistry HTAPP according to the invention with high, preferably over 90%, yields when a catalyst, preferably a Brønsted or Lewis acid, is present.

The following shows the general click chemistry reaction of two HTAP segments, wherein R¹ is a first click chemistry group linked to the HTAPP fragment, here HTAP¹, via a linker group at the C-terminus, wherein R² is a second click chemistry group bound to the HTAPP fragment, here HTAP², via a linker group at the N-terminus. In general, the click chemistry reaction conditions may include a suitable catalyst to facilitate the reaction, preferably Cu(I) for [3+2] cycloadditions and a Lewis or Brønsted acid for Michael additions (1,4), and the reactions are preferably carried out between 0 °C and 30 °C, most preferably at room temperature, to minimize protein degradation and in the case of room temperature not requiring specialized reaction vessels with heating or cooling elements, and preferably in water or in a water/cosolvent mixture, such as water/DMSO, water/tert-butanol, water/DMF, water/ethanol, water/acetonitrile, water/2-MeTHF, water/tert-amyl alcohol, water/acetone, in a ratio of 9: 1 to 1:1. Preferably, all impurities, including organic/inorganic chemicals and by-products, reactants, segments and catalysts, are removed in a single chromatographic step, resulting in a GMP-like product. Copper side products can be removed with a copper chelating agent.
**1) one click chemistry group pair:** 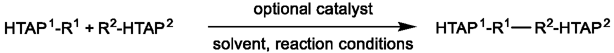 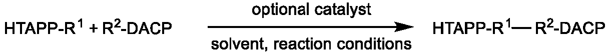 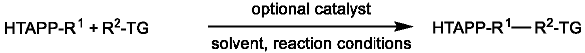
2) two orthagonal click chemistry group pairs:

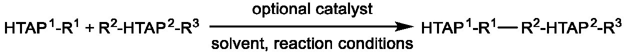

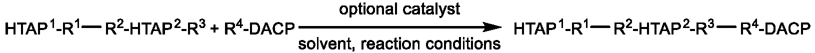

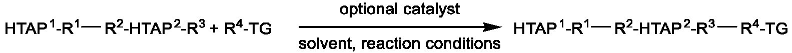

Herein, TG stands for terminal group and especially refers to a lipide, preferably selected from the group comprising or consisting of saturated fatty acids, modified fatty acids, unsaturated fatty acids and their diacylglycerides.

A reaction sequence under click chemistry reaction conditions exemplary reaction sequence representing a preferred embodiment of the click chemistry HTAPP is shown in the following reaction equation, wherein R¹ is a first click chemistry group linked to the peptide via a linker group at the C-terminus, in this case -PEG₂-Mal, wherein R² is a second click chemistry group bound to the peptide via a linker group, in this case CK-PEG₂-, wherein the reaction is a Lewis or Brønsted-acid catalyzed Michael-addition (1,4).

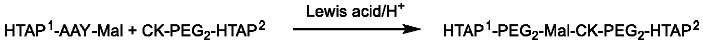

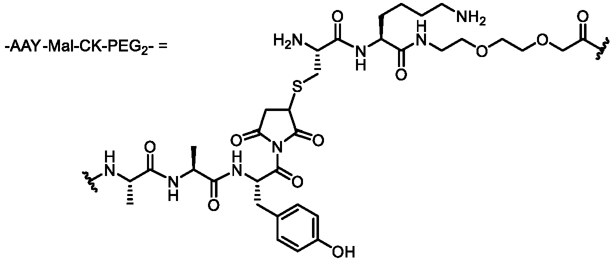

A further reaction sequence under click chemistry reaction conditions representing a preferred embodiment of the click chemistry HTAPP is shown in the following reaction equation, wherein R₁ is a first click chemistry group attached to the peptide via a linker group at the C-terminus, wherein the functional group is an azido- (N₃) group, in this case -PEG₂-AzA, wherein R₂ is a second click chemistry group, attached to the peptide at the N-terminus via a linker group, wherein the functional group is an alkyne group, in this exemplary case the amino acid PrG- attached to -AAY-, wherein the click chemistry reaction is a copper(I)-(Cu(I)-catalyzed [3+2]-cycloaddition to a 1,2,3-triazole with high selectivity for the 1,4-disubstituted regioisomer.

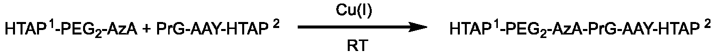

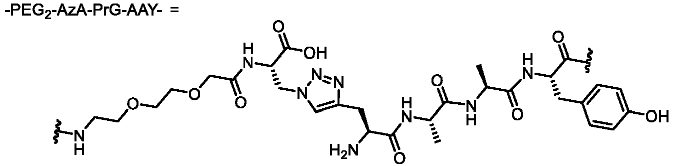

A further reaction sequence under click chemistry reaction conditions representing a preferred embodiment of the click chemistry HTAPP is shown in the following reaction equation, wherein R₁ is a first click chemistry group attached to the peptide via a linker group at the C-terminus, wherein the functional group is an alkene, in this case a trans-cyclooctene bound to a AAY- short peptide linker (TCO-OCO-AAY-), wherein R₂ is a second click chemistry group, attached to the peptide at the *N*-terminus via a linker group, wherein the functional group is an tetrazine group, in this exemplary case the amino acid *L*-Phe(4-MeTz)- (TzF) attached to a -PEG₂- linker, wherein the click chemistry reaction is a highly efficient inverse electron demand Diels Alder (IEDDA) reaction ([4+2] CA) followed by a retro-DA reaction to eliminate nitrogen gas.

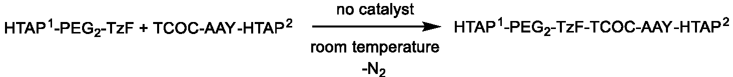

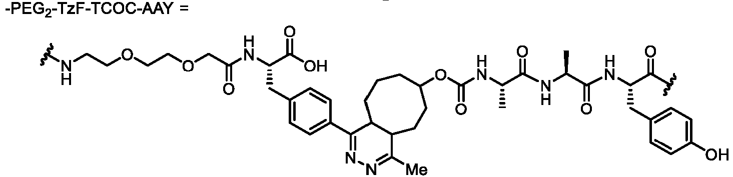

The invention also relates to a kit, for the provision of a **pharmaceutical composition** for use as a medicament, in particular for use in the therapeutic and/or prophylactic treatment of a carcinoma, particularly preferably a locally recurrent or metastatic carcinoma, in particular in a subject or group of subjects suffering from or at risk of suffering from a carcinoma, comprising at least
- a first vial comprising a first segment of a click chemistry HLA tumor antigen polypeptide according to the invention in a carrier solution,
- a second vial comprising a second segment of a click chemistry HLA tumor antigen polypeptide according to the invention in a carrier solution, and
- preferably a third viral comprising a third segment of a click chemistry HLA tumor antigen polypeptide delivery aiding capping peptide (DACP) according to the invention in a carrier solution, and
- a reaction vessel comprising reaction solution and preferably a catalyst. This makes it easy to distribute the pharmaceutical composition to authorized and trained users, such as medical staff in hospitals or pharmacies. By producing the HTAPPs and HTAPP-DACPs on site, formulations can be customized and manufacturing costs can be kept low.

A suitable reaction vessel may be a mixing vial made of chemically inert material, such as perfluoropolymers, PET or borosilicate glass.

The invention further relates to a method for the preparation of a HTAPP and/or HTAPP-DACP from click chemistry and/or a pharmaceutical composition for use as a medicament, comprising the same, from a first, second and preferably third segment via a click chemistry reaction, followed by a chromatographic isolation to yield the individual HTAPP and/or HTAPP-DACP as

### Brief description of the drawings

Herein shows:
**Fig. 1**: Schematic representation of HLA-A-mediated binding of a T cell receptor to a class I MHC molecule, showing the anchor (amino acid) residues of the HLA-A antigen peptide (7-11 amino acids in length).
**Fig. 2****:** Schematic representation of HLA-B-mediated binding of a T cell receptor to an MHC molecule, showing the anchor (amino acid) residues of the HLA-B antigen peptide (12-17 amino acids in length).

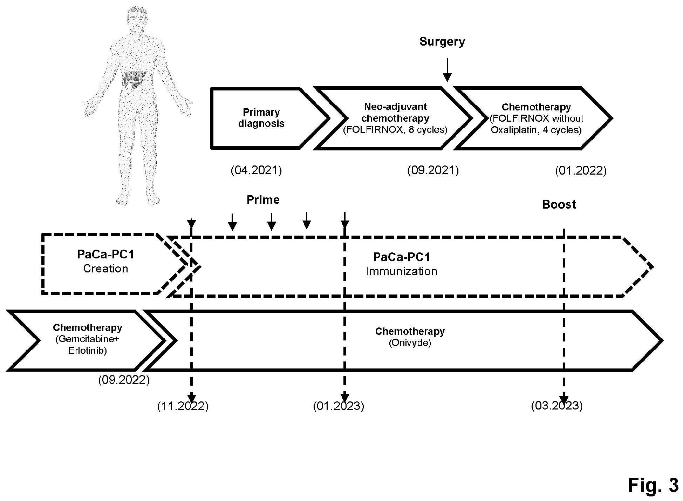

**Fig. 3**: Therapeutic scheme for the treatment of a subject with prostate cancer with a pharmaceutical composition in accordance with the current invention (example 3).

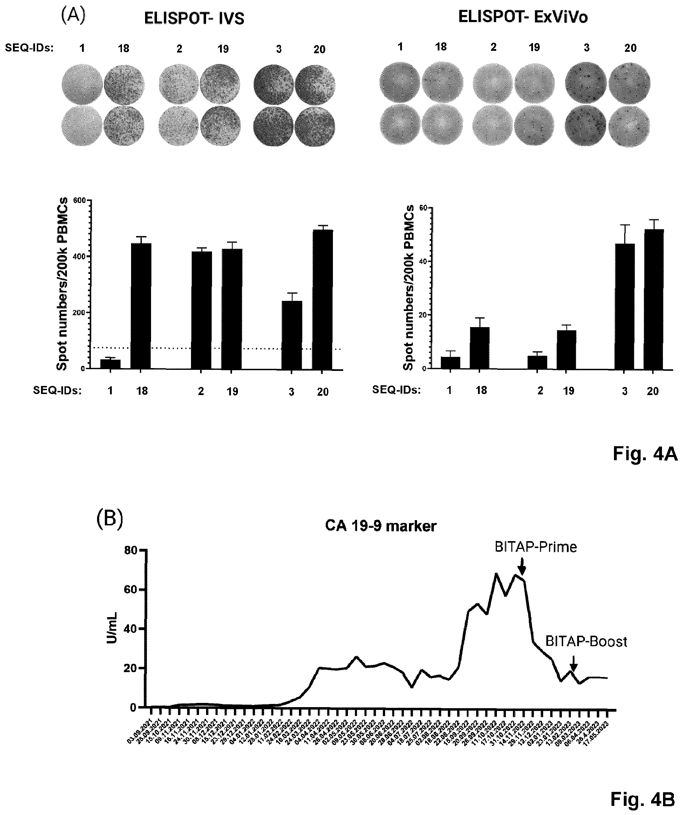

**Fig. 4A****:** IFN-γ ELISPOT assay results of the selected peptides SEQ-ID-Nos.: 1, 18, 2, 19, 3, 20. Depicted Plates and Spot Numbers/200k PBMCs. Elispot-IVS (In Vitro Stimulation) and Elispot-ExViVo (Ex Vivo) Immunoassays depicted.
**Fig. 4B****:** CA19-9 cancer marker levels of the subject throughout the treatment, initial immunization (1) and immunization boost (2) with the pharmaceutical composition in accordance with the current invention marked.

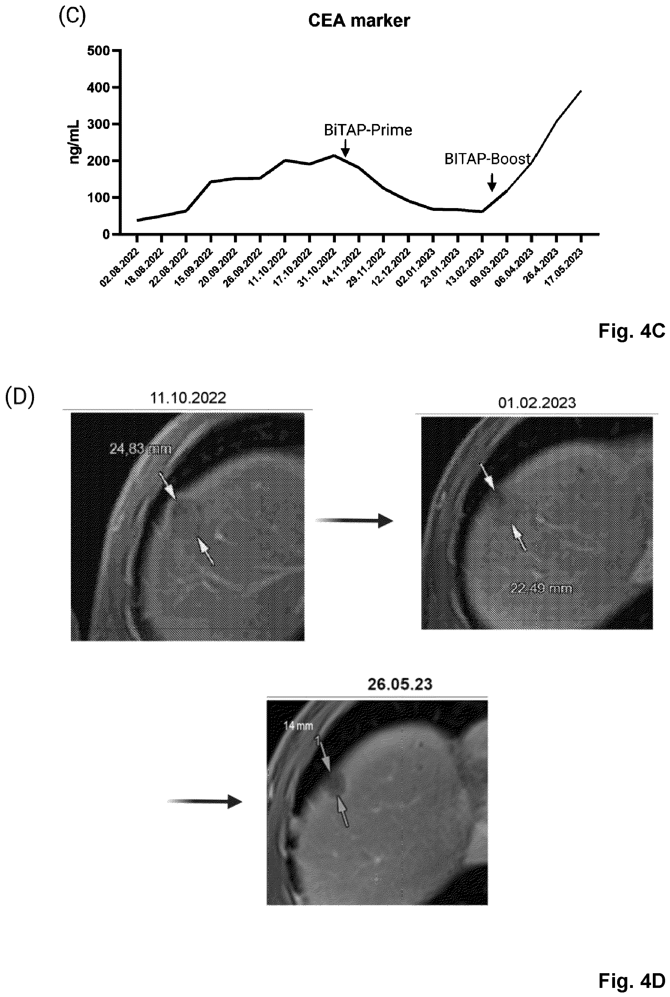

**Fig. 4C****:** CEA marker levels of the subject throughout the treatment, initial immunization (1) and immunization boost (2) with the pharmaceutical composition in accordance with the current invention marked.
**Fig. 4D****:** MRI scan was performed before and approximately three and six months after the start of PrCa-PC1 immunization. Decrease of the tumor von 24,83 mm to 14 mm could be detected as consequence of the treatment with the pharmaceutical composition.

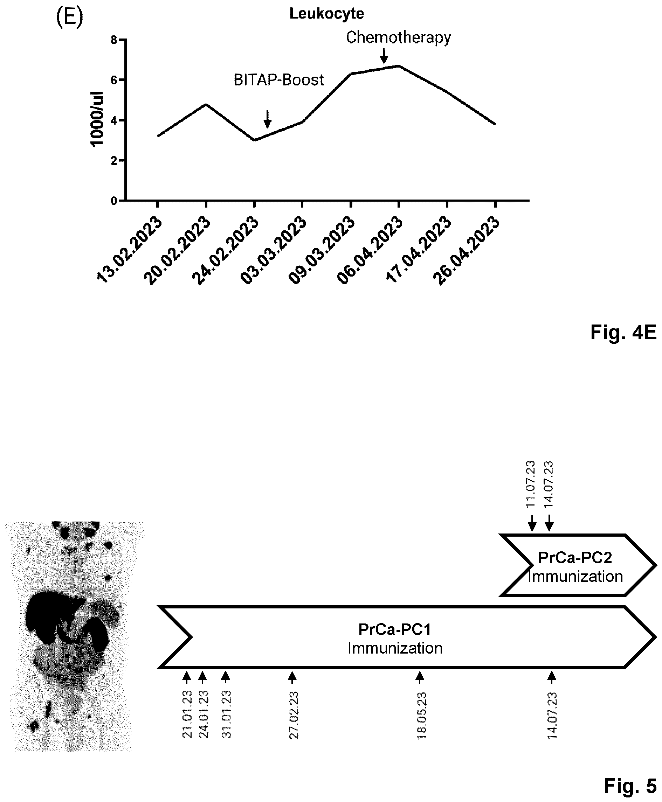

**Fig. 4E****:** Immuno-monitoring of the subject during the treatment, immunization boost (2) with the pharmaceutical composition in accordance with the current invention and chemotherapy (3) marked.
**Fig. 5**: Therapeutic scheme for the treatment of a subject with prostate cancer with bone metastasis with a pharmaceutical composition as sole therapy in accordance with the current invention (example 4).

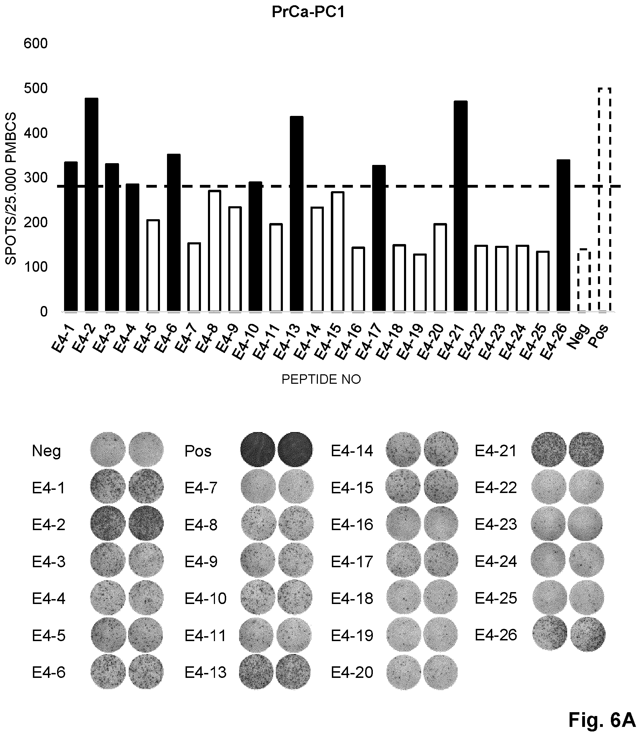

**Fig. 6A** IFN-γ ELISPOT assay results of the selected peptides SEQ-ID-Nos.:E4-1 to E4-11, E4-13 to E4-26 and negative and positive control. Depicted Plates and Spots/25.000 PBMCs.

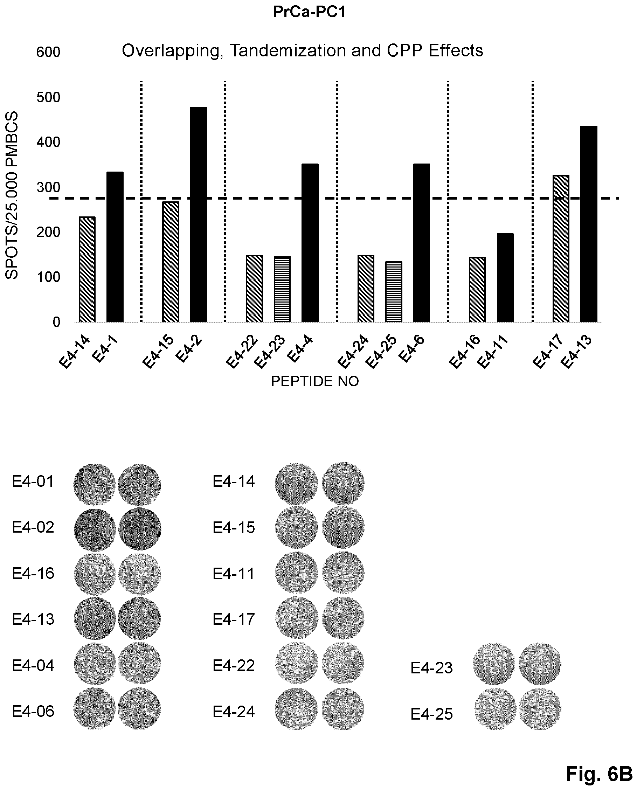

**Fig. 6B** IFN-γ ELISPOT assay results of the selected peptides SEQ-ID-Nos.:E4-1 to E4-11, E4-13 to E4-26 and negative and positive control. Depicted Plates and Spots/25.000 PBMCs, results of the modifications of the HTAPPs and HTAPs according to the current invention (elongation, multimerization, tandemization and delivery aiding capping peptide addition).
**Fig. 7** IFN-γ ELISPOT assay results of the PrCa-PC2 pharmaceutical composition and negative control. Depicted Plates and Spots/Million.
**Fig.8A** PSA level of the subject during the time of subject diagnosis and treatment with a pharmaceutical composition according to the invention (PrCa-PC1).
**Fig. 8B** Subject examinations before and after treatment with a pharmaceutical composition according to the invention (3 months) (determined by PSMA-PET CT).
**Fig. 8C** Examinations before and after treatment (8 months) with a pharmaceutical composition according to the invention (determined by MRI).
**Fig. 9** Demonstrates the increased pharmaceutical efficacy (ca. 30%) of the immunogenically tested HTAPP(s) and HTAPP-DACP(s) of this disclosure compared to their corresponding HTAP(s) that were not tandemized or overlapped or bound to a delivery aiding capping peptide.

### Design examples

With reference to the following figures and examples of embodiments, the present invention will be explained in more detail without limiting the invention thereto.

The following tables list HLA tumor antigen polypeptides, all of which have been tested and are immunogenic. SEQ-ID-Nos.: 1 to 84 list some preferred, but not limiting, examples of amino acid sequences of HLA tumor antigen polypeptides of the invention, each of which is another embodiment of the present invention. The sequences marked with ref. (reference) are included for illustrative reasons and may not represent sequences of the invention.

**Table 1. List of preferred HLA tumor antigen polypeptides (HTAPPs), their respective corresponding HLA alleles (HTAPs) and mutated genes, their respective related gene name, cancer type, amino acid substitution and response in ELISPOT experiments (PaCa pancreatic cancer, LMCa liver metastasis; BMCa bone cancer metastasis, PrCa prostate cancer).**

| SEQ-ID-No.: | HLA Tumor Antigen Polypeptide | Gene/s | corresponding HLA allele (HTAPs) | Substitution AA | Elispot [nₛₚₒₜₛ/pM] | Corr. carcinoma |
|---|---|---|---|---|---|---|
| 1 | KLVVVGAVGVGKSALTI | KRAS | A*02:786; C*03:04; C*06:02 | G12V | 32,5 | PaCa/LMCa |
| 2 | LANSPDLSHLAYYVTKVKEEDAF | ADAM8-NUP50 | C*06:02; B*57:37 | A399S...E351K | 419,5 | PaCa |
| 3 | LQEFRRFQDPGRIVAIV | CEMIP2 | DQA1*01:01; DQB1*02:01 | L301R | 246,0 | PaCa |
| 4 | ETNFKSLEHDLFQKVEE | SMC4 | A*02:01; DQA1*01:01-DQB1*05:01 | V576F | 233,5 | BMCa/PrCa |
| 5 | IKPRILGFDTPHYWLC | KDM3A | DRB1*15:01; DQA1*01:01; DQB1*05:01 | S936R | 267,5 | BMCa/PrCa |
| 6 | NVGLSAVCTYNLSTAAAYCIHSINLHNFSNSVL | SEMA4D-SEMAD4D | DRB1*01:01 | A327T...R77C | 330,5 | BMCa/PrCa |
| 7 | MLFSNITPKAAYSPLTGGNMAF | TNC-LAMC1 | A*03:01; B*07:02; B*35:01 | T1170M..V164M | - | BMCa/PrCa |
| 8 | KKIRKPRTIYSSLQLQAAAYGIYDALFDIESKVDPSK | DLX1-FOLH1 | A*02:01; B*07:02 | TAA | 351,5 | BMCa/PrCa |
| 9 | LPVMLLIVARPVKLAAFPTSLSD | TMEFF2 | DRB1*15:01; A*02:01; A*03:01; B*07:02, C*07:02 | TAA | 289,5 | BMCa/PrCa |
| 10 | LFKDLGLPARTVSTTF | DIP2A | B*07:02; B*35:01; DRB1*01:01 | E33Q | 143,5 | BMCa/PrCa |
| 11 | SPLTGGNMAF | LAMC1 | B*07:02; B*35:01 | TAA | 145,5 | BMCa/PrCa |
| 12 | IPDEYGNTALHYAIYNEDKLM | POTEG; POTEH | B*07:02; B*35:01; C*04:01; DQA1*01:01-DQB1*05:01; DQA1*01:01-DQB1*06:02 | TAA | - | PrCa |
| 13 | LSKNLVAQISALALQLAAYGNSITGIISSVLGHIS | RIF1-RIF1 | DRB1*01:01; DRB1*15:01 | L/V; G/S | 205 | BMCa/PrCa |
| 14 | SPRQCSSWTLAAYKLAAFPTSL | TMEFF2-L-TMEFF2 | A*02:01; B*07:02 | TAA | 137,5 | BMCa/PrCa |
| 15 | HRCATITHLLAAYYLVLNFNKK | ABLIM1, PLD3 | A*03:01; C*07:02 | R54C...P471L | - | PrCa |
| 16 | RERRVASWAMSFERL | RGS12 | C*07:02; DQA1*01:01-DQB1*05:01 | V66M | 326,5 | BMCa/PrCa |
| 17 | QSYPGSASLAAYSGMPRISKL | DXL1, FOLH1 | E*01:01; E*01:03 | TAA | 202,5 | PrCa |

**Table 2. List of preferred HLA tumor antigen polypeptides with delivery aiding capping peptides (HTAPP-DACP), their respective corresponding HLA alleles (HTAPs) and related mutated genes, cancer type, amino acid substitution and response in ELISPOT experiments (PaCa pancreatic cancer, LMCa liver metastasis; BMCa bone cancer metastasis, PrCa prostate cancer).**

| SEQ-ID-No.: | HLA Tumor Antigen Polypeptide (HTAPP-DACP) | rel. Gene(s) | corresponding HLA allele (HTAPs) | Substitution AA | Elispot [nₛₚₒₜₛ/pM] | Corr. carcinoma |
|---|---|---|---|---|---|---|
| 18 | KLVVVGAVGVGKSALTIRQIKIWFQNRRMKWKK | KRAS | A*02:786; C*03:04; C*06:02 | G12V | 447,5 | PaCa |
| 19 | LANSPDLSHLAYYVTKVKEEDAFRQIKIWFQNRRMKWKK | ADAM8, NUP50 | C*06:02; B*57:37 | A399S...E351 K | 430,5 | PaCa |
| 20 | LQEFRRFQDPGRIVAIVRQIKIWFQNRRMKWKK | CEMIP2 | DQA1*01:01; DQB1*02:01 | L301R | 500,0 | PaCa |
| 21 | ETNFKSLFHDLFQKVEERQIKIWFQNRRMKWKK | SMC4 | A*02:01; DQA1*01:01-DQB1 *05:01 | V576F | 334,0 | BMCa/PrCa |
| 22 | IKPRILGFDTPHYWLCRQIKIWFQNRRMKWKK | KDM3A | DRB1*15:01; DQA1*01:01; DQB1 *05:01 | S936R | 477,0 | BMCa/PrCa |
| 23 | MLFSNITPKAAYSPLTGGNMAFRQIKIWFQNRRMKWKK | TNC, LAMC1 | A*03:01; B*07:02; B*35:01 | T1170M..V164M | 285,0 | BMCa/PrCa |
| 24 | RERRVASWAMSFERLRQIKIWFQNRRMKWKK | RGS12 | C*07:02; DQA1*01:01-DQB1 *05:01 | | 436,0 | BMCa/PrCa |
| 25 | RQRLALEAEKAAYEERGAAEALSVVRQIKIWFQNRRMKWKK | EXT2-CACNA2D1; TMPRSS2-ERG | A*03:01; B*35:01 | - Fusion | 317,5 | PrCa |
| 26 | IPDEYGNTALHYAIYNEDKLMRQIKIWFQNRRMKWKK | POTEG, POTEH | B*07:02; B*35:01; C*04:01; DQA1*01:01-DQB1*05:01; DQA1*01:01-DQB1*06:02 | - (TAA/CTA) | 347,5 | PrCa |
| 27 | LFKDLGLPARTVSTTFRQIKIWFQNRRMKWKK | DIP2A | B*07:02; B*35:01; DRB1*01:01 | E33Q | 196,0 | BMCa/PrCa |
| 28 | SPRQCSSWTLAAYKLAAFPTSLRQIKIWFQNRRMKWKK | TMEFF2-TMEFF2 | A*02:01; B*07:02 | TAA/CTA | 320,0 | PrCa |
| 29 | HRCATITHLLAAYYLVLNFNKKRQIKIWFQNRRMKWKK | ABLIM1; PLD3 | A*03:01; C*07:02 | R54C...P471L | 342,5 | PrCa |
| 30 | KDYSVLYVVPGPVRFQRQIKIWFQNRRMKWKK | TMEFF2 | B*35:01, C*07:02; DRB1*15:01 | TAA/CTA | 234,0 | BMCa/PrCa |
| 31 | QSYPGSASLAAYSGMPRISKLRQIKIWFQNRRMKWK | DXL1, FOLH1 | E*01:01; E*01:03 | - (TAA/CTA) | 415,0 | PrCa |

**Table 3: A list of preferred delivery aiding capping peptides (DACP).**

| ID.: | SEQ-ID-No.: | Amino Acid Sequence | designation | % of R | % of K | % R + K |
|---|---|---|---|---|---|---|
| DACP-1 | 123 | -RQIKIWFQNRRMKWKK | Penetratin | 18.75% | 25.00% | 43,75% |
| DACP-2 | 124 | -RKKRRQRRR | TAT | 66.67% | 22.22% | 88,89% |
| DACP-3 | 125 | -GRRRRRRRRRPPQ | R9-TAT | 69.23% | 0.00% | 69,23% |
| DACP-4 | 126 | -RKKRRRESRKKRRRES | DVP3 | 50.00% | 25.00% | 75,00% |
| DACP-5 | 127 | -GRPRESGKKRKRKRLKP | DVP6 | 29.41% | 29.41% | 58,82% |
| DACP-6 | 128 | -RKQIKIWFQNRRMKKKWI | - | 22.22% | 22.22% | 44,44% |
| DACP-7 | 129 | -RQFWIWQNRKWIKIK | - | 21.43% | 14.29% | 35,72% |
| DACP-8 | 130 | -RQIKVWFQRKWIKK | - | 21.43% | 21.43% | 42,86% |
| DACP-9 | 131 | -KIQRKIWFQNRRMKVKWKI | - | 21.05% | 21.05% | 42,10% |
| DACP-10 | 132 | -RKIIWFQNRMKWIK | - | 21.43% | 14.29% | 35,72% |
| DACP-11 | 133 | -IKVRQWFQNRRMIKWK | - | 23.53% | 11.76% | 35,29% |
| DACP-12 | 134 | -RQIKIWFQNRKWKK | - | 14.29% | 21.43% | 35,72% |
| DACP-13 | 135 | -RQIKIWFQNRRMKIKW | - | 21.05% | 15.79% | 36,84% |
| DACP-14 | 136 | -RKQIKVWFQNRRMKWK | - | 25.00% | 12.50% | 37,50% |
| DACP-15 | 137 | -NRRMRQIKIWFQKWKIK | - | 17.65% | 23.53% | 41,18% |
| DACP-16 | 138 | -RQIKIWFVNRKMWKQRI | - | 23.53% | 11.76% | 35,29% |
| DACP-17 | 139 | -QRIKVWFQRKWKIKK | - | 20.00% | 20.00% | 40,00% |
| DACP-18 | 140 | -RQIKIWFRMKWKIK | - | 20.00% | 13.33% | 33,33% |
| DACP-19 | 141 | -QRIKFWIVNRKMKQRIKIW | - | 21.05% | 21.05% | 42,10% |
| DACP-20 | 142 | -IRQKIWFQNRKMVK | - | 21.43% | 14.29% | 35,72% |
| DACP-21 | 143 | -RQIKFWIQNRIKWKWK | - | 18.75% | 25.00% | 43,75% |
| DACP-22 | 144 | -KIRQIWFQNRRMKWKRFKI | - | 22.22% | 22.22% | 44,44% |
| DACP-23 | 145 | -RQIKIQWFNRRWVKKMWIK | - | 21.05% | 21.05% | 42,10% |
| DACP-24 | 146 | -RIQKKIWFQNRKWIKM | - | 23.53% | 11.76% | 35,29% |
| DACP-25 | 147 | -QRKIKIWFQNRRMKVWKIKI | - | 22.22% | 22.22% | 44,44% |
| DACP-26 | 148 | -AAYRRRRRRRRRR | - | 76.92% | 0.00% | 76.92% |
| DACP-27 | 149 | -AAY-Cyclic(RRRRRRRRRR) | - | 76.92% | 0.00% | 76.92% |
| DACP-28 | 150 | -AAYR-Cyclic(RRRRRRRRR) | - | 76.92% | 0.00% | 76.92% |
| DACP-29 | 151 | -AAYRRRRRRRRR | - | 75.0% | 0.00% | 75.0% |
| DACP-30 | 152 | -AAY-Cyclic(RRRRRRRRR) | - | 75.0% | 0.00% | 75.0% |
| DACP-31 | 153 | -AAYR-Cyclic(RRRRRRRR) | - | 75.0% | 0.00% | 75.0% |

**Table 4. List of preferred HLA tumor antigen polypeptides with delivery aiding capping peptides (HTAPP-DACP), preferably related to group therapy, their respective corresponding HLA alleles (HTAPs) and mutated genes exclusive for and/or associated with cancer and response in ELISPOT experiments.**

| SEQ-ID-No.: | HLA Tumor Antigen Polypeptide (HTAPP-DACP) | corresponding HLA allele (HTAPs) | Elispot [nₛₚₒₜₛ/pM] | rel. Gene(s) |
|---|---|---|---|---|
| 32 | AAKALMSAFYTFRYPLSLRQIKIWFQNRRMKWKK | B*15:01; A*24:02; A*01:01; A*11:01; A*03:01; A*02:01; C*07:02 | 75,8 | MMP11 |
| 33 | ATYLWWVNNQSLPVSPRAAYLLLTASLLTFWNPRQIKIWFQNRR MKWKK | A*02:01; B*15:01; A*01:01; A*11:01 | 271,7 | CEACAM5 |
| 34 | DSTPPPGTRVLAMAIYKQSQRQIKIWFQNRRMKWKK | A*11:01; A*30:01; A*03:01; B*07:02; A*31:01; A*68:01; A*30:02 | 35,8 | TP53 |
| 35 | EYFMKQMNDARHGGWTTKMDRQIKIWFQNRRMKWKK | A*33:01; A*31:01; A*68:01 | 20,0 | TP53 |
| 36 | EYKLVVVGAVGVGKSALTIQRQIKIWFQNRRMKWKK | A*02:03; A*68:02; A*11:01; A*02:06; A*02:01; A*03:01; A*68:01 | 140 | PIK3CA |
| 37 | GACRHGDRCSRLHNKPTFSRQIKIWFQNRRMKWKK | A*30:01; B*57:01 | 47,5 | KRAS |
| 38 | - | - | - | - |
| 39 | - | - | - | - |
| 40 | HTLYGHTSTVCCMHLHEKRVRQIKIWFQNRRMKWKK | A*02:03; A*11:01; A*68:02; A*68:01; A*03:01 | 95,0 | FBXW7 |
| 41 | HTLYGHTSTVHCMHLHEKRVRQIKIWFQNRRMKWKK | A*02:03; A*68:02; A*11:01; A*68:01; A*30:01; A*03:01; A*31:01; B*58:01 | 39,2 | FBXW7 |
| 42 | ICLTSTVQLIMQLMPFGCLLRQIKIWFQNRRMKWKK | B*15:01; B*58:01; A*02:06; A*68:02; A*02:01; A*32:01; A*02:03; A*26:01; B*57:01; A*23:01 | 20,8 | EGFR |
| 43 | IGHKVDAVFMKDGFFYFFHGTRQYRQIKIWFQNRRMKWKK | A*01:01; B*15:01; A*24:02; A*03:01; A*24:02; A*11:01; A*02:01; C07:02 | 134,2 | MMP1 |
| 44 | KAISTRDPLSKITEQEKDFLWRQIKIWFQNRRMKWKK | A*03:01; B*58:01; A*30:01; A*11:01; B*57:01 | 103,3 | PIK3CA |
| 45 | KIGDFGLATEKSRWSGSHQRQIKIWFQNRRMKWKK | B*58:01; A*03:01; B*57:01; A*33:01; A*11:01 | 55,0 | BRAF |
| 46 | KLVVVGACGVGKSALTIQLIQNHRQIKIWFQNRRMKWKK | A*02:03; A*68:02; A*11:01; A*02:06; A*02:01; A*03:01; A*68:01 | 54,2 | KRAS |
| 47 | KLVVVGADGVGKSALTIQLIQNHRQIKIWFQNRRMKWKK | A*02:03; A*68:02; A*11:01; A*02:06; A*02:01; A*03:01; A*68:01 | 120,0 | KRAS |
| 48 | KVKIPVAIKTSPKANKEIRQIKIWFQNRRMKWKK | A*03:01; A*11:01; A*30:01; A*68:01 | 39,2 | EGFR |
| 49 | LLDILDTAGHEEYSAMRDQRQIKIWFQNRRMKWKK | A*02:03; A*11:01; A*68:01; A*02:01 | 161,7 | KRAS |
| 50 | LLGRNSFEVCVCACPGRDRRQIKIWFQNRRMKWKK | A*68:01; A*68:02; A*33:01 | 256,7 | TP53 |
| 51 | LLGRNSFEVHVCACPGRDRRQIKIWFQNRRMKWKK | A*68:01; A*68:02; A*33:01; A*30:01; A*31:01 | 264,2 | TP53 |
| 52 | LLGRNSFEVLVCACPGRDRRQIKIWFQNRRMKWKK | A*68:01; A*68:02; A*33:01; A*02:06 | 275,0 | TP53 |

**Table 5. List of preferred HLA tumor antigen polypeptides with delivery aiding capping peptides (HTAPP-DACP), preferably related to group therapy, their respective corresponding HLA alleles (HTAPs) and mutated genes exclusive for and/or associated with cancer and response in ELISPOT experiments.**

| SEQ-ID-No.: | HLA Tumor Antigen Polypeptide (HTAPP-DACP) | corresponding HLA allele (HTAPs) | Elispot [nₛₚₒₜₛ/pM] | rel. Gene(s) |
|---|---|---|---|---|
| 53 | MAQYLSTLLLLLATLAVALRQIKIWFQNRRMKWKK | A*02:01; A*24:02; B*15:01; B*40:01; C*07:02 | 190,8 | CST1 |
| 54 | MLPQIPFLLLVSLNLVHGVFRQIKIWFQNRRMKWKK | A*01:01; B*15:01; A*03:01; A*11:01; A*02:01; B*07:02; A*24:02 | 246,7 | COL10A1 |
| 55 | MTEYKLVVVGARGVGKSALRQIKIWFQNRRMKWKK | A*02:03; A*68:02; A*11:01; A*02:06; A*02:01; A*03:01; A*68:01 | 44,2 | KRAS |
| 56 | MTEYKLVVVGASGVGKSALTIQLIQNHRQIKIWFQNRRMKWKK | A*02:03; A*68:02; A*11:01; A*02:06; A*02:01; A*03:01; A*68:01 | 56,7 | KRAS |
| 57 | NSSCMGGMNQRPILTIITLRQIKIWFQNRRMKWKK | A*68:01; A*31:01; A*11:01; B*15:01 | 102,5 | TP53 |
| 58 | NSSCMGGMNWRPILTIITLRQIKIWFQNRRMKWKK | A*68:01; B*58:01; B*57:01; A*31:01; A*11:01; A*02:01; A*32:01; A*23:01; A*30:01; A*02:03 | 228,3 | TP53 |
| 59 | PSDQDLLRCCVLTSGIFETRQIKIWFQNRRMKWKK | A*02:03; B*08:01; A*30:01 | 100,8 | GNAS |
| 60 | PSDQDLLRCHVLTSGIFETRQIKIWFQNRRMKWKK | A*02:03; B*08:01; A*30:01 | 163,3 | GNAS |
| 61 | QHVKITDFGRAKLLGAEEKRQIKIWFQNRRMKWKK | A*68:01; A*31:01; A*30:01; A*33:01; A*11:01; A*03:01; B*08:01 | 76,7 | EGFR |
| 62 | RVCACPGRDWRTEEENLRKRQIKIWFQNRRMKWKK | B*58:01; B*57:01; A*33:01; A*31:01; A*68:01 | 279,2 | TP53 |
| 63 | SQHMTEVVRHCPHHERCSDRQIKIWFQNRRMKWKK | A*31:01; A*68:01; A*33:01 | 88,3 | TP53 |
| 64 | STRDPLSEITKQEKDFLWSHRQIKIWFQNRRMKWKK | B*58:01; B*57:01; A*30:01; B*44:02; A*11:01 | 80,8 | PIK3CA |
| 65 | TEYKLVVVGAAGVGKSALTIQRQIKlWFQNRRMKWKK | A*02:03; A*68:02; A*11:01; A*02:06; A*02:01; A*03:01; A*68:01 | 22,5 | KRAS |
| 66 | TIHYNYMCNSSCMGSMNRRPILTIRQIKIWFQNRRMKWKK | A*68:01; A*31:01; A*11:01; A*02:03; B*08:01; A*30:01 | 13,3 | TP53 |
| 67 | YGIPFIETSTKTRQRVEDARQIKIWFQNRRMKWKK | A*02:03; A*11:01; A*68:01; A*02:01 | 248,3 | KRAS |
| 68 | YKLVVVGAGDVGKSALTIQLIQNHFVDRQ IKIWFQNRRMKWKK | A*02:03; A*11:01; A*68:01; A*02:01 | 154,2 | KRAS |

**Table 6. List of further preferred HTAPPs and HTAPP-DACPs of the invention and sequences related to polypeptides of the invention (ref.), their respective corresponding HLA alleles (HTAPs) and mutated genes, their respective related gene name, cancer type and response in ELISPOT experiments (PaCa pancreatic cancer, LMCa liver metastasis; BMCa bone cancer metastasis, PrCa prostate cancer).**

| SEQ-ID-No.: | HLA Tumor Antigen Polypeptide | rel. Gene(s) | corresponding HLA allele (HTAPs) | Elispot [nₛₚₒₜₛ/pM] | Corr. carcinoma |
|---|---|---|---|---|---|
| 69 | VAFLGCPGFAGVLQT | D2HGDH | DRB1*01:01; DRB1*15:01 | 149,0 | BMCa/PrCa |
| 70 (ref.) | ESGCEERGAAMIQTVDPAAHRQIKIWFQNRRMKWKK | TMPRSS2-ERG | NONE | 153,5 | BMCa/PrCa |
| 71 (ref.) | QRQLNTALPQPFREAPAYSNRQIKIWFQNRRMKWKK | PPFIA1-SHANK2 | B*35:01 | 270,5 | BMCa/PrCa |
| 72 | MLFSNITPK (ref for SEQ ID No.: 23) | TNC | A*03:01 | 148,0 | BMCa/PrCa |
| 73 | LLFVMESFLLSSALTLCAR | OPA1 | A*02:01; DRB1*01:01 | - | BMCa/PrCa |
| 74 (ref.) | SP**I**TSVLSADLFPKA | GRB14 | DRB1*01:01 | 128,5 | BMCa/PrCa |
| 75 (ref.) | SAANIRALN**V**PPSLA | PCM1 | DRB1*01:01 | 196,0 | BMCa/PrCa |
| 76 | PQELW**H**FLTQNLSLPN | ABCA2 | DRB1*15:01; DQA1*01:01-DQB1*05:01 | 470,5 | BMCa/PrCa |
| 77 (ref.) | KKIRKPRTIYSSLQLQA (ref for SEQ ID No.: 8) | DLX1 | B*07:02 | 148,0 | BMCa/PrCa |
| 78 (ref.) | GIYDALFDIESKVDPSK (ref for SEQ ID No.: 8) | FOLH1 | A*02:01 | 134,5 | BMCa/PrCa |
| 79 | AIYNEDKLMAKALLLYGADIERQIKIWFQNRRMKWKK | POTEG | A*03:01; B*07:02; DRB1*15:01; DRB1*01:01; DQA1*01:02-DQB1*06:02; DQA1 *01:01-DQB1*06:02; DQA1*01:02-DQB1*05:01 | 242,5 | PrCa |
| 80 | NFMLNQNVMLSVKGITKNVHTRQIKIWFQNRRMKWKK | TDRD1 | A*02:01; A*03:01; DQA1*01:02-DQB1*05:01; DQA1*01:02-DQB1*06:02; DRB1*15:01; DRB1*01:01; | 232,5 | PrCa |
| 81 (ref.) | LTPKKLQCVDLHVISNDRQIKIWFQNRRMKWKK | KLK3 | A*0201 | 272,5 | PrCa |
| 82 | LEKLYLYFNKISKIENLEKLIKRQIKIWFQNRRMKWKK | LRRC9 | A*02:01; A*03:01; DRB1*15:01; DRB1*01:01; DQA1*01:01-DQB1*05:01 | 305,0 | PrCa |
| 83 | QSYPGSASLAAYSGMPRISKLRQIKIWFQNRRMKWKK | DLX1, FOLH1 | E*01:01; E*01:03 | 367,5 | PrCa |
| 84 | PPSNATAETQPIPQKAAYLEAIFLRDWDSLYSHDLDTSADS | LUZP2, PLD3 | DQA1*01:01-DQB1*05:01 | 197,5 | PrCa |

**Table 7. List of exemplary first (Seg-1), second segments (Seg-2), corresponding to HTAPs, and third segments (Seg-3), corresponding to DACPs, of click chemistry HTAPPs, which are related to preferred HTAPPs and HTAPP-DACPs (rel. SEQ-ID. No) of the invention.**

| SEQ-ID-No.: | Sequence first segment (Seg-1) | SEQ-ID-No.: | Sequence second segment (Seg-2) | Sequence third segment (Seg-3) | rel. SEQ. ID- No.: |
|---|---|---|---|---|---|
| 85 | LANSPDLSHL-R¹ | 86 | R²-VTKVKEEDAF | - | 2 |
| 87 | NVGLSAVCTYNLSTA-R¹ | 88 | R²-CIHSINLHNFSNSVL | - | 6 |
| 89 | MLFSNITPK-R¹ | 90 | R²-SPLTGGNMAF | - | 7 |
| 91 | KKIRKPRTIYSSLQLQA-R¹ | 92 | R²-GIYDALFDIESKVDPSK | - | 8 |
| 93 | LSKNLVAQISALALQL-R¹ | 94 | R²-GNSITGIISSVLGHIS | - | 13 |
| 95 | HRCATITHLL-R¹ | 96 | R²-YLVLNFNKK | - | 15 |
| 97 | QSYPGSASL-R¹ | 98 | R²-SGMPRISKL | - | 17 |
| 99 | ANSPDLSHL-R¹ | 100 | R²-VTKVKEEDAFRQIKIWFQNRRMKWKK | - | 19 |
| 101 | ATYLWWVNNQSLPVSPR-R¹ | 102 | R²-LLLTASLLTFWNP-R³ | R⁴-RQIKIWFQNRRMKWKK (DACP-1) | 33 |
| 103 | QSYPGSASL-R¹ | 104 | R²-SGMPRISKL-R³ | R⁴-RQIKIWFQNRRMKWKK (DACP-1) | 83 |
| 105 | PPSNATAETQPIPQK-R¹ | 106 | R²-LEAIFLRDWDSLYSHDLDTSADS | - | 84 |
| 107 | MLFSNITPK-R¹ | 108 | R²-SPLTGGNMAF-R³ | R⁴-RQIKIWFQNRRMKWKK (DACP-1) | 23 |
| 109 | RQRLALEAEK-R¹ | 110 | R²-EERGAAEALSVVRQIKIWFQNRRMKWKK | - | 25 |
| 111 | QSYPGSASL-R¹ | 112 | R²-SGMPRISKLRQIKIWFQNRRMKWKK | - | 83 |
| 113 | PPSNATAETQPIPQK-R¹ | 114 | R²-LEAIFLRDWDSLYSHDLDTSADS | - | 84 |
| 115 | QSYPGSASLAAYSGMPRISKL-R¹ | 116 | R²-RQIKIWFQNRRMKWK | - | 31 |
| 117 | LANSPDLSHL-R¹ | 118 | R²-VTKVKEEDAF-R³ | R⁴-GlyP-(CO-(CH₂)₁₆CH₃)₂ | 2 |
| 119 | R¹-NVGLSAVCTYNLSTA-R³ | 120 | R⁴-CIHSINLHNFSNSVL | R²-CO-(CH₂)₂₀CH₃ | 6 |
| 121 | R³-MLFSNITPK-R¹ | 122 | R²-SPLTGGNMAF | R⁴-CO-(CH₂)₁₀CH₃ | 7 |

### Example 1: Transcriptome analysis [mRNA expression].

Transcriptome analysis (sequencing) was performed in all subjects using PANTHER chip analysis (44K chip) from Agilent Technologies. The 44K chip used contains 44,000 gene probes, so that the activity of >34,000 gene expression markers could be analyzed with this chip per subject in each case. In each case, 1,000 genes with increased relevance were determined 10-fold. The evaluation is based on published gene signatures known to the expert.

### Example 2: EXOM sequencing

Exome sequencing is performed from the standard frozen specimen of the subjects from whom the tumor DNA is extracted. Next-generation sequencing of the exome is performed from the tumor sample with coverage of more than 95 % of the entire human coding exon region. This includes selectively amplifying >290,000 relevant segments of DNA and sequencing them by semiconductor sequencing technology.

The aim of the analysis is to define possible mutations for the design of an individual tumor antigen peptide immunization. To exclude non-tumor associated variants (SNPs, Single Nucleotide Polymorphism) of the germline, DNA is simultaneously isolated from nucleated blood cells of the subjects and comparatively sequenced using the same methodology. The differentiation of potential neoantigen candidates was performed in the following six steps:
1) Selection of tumor/somatic mutations is reduced by excluding non-tumor specific polymorphisms.
2) The predefined selection of somatic mutations is further restricted to quality parameters, read coverage and influence on the protein sequence. More complex mutations than single amino acid substitutions SNPs, and mutations in non-protein coding regions are excluded. Silent mutations are also excluded.
3) The mutations defined in this way are expanded to flanking 20s oligopeptides using the known protein sequences (RefGene database). From these, 12 x 9 oligopeptides each were formed sequentially and the affinity to the pre-known, subject-specific hypervariable HLA-I paratopes was determined (program NetMHC-4.0). Nonapeptides with high affinity to the paratopes, which show a further gain in affinity compared to the wild type sequence due to the mutation (SNP), are selected.
4) Candidates carrying this mutation as a germline mutation in an exome pool at the same position are excluded (NIH-NHLBI 6500 exome database version-2 program ANNOVAR-Tool2.).
5) Based on the results of the expression analysis (see findings under embodiment 1), peptides with a low expression relevance in the tumor can be excluded.
6) Finally, we will examine in detail whether further polymorphisms may be present in the vicinity of the single base exchange and thus represent further, subject-specific deviations from the hg19 defined genome sequence.

Below is an example of the evaluation of the results of the described mutation analysis and filtration steps for one subject:
1) Comparison of tumor DNA versus normal DNA
   a. The single analysis "Exome_single_sample_Somatic" using the lonReporter results in:
      - 37494 variants in normal DNA, and
      - 37299 variants in tumor DNA.
   b. The AmpliSeq Exome tumor-normal pair analysis detects and annotates rare somatic variants (SNPs, InDels, CNVs) using statistical analysis in the Ion AmpliSeq Exome Panel (Ion Reporter Software 4.6. Workflow Version: 1.0). This results in: 1477 mutations in tumor with concurrent wild type categorization in normal DNA.
   These 1477 variants are the starting point for further restrictions.
2) Restriction of variants to protein-coding SNP (single nucleotide polymorphisms) with amino acid substitution.
   - Restriction to SNPs
   - At least 50x sequenced in tumor and min. 20x sequenced in blood
   - Must not have occurred in the blood in any single run
   - Must be positioned in an exon
   - Must result in amino acid exchange (missense mutation).
3) From these variants, 20s peptides are defined and fed to the HLA paratope affinity analysis NetMHC.
   ~25*12 nonapeptide pairs (each mutant and wild type) are submitted to affinity analysis for analysis in 9 HLA loci each. Out of these 5814 affinities (2907 pairs) are analyzed. In 40 pairs, an -affinity at least 2-fold -higher in the mutant peptide than in the wild-type peptide is detected.
   Summary of variants with increased HLA affinity
4) Candidates that carry this mutation as a germline mutation at the same position in an exome pool (NIH-NHLBI 6500 exome database version-2) are excluded (Program ANNOVAR - Tool3.).
   Database filters used: hg19_esp6500siv2_all
5) Selection from remaining 25 variants with significant mRNA expression (> 3 fold versus normal tissue) in alignment with transcriptome

### Example 3: Subject with pancreatic cancer and liver metastasis

### 1) Introduction

Pancreatic ductal adenocarcinoma (PDAC) remains a highly lethal cancer with limited treatment options and poor prognosis. Despite advances in chemotherapy, the overall survival rate remains low. The immunotherapy landscape for PDAC is challenging due to the immunologically "cold" tumor microenvironment.

### 2) Clinical history of the subject

- Subject, male (53 years)
- Initial clinical findings: Pancreatic ductal adenocarcinoma (PDAC) with liver metastasis; Stage T2N0M1; Diagnosed in April 2021.

### Initial clinical findings:

- moderately differentiated Pancreatic ductal adenocarcinoma (PDAC) with liver metastasis; Stage T2N0M1; Diagnosed in April 2021, size: 24, 83 mm (Figure 8D)

### Treatment history and methods:

- Initial diagnosis: Received 8 cycles of neo-adjuvant chemotherapy with FOLFIRNOX.
- Surgery: Total pancreatectomy and removal of four liver metastases in September 2021.
- Post-surgery: Received 4 cycles of adjuvant chemotherapy with FOLFIRNOX.
- Disease recurrence: Developed 2 liver metastases during chemotherapy.
- Change in chemotherapy regimen: Treated with Gemcitabine and Erlotinib from March 2022 to September 2022, followed by Onivyde from October 2022.
- Increased CA19-9 levels and CT scan confirmation of liver metastases during chemotherapy.
- Immunization with an pharmaceutical composition PaCa-PC1 in accordance with the current invention, comprising HLA tumor antigen peptides and HLA tumor antigen polypeptides started in November 2022

### 3) Method for determining the pharmaceutical composition

After classic therapy/treatment procedures had been carried out without success, the immunological therapy approach of informing the subject's own immune system by means of application of tumor-associated and tumor-specific HLA tumor antigen peptides arranged on HLA tumor antigen polypeptides in the form of synthesized peptides was used as part of a curative trial.

The focus was on the cellular immune defense, i.e. the activation of the endogenous cytotoxic CD8+T cells, which as I T cells are able to recognize virtually all conceivable pathogenic as well as malignant amino acid sequences (the so-called "targets") by means of a highly differentiated receptor system and thus develop into effector cells. These effector T cells can destroy the tumor cells recognized via the HLA-present antigens by secreting granzyme and perforin.

The procedure described below corresponds to the procedure for determining a pharmaceutical composition containing HLA tumor antigen polypeptides from tissue samples of subjects with carcinoma or suspected carcinoma obtained without surgical intervention. Collectively, the samples are sequenced to generate a transcriptome (b). Bioinformatic methods are then used to pre-select HLA tumor antigen peptides that match MHC class I and class II complexes based on the transcriptome data, taking into account factors such as mRNA regulation, subject HLA subtypes, tumor-related characteristics, and presentation on tumor cells (c). This process involves a bioinformatics feedback loop (d) that includes sequence weighting, peptide alignment, and assembly of an HLA tumor antigen polypeptide composition with iterative validation and refinement steps and a final bioanalytical validation step I.

In the first step, corresponding to step a) of the method of the invention, formalin-fixed paraffin-embedded (FFPE) tissue samples were used for whole exome and transcriptome sequencing. In that respect, after tissue sectioning and evaluation by pathologist, DNA and RNA were isolated from FFPE tumor blocks with at least 40% tumor cells (MagMax FFPE DNA/RNA Ultra; Thermo Fisher). DNA from PBMCs in peripheral blood was isolated (QIA Symphony DSP DNA Mini Kit 96, Qiagen) and considered as normal tissue. Additionally, for the liquid biopsy, three 10-mL peripheral blood samples were collected in Streck cell-free DNA tubes and used for cfDNA isolation.

According to step b) of the method, the data collected from biopsied tumor tissue of the subjects via "next generation sequencing" (NGS) and "liquid chromatography-coupled tandem mass spectrometry" (LC-MS/MS) was further analyzed to determine:
- the mRNA expression of all coding gene regions (transcriptome),
- the tumor-specific somatic miss-sense mutations as well as via (Whole Exome Sequencing WES)
- the HLA-restricted ligands of the HLA classes I+II presented by the tumor cells (HLA ligandome)

Library preparation for WES was performed using Twist Human Core Exome kit with RefSeq and Mitochondrial Panel (Twist Bioscience). Library preparation for RNA-seq was carried out by SMART-Seq Stranded Kit (Takara). The quality controls were performed by fluorescence-based quantification method and fragment length analysis. The libraries were sequenced on a NovaSeq 6000 using 2x100 bp paired end reads. The DNA sequencing resulted in about 320 million mapped reads, and RNA sequencing resulted in 94 million reads.

The following HLA class I and class II alleles were determined:
- HLA I Class Alleles: A*02:786, A*24:02, B*40:221, B*57:37, C*03:04, C*06:02;
- HLA Class II alleles: DPB1*02:01, DPB1*04:02, DQA1*01:31, DQA1*05:40, DQB1*06:04, DRB1*03:01, DRB1*13:275

According to step c) of the method according to the present invention, from the transcriptome determined in step b) using bioinformatic methods factors were determined, such as
▪ significant (>3-fold) deviation of the expression values, and further
▪ up- and/or down-regulated mRNA,
▪ with regard to the significance of the genes concerned for tumor development
▪ according to the criterion that these genes may not be expressed or only slightly expressed in other tissue types
▪ association with proliferation, invasiveness, angiogenesis and an increase in cytokeratin production
▪ Furthermore, with regard to the group of cancer-testis antigens, which are generally not expressed in healthy mammary tissues of adults, notable expressions in the tumor were recorded, since these may have the quality of a tumor-specific antigen

This resulted in a separate dataset of possible HLA tumor antigen peptides, which were weighted using algorithms, databases, and neural networks according to their expression abnormalities/deviations from normal and their known importance for tumor proliferation - > factor 3 compared to normal; importance for tumor development: growth factors, angiogenesis factors, metastasis factors.

The somatic miss-sense mutations (single nucleotide variants (SNV) with an exchange of one amino acid as well as frame-shift mutations) were combined in a further data set, since they are potentially significant HLA tumor antigen peptides and thus highly tumor-specific.

Then, the HLA-restricted ligands (amino acid sequences of 9-10 AS (corresponding to MHC class I complexes) (number:1433) and sequences of 11-17 AS (corresponding to MHC class II complexes) (number: 835) were checked by means of information from specialized databases and correlated with neural networks and algorithms for
▪ already appeared in healthy tissue (=negative)
▪ in protein match with promising sequences from transcriptome and exome, whether sequences of the identified tumor-associated HLA tumor antigen peptides are associated with proliferation, invasiveness, angiogenesis, and/or an increase in cytokeratin production of carcinoma.

In parallel to these described tumor tissue examinations, the genetic haplotype, the alleles of the HLA tumor antigen peptides corresponding to the subject's MHC class I complexes were determined. These factors were included as parameters.

From the data sets of the transcriptome, amino acid sequences of the respective proteins were first used to select amino acid sequences of the HLA tumor antigen peptides corresponding to the MHC complexes (nonamers) with the highest allele affinities (specific activity towards T cell receptor [nM]). This selection criterion is used to algorithmically determine the probability with which the respective HLA tumor antigen peptide is presented in *vivo* on the corresponding MHC complexes (a first prerequisite for a possible cellular immune response).

From the data sets of the mutation tests, nonameric variants involving amino acid exchange were determined with respect to the highest affinities (specific activity towards T cell receptor [nM]) based on the alleles of the subject. Also, polymers of 17 amino acids (oligopeptides) were determined under the affinity criterion for this purpose. This selection criterion algorithmically determines the probability with which the respective tumor antigen peptide corresponding to the MHC complexes is presented in *vivo* on the corresponding MHC complexes (a second prerequisite for a possible cellular immune response).

Based on the new datasets, with the addition of the HLA-restricted ligands (the dataset of the ligandome), a pre-selection according to step c) of the bioinformatic method according to the current invention was made of HLA tumor antigen peptides corresponding to MHC class I complexes and corresponding to MHC class II complexes that were the most promising epitope candidates for eliciting a cellular immune response, both individually and, most importantly, in combination.

The pre-selection together with the weighting factors, database values and algorithmically determined MHC class I and II binding affinity score, biochemical behavior, stability and solubility together with conformational data, presentation score on tumor cell line, overexpression score (preferably at least factor 3 with respect to healthy cells), proliferation score, invasiveness score, angiogenesis score and an increase in cytokeratin production score; distribution factor in a group of subjects with the same tumor type, preferably the same tumor cell line; point mutation score, sequence identity score, robustness score, exome part score according to step i) of the bioinformatic feedback loop pipeline according to the method of the invention, in particular step d), used to weight the obtained HLA tumor antigen peptides and prepare them for step ii). Matching at least two HLA antigen peptides with high scores associated with tumor antigens, the subject-specific result yielded the following assignment of HLA antigen peptides matching class I and II alleles corresponding to genes with high scores as determined by databases, algorithms and neural networks, which were arranged on 3 HLA antigen polypeptides. These were then manually extended with delivery aiding capping peptides (DACP) to demonstrate the in vivo capabilities of this delivering system in accordance with the current invention. The sequences include the following HLA tumor anti-peptides corresponding to HLA alleles:
▪ HLA Class I Alleles: A02:786, C03:04, C06:02, B57:37
▪ HLA Class II Alleles: DQA1 *01:01, DQB1*02:01

**Table E3-1: The PaCa-PC1 pharmaceutical composition comprising 3 HLA tumor antigen polypeptides HTAPP and 3 HTAPP-DACPs for the treatment of pancreas carcinoma with liver metastasis**

| No. | Amino acid sequence | Identification number | Class |
|---|---|---|---|
| E3-1 | KLVVVGAVGVGKSALTI | SEQ-ID-No.: 1 | HTAPP |
| E3-2 | KLVVVGAVGVGKSALTIRQIKIWFQNRRMKWKK | SEQ-ID-No.: 18 | HTAPP-DACP |
| E3-3 | LANSPDLSHLAYYVTKVKEEDAF | SEQ-ID-No.: 2 | HTAPP |
| E3-4 | LANSPDLSHLAYYVTKVKEEDAFRQIKIWFQNRRMKWKK | SEQ-ID-No.: 19 | HTAPP-DACP |
| E3-5 | LQEFRRFQDPGRIVAIV | SEQ-ID-No.: 3 | HTAPP |
| E3-6 | LQEFRRFQDPGRIVAIVRQIKIWFQNRRMKWKK | SEQ-ID-No.: 20 | HTAPP-DACP |

The composition comprising the sequences 1 to 6 were fed back once in step (i), and the result was checked for autoimmune reactions and other factors associated with the individual subject according to step iii) of step d) of the invention in accordance with the current invention. The peptides of Table **E3-1** were then prepared by standard procedures in accordance with GMP standards of peptide synthesis known to those skilled in the art, in this case via an Fmoc (9-fluorenylmethoxycarbonyl) SPPS (solid-phase peptide synthesis) with reversed-phase high-performance liquid chromatography (RP-HPLC) and HPLC HRMS control of the peptides obtained.

### 4) Individualized Immune Information Therapy: Peptide Synthesis and Delivery Solution

Step (a): the pharmaceutical composition (PaCa-PC1) comprising the six synthetic HLA tumor antigen polypeptides and XS15 as adjuvants were synthesized as chemical peptides,
Step (b): the following 6 HLA tumor antigen polypeptides were mixed in 25 % DMSO/H₂O application solution and divided multiple vial units of 1 ml each, Dose per HLA tumor antigen polypeptide: 300 µg

### 5) Individualized Immune Information Therapy: Application Protocol

Application: The PrCa-PC1 immunization polypeptide composition is emulsified in Montanide ISA 51 VG on the bedside prior to injection.

Application Site: Injections are applied subcutaneously (s.c.) at 2-4 locations, including the left and right upper arms and/or abdomen.

Administration Schedule: Over two months, five PrCa-PC1 prime injections are given every two weeks, spanning from November 2022 to January 2023.

Adjuvants Added Per Application: Approximately 20-30 minutes before injection, 1300 µg of nivolumab (Opdivo) is administered subcutaneously next to the injection site. Following the injection, 250 mg of imiquimod (Aldara) is applied to the skin at the injection sites.

### 6) Demonstration of Immunogenicity of each HTAPP and Analysis of T-cell Responses Using ExVivo Elispot Assay:

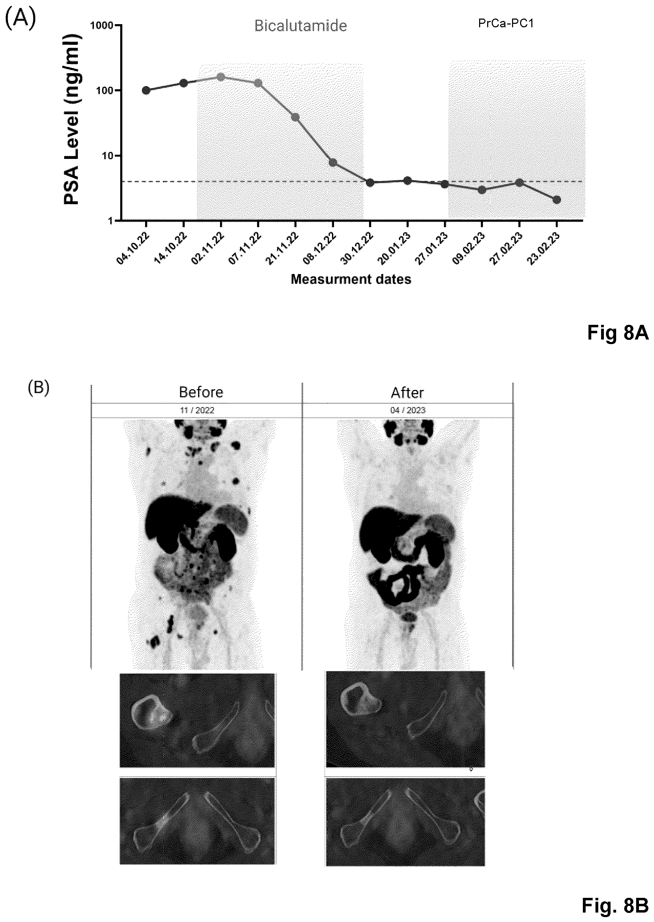

The immunogenicity of each of the six peptides was analyzed by IFN-γ secretion by subject peripheral blood mononuclear cells (PBMCs) using ELIspot assay. As shown in Figure 8A, the number of IFN-γ positive spots shows the subject's strong response to 4 out of 6 HLA tumor antigen polypeptides (HTAPP). In 2 out of 3 HTAPPs, addition of a DACP showed an increase in the response rate (SEQ-ID-No.: 18 and SEQ-ID-No.: 20 compared to SEQ-ID-No.: 1 and SEQ-ID-No.: 5, respectively)

### 7) Evidence of clinical effects and immunogenicity of the pharmaceutical composition:

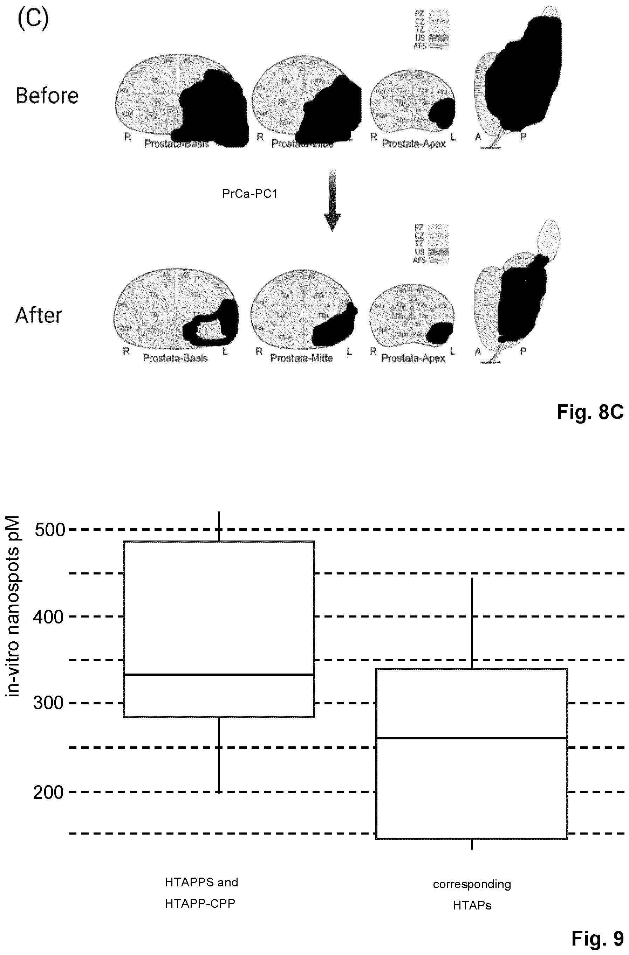

Based on the following 3 significant examples, the clinical efficacy of the applied immunotherapy in the presented case can be shown:
This subject serves as a case study demonstrating the clear benefit of the pharmaceutical composition comprising HLA tumor antigen polypeptides. After administration of five doses of the pharmaceutical composition during the initial immunization phase, concurrent with chemotherapy, the subject showed clear positive results during subsequent follow-up examinations. As shown in Figure 8B and 8C, the use of immunotherapy based on HLA tumor antigen polypeptides in combination with a novel chemotherapeutic agent (Onivyde) resulted in a rapid decrease in tumor markers CA19-9 and CEA during the first treatment phase. In addition, magnetic resonance imaging (MRI) scans performed before and approximately three months after immunotherapy showed a remarkable reduction in the size of the liver lesion from 25 mm to 22 mm, as shown in Figure 8D. The subject remains alive and received the first booster injection two months after the last initial injection. Interestingly, CA19-9 tumor marker levels decreased again after the first booster injection and stabilized (Figure 8B), albeit with a concomitant increase in CEA levels (Figure 8C). Subsequent MRI scans performed approximately three months apart showed further regression of the liver lesion from 22 mm to 14 mm compared with the previous examination (Figure 8D). During follow-up, the subject showed positive indicators of improved health, including a significant reduction in cancer-related symptoms, increased energy levels, improved quality of life, and positive outcomes resulting from the initiation of immunization.

During some rounds of injections, the subject experienced mild local reactions at the injection sites, including redness, swelling, pain, and mild muscle tremors. The muscle tremors usually lasted about 15 minutes after the injections. In addition, the subject reported the appearance of red granulomas at the injection sites days after the injections, which later became discolored and darker, often to a black or bluish-black hue. It is worth mentioning that the subject did not document any systemic symptoms or side effects, indicating that the treatment had an overall positive effect on the subject's well-being.

### Example 4: Subject with prostate cancer and metastatic bone cancer

### 1) Introduction

In Western countries, prostate cancer (PrCa) is the most commonly diagnosed cancer and a leading cause of cancer-related deaths in men. In recent years, many advances have been made in the treatment of metastatic prostate cancer, and the 5-year survival rate for advanced prostate cancer has improved. Bone metastases develop in most subjects with metastatic castration-resistant prostate cancer. Although targeting bone metastases in prostate cancer leads to improved clinical outcomes, there are currently no effective therapies for these subjects.

Over the past decade, immunotherapy has shown clinical benefit in several tumor types including melanoma, lung cancer, and renal cell carcinoma. Bone metastatic PrCa is considered an "immune-naive" tumor that responds poorly to immune checkpoint therapy (ICT). Numerous clinical trials have been completed or are ongoing to evaluate different immunotherapeutic approaches for subjects with metastatic castration resistant PrCa. This example therefore discloses an individualized peptide-based immunization of a prostate cancer subject with bone metastases with a pharmaceutical composition according to the current invention as only therapy. It is shown that a single composition had an effect on two different tumor types (bone and pancreas) which shared only one common mutation.

### 2) Clinical history of the subject

- Subject: A 72-year-old Caucasian male
- Date of Admission: October 04, 2022
- Complaints: Urinary urgency, interrupted urination, nocturia, and erectile dysfunction

### Initial clinical findings:

- PSA Level: 99.77 ng/mL (See Fig. 9A)
- MRI Examination: Moderately enlarged prostate with extensive, organ-crossing, and infiltrating prostate cancer on the left side.
- PSMA-PET Examination: Involvement of the middle gland and apex on the left periphery, infiltrating the left seminal vesicles. No PSMA positive lymph node metastases found, but sclerotic bone metastases reported (See Fig. 9B).
- First Biopsies: Taken from bone metastases in the area of the lateral mass of the left sacrum. Pathological assessment confirmed malignant prostatic origin.

### Prostate Primary Tumor Assessment:

- Second Biopsies: Taken from the prostate primary tumor and no-tumor region.
- Pathological Assessment: Revealed Gleason pattern of 4+4, indicating a Gleason score of 8 adenocarcinoma.

### Treatment history and methods:

- Initiation of Androgen Receptor Inhibitor Therapy (Bicalutamide): Started on October 20th, resulting in a significant PSA decline to normal levels (See Fig. 9A).
- Discontinuation of Bicalutamide: After 70 Days due to side effects.

### Immunization with pharmaceutical composition PrCa-PC1:

- Start Date: Approximately 3 weeks after stopping bicalutamide.
- Two Months After PrCa-PC1: PSMA-PET examination showed regression of multiple bone metastases and minimal residual viability (See Fig. 9B).
- Five Months After PrCa-PC1: MRI analysis indicated a clear regression of 40% in the primary tumor mass (See Fig. 9C).
- Immunization with a pharmaceutical composition in accordance with the current invention, comprising HLA tumor antigen peptides arranged on HLA tumor antigen polypeptides started in November 2022

### 3) Method for determining the pharmaceutical composition related to the bone metastasis cancer (PrCa-PC1) and prostate cancer (PrCa-PC2)

After the hormone therapy had been abandoned due to unbearable side effects, the immunological therapy approach of informing the subject's own immune system by means of application of tumor-associated and tumor-specific HLA tumor antigen peptides arranged on HLA tumor antigen polypeptides in the form of synthesized peptides was used as part of a curative trial. Two compositions, PrCa-PC1(targeting bone metastasis cancer related to prostate cancer) and PrCa-PC2 (follow up targeting explicitly the prostate cancer) were devised using the method for determining a composition in accordance with the current invention.

The focus was on the cellular immune defense, i.e. the activation of the endogenous cytotoxic CD8+T cells, which as naive T cells are able to recognize virtually all conceivable pathogenic as well as malignant amino acid sequences (the so-called "targets") by means of a highly differentiated receptor system and thus develop into effector cells. These effector T cells can destroy the tumor cells recognized via the HLA-present antigens by secreting granzyme and perforin.

The procedure described below corresponds to the procedure for determining a pharmaceutical composition containing HLA tumor antigen polypeptides from tissue samples of subjects with carcinoma or suspected carcinoma obtained without surgical intervention. Collectively, the samples are sequenced to generate a transcriptome (b). Bioinformatic methods are then used to pre-select HLA tumor antigen peptides that match MHC class I and class II complexes based on the transcriptome data, taking into account factors such as mRNA regulation, subject HLA subtypes, tumor-related characteristics, and presentation on tumor cells (c). This process involves a bioinformatics feedback loop (d) that includes sequence weighting, peptide alignment, and assembly of an HLA tumor antigen polypeptide composition with iterative validation and refinement steps and a final bioanalytical validation step (e).

In the first step, corresponding to step a) of the determination method of the invention, fresh frozen tissue samples from bone metastases, primary tumor and non-tumor inflammatory regions of the prostate were used for molecular profiling by NGS. The metastatic tumor tissue and the primary tumor and non-tumor inflammatory regions of the prostate were used for the preparation of the first and second pharmaceutical compositions (PrCa-PC1 and PrCa-PC2), respectively. In this context, DNA and RNA were isolated from biopsy tissue samples extracted and evaluated by a clinical pathologist (AllPrep DNA/RNA Mini Kit; Qiagen). DNA from EDTA blood samples was isolated (QIA Symphony DSP DNA Mini Kit 96, Qiagen) and considered normal tissue in subsequent analysis. Library preparation for WES was performed using the Twist Human Core Exome Kit with RefSeq and Mitochondrial Panel (Twist Bioscience). Library preparation for RNA-seq was performed using the KAPA RNA HyperPrep Kit with RiboErase Globin (Roche). Quality controls were performed using fluorescence-based quantification method and fragment length analysis. Libraries were sequenced on a NovaSeq 6000 with 2x100 bp paired-end reads.

According to step b) of the method, the data collected from biopsied tumor tissue of the subjects via "next generation sequencing" (NGS) and "liquid chromatography-coupled tandem mass spectrometry" (LC-MS/MS) was further analyzed to determine:
▪ the mRNA expression of all coding gene regions (transcriptome),
▪ the tumor-specific somatic miss-sense mutations as well as via (Whole Exome Sequencing WES)
▪ the HLA-restricted ligands of the HLA classes I+II presented by the tumor cells (HLA ligandome)

The following HLA class I and class II alleles were determined:
▪ HLA Class I Alleles: A*03:01, A*02:01, B*07:02, B*35:01, C*04:01,C*07:02
▪ HLA Class II Alleles: PB1*04:01, DQA1*01:01, DQA1*01:02, DQB1*05:01, DQB1*06:02, DRB1*15:01, DRB1*01:01

Library preparation for WES was performed using Twist Human Core Exome kit with RefSeq and Mitochondrial Panel (Twist Bioscience). Library preparation for RNA-seq was carried out by SMART-Seq Stranded Kit (Takara). The quality controls were performed by fluorescence-based quantification method and fragment length analysis. The libraries were sequenced on a NovaSeq 6000 using 2x100 bp paired end reads. The DNA sequencing resulted in about 330 million mapped reads, and RNA sequencing resulted in 97 million reads.

According to step c) of the method according to the present invention, from the transcriptome determined in step b) using bioinformatic methods factors were determined, such as
▪ significant (>3-fold) deviation of the expression values, and further
▪ up- and/or down-regulated mRNA,
▪ with regard to the significance of the genes concerned for tumor development
▪ according to the criterion that these genes may not be expressed or only slightly expressed in other tissue types
▪ association with proliferation, invasiveness, angiogenesis and an increase in cytokeratin production
▪ Furthermore, with regard to the group of cancer-testis antigens, which are generally not expressed in healthy mammary tissues of adults, notable expressions in the tumor were recorded, since these may have the quality of a tumor-specific antigen

This resulted in a separate dataset of possible HLA tumor antigen peptides, which were weighted using algorithms, databases, and neural networks according to their expression abnormalities/deviations from normal and their known importance for tumor proliferation - > factor 3 compared to normal; importance for tumor development: growth factors, angiogenesis factors, metastasis factors.

The somatic miss-sense mutations (single nucleotide variants (SNV) with an exchange of one amino acid as well as frame-shift mutations) were combined in a further data set, since they are potentially significant HLA tumor antigen peptides and thus highly tumor-specific.

Then, the HLA-restricted ligands (amino acid sequences of 9-10 AS (corresponding to MHC class I complexes) (number:1253) and sequences of 11-17 AS (corresponding to MHC class II complexes) (number: 795) were checked by means of information from specialized databases and correlated with neural networks and algorithms for
▪ already appeared in healthy tissue (=negative)
▪ in protein match with promising sequences from transcriptome and exome, whether sequences of the identified tumor-associated HLA tumor antigen peptides are associated with proliferation, invasiveness, angiogenesis, and/or an increase in cytokeratin production of carcinoma.

In parallel to these described tumor tissue examinations, the genetic haplotype, the alleles of the HLA tumor antigen peptides corresponding to the subject's MHC class I complexes were determined. These factors were included as parameters.

From the data sets of the transcriptome, amino acid sequences of the respective proteins were first used to select amino acid sequences of the HLA tumor antigen peptides corresponding to the MHC complexes (nonamers) with the highest allele affinities (specific activity towards T cell receptor [nM]). This selection criterion is used to algorithmically determine the probability with which the respective HLA tumor antigen peptide is presented in *vivo* on the corresponding MHC complexes (a first prerequisite for a possible cellular immune response).

From the data sets of the mutation tests, nonameric variants involving amino acid exchange were determined with respect to the highest affinities (specific activity towards T cell receptor [nM]) based on the alleles of the subject. Also, polymers of 17 amino acids (oligopeptides) were determined under the affinity criterion for this purpose. This selection criterion algorithmically determines the probability with which the respective tumor antigen peptide corresponding to the MHC complexes is presented in *vivo* on the corresponding MHC complexes (a second prerequisite for a possible cellular immune response).

Based on the new datasets, with the addition of the HLA-restricted ligands (the dataset of the ligandome), a pre-selection according to step c) of the bioinformatic method according to the current invention was made of HLA tumor antigen peptides corresponding to MHC class I complexes and corresponding to MHC class II complexes that were the most promising epitope candidates for eliciting a cellular immune response, both individually and, most importantly, in combination.

The pre-selection together with the weighting factors, database values and algorithmically determined MHC class I and II binding affinity score, biochemical behavior, stability and solubility together with conformational data, presentation score on tumor cell line, overexpression score (preferably at least factor 3 with respect to healthy cells), proliferation score, invasiveness score, angiogenesis score and an increase in cytokeratin production score; distribution factor in a group of subjects with the same tumor type, preferably the same tumor cell line; point mutation score, sequence identity score, robustness score, exome part score according to step i) of the bioinformatic feedback loop pipeline according to the method of the invention, in particular step d), used to weight the obtained HLA tumor antigen peptides and prepare them for step ii). Matching at least two HLA antigen peptides with high scores associated with tumor antigens, the subject-specific result yielded the following assignment of HLA antigen peptides matching class I and II alleles corresponding to genes with high scores as determined by databases, algorithms and neural networks, which were arranged on 17 HLA antigen polypeptides (HTAPPs). A selection of those were then manually extended with delivery aiding capping peptides (DACP) to demonstrate the in vivo capabilities of this delivering system in accordance with the current invention (see table E4-1 for details). The sequences include the following HLA tumor antigen peptides corresponding to HLA alleles:
▪ HLA Class I Alleles: A*02:01, A*03:01, B*07:02, B*35:01, C*07:02
▪ HLA Class II Alleles: DQA1*01:01, DQB1 *05:01, DRB1*01:01, DRB1*15:01

**Table E4-1: Sequences of the 25 HLA tumor antigen polypeptides HTAPP and HTAPP-DACPs for the treatment of prostate metastatic bone cancer carcinoma**

| No. | Amino acid sequence | SEQ-ID-No.: | Class |
|---|---|---|---|
| E4-1 | ETNFKSLFHDLFQKVEERQIKIWFQNRRMKWKK | 21 | HTAPP-DACP |
| E4-2 | IKPRILGFDTPHYWLCRQIKIWFQNRRMKWKK | 22 | HTAPP-DACP |
| E4-3 | NVGLSAVCTYNLSTAAAYCIHSINLHNFSNSVL | 6 | HTAPP |
| E4-4 | MLFSNITPKAAYSPLTGGNMAFRQIKIWFQNRRMKWKK | 23 | HTAPP-DACP |
| E4-5 | LSKNLVAQISALALQLAAYGNSITGIISSVLGHIS | 13 | HTAPP |
| E4-6 | KKIRKPRTIYSSLQLQAAAYGIYDALFDIESKVDPSK | 8 | HTAPP |
| E4-7 | ESGCEERGAAMIQTVDPAAHRQIKIWFQNRRMKWKK | 70 | HTAPP-DACP |
| E4-8 | QRQLNTALPQPFREAPAYSNRQIKIWFQNRRMKWKK | 71 | HTAPP-DACP |
| E4-9 | KDYSVLYVVPGPVRFQRQIKIWFQNRRMKWKK | 30 | HTAPP-DACP |
| E4-10 | LPVMLLIVARPVKLAAFPTSLSD | 9 | HTAPP |
| E4-11 | LFKDLGLPARTVSTTFRQIKIWFQNRRMKWKK | 27 | HTAPP-DACP |
| E4-13 | RERRVASWAMSFERLRQIKIWFQNRRMKWKK | 24 | HTAPP-DACP |
| E4-14 | ETNFKSLFHDLFQKVEE | 4 | HTAPP |
| E4-15 | IKPRILGFDTPHYWLC | 5 | HTAPP |
| E4-16 | LFKDLGLPARTVSTTF | 10 | HTAPP |
| E4-17 | RERRVASWAMSFERL | 16 | HTAPP |
| E4-18 | VAFLGCPGFAGVLQT | 69 | HTAPP |
| E4-19 | SPITSVLSADLFPKA | 74 | HTAPP |
| E4-20 | SAANIRALNVPPSLA | 75 | HTAPP |
| E4-21 | PQELWHFLTQNLSLPN | 76 | HTAPP |
| E4-22 | MLFSNITPK | 72 | HTAPP |
| E4-23 | SPLTGGNMAF | 11 | HTAPP |
| E4-24 | KKIRKPRTIYSSLQLQA | 77 | HTAPP |
| E4-25 | GIYDALFDIESKVDPSK | 78 | HTAPP |
| E4-26 | SPRQCSSWTLAAYKLAAFPTSL | 14 | HTAPP |

The composition comprising the sequences E4-1 to E4-26 were fed back once in step (i), and the result was checked for autoimmune reactions and other factors associated with the individual subject according to step iii) of step d) of the invention in accordance with the current invention. The peptides of table E4-1 were then prepared by standard procedures in accordance with GMP standards of peptide synthesis known to those skilled in the art, in this case via an Fmoc (9-fluorenylmethoxycarbonyl) SPPS (solid-phase peptide synthesis) with reversed-phase high-performance liquid chromatography (RP-HPLC) and HPLC HRMS control of the peptides obtained.

**Table E4-2: Sequences of the 12 HLA tumor antigen polypeptides HTAPP and HTAPP-DACPs for the treatment of prostate carcinoma**

| No. | Amino acid sequence | SEQ-ID-No.: | Class |
|---|---|---|---|
| E4-27 | RQRLALEAEKAAYEERGAAEALSVVRQIKIWFQNRRMKWKK | 25 | HTAPP-DACP |
| E4-28 | IPDEYGNTALHYAIYNEDKLMRQIKIWFQNRRMKWKK | 26 | HTAPP-DACP |
| E4-29 | AIYNEDKLMAKALLLYGADIERQIKIWFQNRRMKWKK | 79 | HTAPP-DACP |
| E4-30 | NFMLNQNVMLSVKGITKNVHTRQIKIWFQNRRMKWKK | 80 | HTAPP-DACP |
| E4-31 | LTPKKLQCVDLHVISNDRQIKIWFQNRRMKWKK | 81 | HTAPP-DACP |
| E4-32 | LEKLYLYFNKISKIENLEKLIKRQIKIWFQNRRMKWKK | 82 | HTAPP-DACP |
| E4-33 | QSYPGSASLAAYSGMPRISKLRQIKIWFQNRRMKWKK | 83 | HTAPP-DACP |
| E4-34 | SPRQCSSWTLAAYKLAAFPTSLRQIKIWFQNRRMKWKK | 28 | HTAPP-DACP |
| E4-35 | HRCATITHLLAAYYLVLNFNKKRQIKIWFQNRRMKWKK | 29 | HTAPP-DACP |
| E4-36 | PPSNATAETQPIPQKAAYLEAIFLRDWDSLYSHDLDTSADS | 84 | HTAPP |
| E4-37 | QSYPGSASLAAYSGMPRISKLKDEL | 17 | HTAPP |
| E4-38 | QSYPGSASLAAYSGMPRISKLRQIKIWFQNRRMKWKKKDEL | 31 | HTAPP-DACP |

The composition comprising the sequences E4-27 to E4-38 were fed back once in step (i), and the result was checked for autoimmune reactions and other factors associated with the individual subject according to step iii) of step d) of the invention in accordance with the current invention. The peptides of table E4-2 were then prepared by standard procedures in accordance with GMP standards of peptide synthesis known to those skilled in the art, in this case via an Fmoc (9-fluorenylmethoxycarbonyl) SPPS (solid-phase peptide synthesis) with reversed-phase high-performance liquid chromatography (RP-HPLC) and HPLC HRMS control of the peptides obtained.

According to the step e) of the method of the pharmaceutical composition determination, the immunogenicity of the sequences were determined with EliSpot assays with PMC and two final peptide pools (PrCa-PC1 and PrCa-PC2)were assembled.

**Table E4-3: PrCa-PC1 pharmaceutical composition comprising 12 HTAPP and HTAPP-DACP peptides for the treatment of bone metastasis and related prostate cancer**

| No. | Amino acid sequence | SEQ-ID-No.: | Class |
|---|---|---|---|
| E4-1 | ETNFKSLFHDLFQKVEERQIKIWFQNRRMKWKK | 21 | HTAPP-DACP |
| E4-2 | IKPRILGFDTPHYWLCRQIKIWFQNRRMKWKK | 22 | HTAPP-DACP |
| E4-3 | NVGLSAVCTYNLSTAAAYCIHSINLHNFSNSVL | 6 | HTAPP |
| E4-4 | MLFSNITPKAAYSPLTGGNMAFRQIKIWFQNRRMKWKK | 23 | HTAPP-DACP |
| E4-5 | LSKNLVAQISALALQLAAYGNSITGIISSVLGHIS | 13 | HTAPP |
| E4-6 | KKIRKPRTIYSSLQLQAAAYGIYDALFDIESKVDPSK | 8 | HTAPP |
| E4-7 | ESGCEERGAAMIQTVDPAAHRQIKIWFQNRRMKWKK | 70 | HTAPP-DACP |
| E4-8 | QRQLNTALPQPFREAPAYSNRQIKIWFQNRRMKWKK | 71 | HTAPP-DACP |
| E4-9 | KDYSVLYVVPGPVRFQRQIKIWFQNRRMKWKK | 30 | HTAPP-DACP |
| E4-10 | LPVMLLIVARPVKLAAFPTSLSD | 9 | HTAPP |
| E4-11 | LFKDLGLPARTVSTTFRQIKIWFQNRRMKWKK | 27 | HTAPP-DACP |
| E4-13 | RERRVASWAMSFERLRQIKIWFQNRRMKWKK | 24 | HTAPP-DACP |

**Table E4-4: PrCa-PC2 pharmaceutical composition comprising 7 HTAPP and HTAPP-DACP sequences for the treatment of prostate cancer**

| No. | Amino acid sequence | SEQ-ID-No.: | Class |
|---|---|---|---|
| E4-27 | RQRLALEAEKAAYEERGAAEALSVVRQIKIWFQNRRMKWKK | 25 | HTAPP-DACP |
| E4-28 | IPDEYGNTALHYAIYNEDKLMRQIKIWFQNRRMKWKK | 26 | HTAPP-DACP |
| E4-31 | LTPKKLQCVDLHVISNDRQIKIWFQNRRMKWKK | 81 | HTAPP-DACP |
| E4-34 | SPRQCSSWTLAAYKLAAFPTSLRQIKIWFQNRRMKWKK | 28 | HTAPP-DACP |
| E4-35 | HRCATITHLLAAYYLVLNFNKKRQIKIWFQNRRMKWKK | 29 | HTAPP-DACP |
| E4-36 | PPSNATAETQPIPQKAAYLEAIFLRDWDSLYSHDLDTSADS | 84 | HTAPP |
| E4-38 | QSYPGSASLAAYSGMPRISKLRQIKIWFQNRRMKWKKKDEL | 31 | HTAPP-DACP |

### 4) Individualized Immune Information Therapy: Peptide Synthesis and Delivery Solution

### PrCa-PC1:

Step (a): the pharmaceutical composition comprising the 12 synthetic HLA tumor antigen polypeptides and XS15 as adjuvant were synthesized as chemical peptides,
Step (b): the 12 HLA tumor antigen polypeptides were mixed in 25 % DMSO/H₂O application solution and divided multiple vial units of 1 ml each, Dose per HLA tumor antigen polypeptide: 300 µg

### PrCa-PC2:

Step (a): the pharmaceutical composition comprising the 7 synthetic HLA tumor antigen polypeptides and XS15 as adjuvant were synthesized as chemical peptides,
Step (b): the 7 HLA tumor antigen polypeptides were mixed in 25 % DMSO/H₂O application solution and divided multiple vial units of 1 ml each, Dose per HLA tumor antigen polypeptide: 300 µg

### 5) Individualized Immune Information Therapy: Application Protocol

Application: The PrCa-PC1 and PrCa-PC2 immunization peptide pool is emulsified in Montanide ISA 51 VG on the bedside prior to injection.

Application Site: Injections are applied subcutaneously (s.c.) at 2-4 locations, including the left and right upper arms and/or abdomen.

Administration Schedule: Over six months, six PrCa-PC1 injections are given in increasing intervals, first 3 days, then 7 days, then 27 days, then approx. 3 and 2 months. Two injections PrCa-PC2 were given in 3 days (see Fig. 7). Adjuvants Added Per Application: Approximately 20-30 minutes before injection, 1300 µg of nivolumab (Opdivo) is administered subcutaneously next to the injection site. Following the injection, 250 mg of imiquimod (Aldara) is applied to the skin at the injection sites.

### 6) Demonstration of Immunogenicity of each HTAPP and Analysis of T-cell Responses Using ExVivo Elispot Assay:

The immunogenicity of each peptide was assessed in peripheral blood mononuclear cells (PBMCs) from patients and using the IFN-γ-ELISPOT assay. ELISPOT results were considered positive if the number of spots was at least 2-fold higher than the negative control (medium) and at least 50 spots were detected. Accordingly, 7 of 12 peptides (~58 % positivity) from the injection composition (PrCA-PC1) showed a strong response (Fig. 7A). We also tested the effect of delivery aiding peptides (DACP-1) and peptide extension by overlapping tandemization and tandemization on the immunogenicity of the epitopes. The result shows that the addition of DACP and peptide extension by epitope multimerization showed an increased positive response compared with the originally predicted epitopes (HTAPs) (Fig. 7B). The 7 peptides of the pharmaceutical composition PrCA-PC2 also showed a strong Elispot response (Fig. 8).

### 7) Evidence of clinical effects and immunogenicity of the pharmaceutical composition:

Based on the following significant examples, the clinical efficacy of the applied immunotherapy in the presented case can be shown: The subject received the first dose of PrCa-PC1 approximately 3 weeks after discontinuation of anti-hormone therapy (bicalutamide), see Fig. 9A. Approximately two months after the first dose of PrCa-PC1, the subject's PSMA-PET examination showed significant regression of the multiple bone metastases compared to the previous examination performed prior to the initiation of pharmaceutical compounding treatment. The PSMA-expressing metastases still show low PSMA expression, indicating minimal residual viability. In addition, there is no evidence of new bone metastases (Fig. 9B). Five months after initiation of PrCa-PC1 application, further MRI analysis showed a significant 40% regression of the primary tumor mass, indicating the efficacy of PrCa-PC immunization (Fig. 9C). The patient received multiple initial and booster injections of PrCa-PC1, and then PrCa-PC2 immunization was started. Remarkably, both the primary tumor (prostate) and metastases were affected by the first composition, demonstrating the ability to target different cancers in a patient carrying at least one HLA allele corresponding to a mutation in a gene associated with the parent carcinoma and related metastases.

### Example 5: Determination of HLA antigen peptides as pool for HTAPPs of the invention

According to a bioinformatic method as provided in the present disclosure, a number of tumor-associated and tumor-exclusive HTAPs were determined for the preparation of a pharmaceutical composition providing these HTAPs in the form of HTAPPs and HTAPP-DACPs, according to the present invention for the treatment or prophylaxis of a cancer, wherein a random forest approach was chosen in which a mutant peptides corresponding to different cancer types selected from the literature, corresponding genes and HLA alleles were weighted with weighting factors according to the present invention, in particular MHC class I and II binding affinity scores; affinity percentile value, presentation percentile, pl, processing value, half-life, length, charge (at physiological pH), length, charge molecular weight, instability value, immunogenicity value.

The results of immunogenic peptides according to the current invention according to the model are shown in table 8 and table 9, each representing another specific embodiment of the current invention. The HTAPs might be used in the formulation of a large combinations of pharmaceutical compositions comprising HTAPPs according to the current invention.

The HTAPs (TEA, table 8) have a Hallmark score between 1 and 16, so they are directly linked to important genes of the corresponding cancer lines, which makes adaptations less likely.

### Example 6: Producing exemplary click chemistry HTAPPs and HTAPP-DACPs of the invention

According to this specific example, a number of click chemistry HTAPPs and click chemistry HTAPP-DACPs corresponding to HTAPPs and HTAPP-DACPs of the invention are disclosed. The following table lists the segments with the specific click chemistry groups attached to the optional linker and peptide, each representing a different embodiment of the present invention.

**Table E6-1**

| No. | SEQ-ID-No.: (Seg-1) | R¹- = | SEQ-ID-No.: (Seg-2) | R²- = | R³- = | SEQ-ID-No.: (Seg-3) | R⁴- = | rel. SEQ.-ID- No.: |
|---|---|---|---|---|---|---|---|---|
| E6-1 | 85 | -AAY-Mal | 86 | C-GPGPG- | - | - | - | 2 |
| E6-2 | 87 | -AAY-AzA | 88 | PrG-PEG₃- | - | - | - | 6 |
| E6-3 | 89 | -AAY-TzF | 90 | TCOC- | - | - | - | 7 |
| E6-4 | 91 | -DBCOam | 92 | AzK-AAY- | - | - | - | 8 |
| E6-5 | 93 | -PEG₂-Mal | 94 | CK-AAY- | - | - | - | 13 |
| E6-6 | 95 | -AAY-AzK | 96 | HPrG-AAY- | - | - | - | 15 |
| E6-7 | 97 | -AzP | 98 | PrY-AAY- | - | - | - | 17 |
| E6-8 | 99 | -AAY-Mal | 100 | C- | - | - | - | 19 |
| E6-9 | 101 | -AAY-Mal | 102 | C-PEG₆- | -AAY-TzF | R⁴-DACP-1 | TCOC- | 33 |
| E6-10 | 103 | -AAY-AzA | 104 | DBCOac- | -AAY-Mal | R⁴-DACP-1 | C-PEG₈- | 83 |
| E6-11 | 105 | -AAY-TzF | 106 | TCOC- | | | | 84 |
| E6-12 | 107 | -PEG₂-TzF | 108 | TCOC-AAY- | -DBCOam | R⁴-DACP-1 | AzA-AAY- | 23 |
| E6-13 | 109 | -GPGPG-Mal | 110 | C-AAY- | | | | 25 |
| E6-14 | 111 | -Mal | 112 | C-AAY- | - | - | - | 83 |
| E6-15 | 113 | -AAY-TzF | 114 | TCOC-AAY- | - | - | - | 84 |
| E6-16 | 115 | -AAY-PrG | 116 | AzK- | - | - | - | 31 |
| E6-17 | 117 | -AAY-Mal | 118 | C- | -DBCOam | - | AzA-AAY- | 2 |
| E6-18 | 119 | -Mal | 120 | -AAY-C | -AAY-TzF | - | TCOC- | 6 |
| E6-19 | 121 | -AAY-TzF | 122 | TCOC- | C-AAY- | - | Mal- | 7 |

Through click chemistry reactions under the conditions disclosed herein, the segments were reacted at room temperature in aqueous media and purified by column chromatography.

For the purification of click chemistry peptides according to the provided document, the process involves utilizing a combination of Liquid Chromatography (LC) and Mass Spectrometry (MS) for precise separation and identification. Specifically, the LC setup employs a Thermo Dionex Vanquish Flex UPLC system with Waters T3 UPLC columns of varying particle sizes for optimal separation, coupled with UV/Vis in tandem with HRMS detection for monitoring the elution of peptides. The detection setup included the following instruments with ranges in brackets: TOF (Mass range 0 - 40,000 m/z), Quadrupole (isolation up to 3,000 m/z; mass range up to 40,000 m/z), with amass accuracy for MS and MS/MS 1ppm, a mass resolution of 30,000 FSR and detection rate up to 50 Hz for MS and MS/MS.

The following purification methods were employed:

### Purification Method 1

| | |
|---|---|
| Column: | Waters Atlantis T3 C18 2.7µm 2.1x100mm |
| Detector: | UV 214nm |
| Solvent A: | water + 0.1 %(v/v) trifluoroacetic acid |
| Solvent B: | acetonitrile + 0.1%(v/v) trifluoroacetic acid |
| Flow: | 0.7ml/min |
| Temperature: | 55°C |

### Gradient:

| Time | %B | Flow [ml/min] |
|---|---|---|
| 0 | 5 | 0.7 |
| 0.1 | 5 | 0.7 |
| 9 | 50 | 0.7 |
| 9.2 | 95 | 0.7 |
| 9.6 | 95 | 0.7 |
| 9.8 | 5 | 0.7 |
| 12 | 5 | 0.7 |

### Purification Method 2

| | |
|---|---|
| Column: | Waters Atlantis T3 C18 1.8µm 2.1x100mm |
| Detector: | UV 214nm |
| Solvent A: | water + 0.1 %(v/v) trifluoroacetic acid |
| Solvent B: | acetonitrile + 0.1%(v/v) trifluoroacetic acid # |
| Flow: | 0.48ml/min |
| Temperature: | 55°C |

### Gradient:

| Time | %B | Flow [ml/min] |
|---|---|---|
| 0 | 5 | 0.7 |
| 0.1 | 5 | 0.7 |
| 9 | 50 | 0.7 |
| 9.2 | 95 | 0.7 |
| 9.6 | 95 | 0.7 |
| 9.8 | 5 | 0.7 |
| 12 | 5 | 0.7 |

Following LC, a Bruker Compact ESI-Q-TOF mass spectrometer in tandem with UV/Vis-detectors is used for the accurate mass determination of the peptides and potential contaminants, utilizing its high-resolution mass accuracy capabilities to determine the click chemistry HTAPP capabilities. The peak area of the UV signal is used for relative quantification and purity determination. The identity of the product and any impurities present is determined via the mass.

To ensure the purity of the peptides and to check for possible contamination, such as residual copper catalyst from the click chemistry reaction, an additional step involving Inductively Coupled Plasma Optical Emission Spectrometry (ICP-OES) should be incorporated. This technique is highly sensitive and capable of detecting trace amounts of metals. The procedure would involve preparing the peptide samples according to ICP-OES requirements, typically involving dilution in an appropriate acid matrix, and then analyzing the samples using an ICP-OES instrument calibrated for copper detection. This step is crucial for confirming the absence of heavy metal contaminants to meet the stringent purity criteria for pharmaceutical applications, which were below 5 ppm in all cases.

**Table E6-2**

| No. | Click chemistry HTAPP and click chemistry HTAPP-DACPs | rel. SEQ.-ID-No.: |
|---|---|---|
| E6-1 | LANSPDLSHL-AAY-Mal-C-GPGPG-VTKVKEEDAF | 2 |
| E6-2 | NVGLSAVCTYNLSTA-AAY-AzA-PrG-PEG₃-CIHSINLHNFSNSVL | 6 |
| E6-3 | MLFSNITPK-AAY-TzF-TCOC-SPLTGGNMAF | 7 |
| E6-4 | KKIRKPRTIYSSLQLQA-DBCOam-AzK-AAY-GIYDALFDIESKVDPSK | 8 |
| E6-5 | LSKNLVAQISALALQL-PEG₂-Mal-CK-AAY-GNSITGIISSVLGHIS | 13 |
| E6-6 | HRCATITHLL-AAY-AzK-HPrG-AAY-YLVLNFNKK | 15 |
| E6-7 | QSYPGSASL-AzP-PrY-AAY-SGMPRISKL | 17 |
| E6-8 | ANSPDLSHL-AAY-Mal-C-VTKVKEEDAFRQIKIWFQNRRMKWKK | 19 |
| E6-9 | ATYLWWVNNQSLPVSPR-AAY-Mal–C-PEG6-LLLTASLLTFWNP-AAY-TzF–TCOC- RQIKIWFQNRRMKWKK | 33 |
| E6-10 | QSYPGSASL-AAY-AzA-DBCOac-SGMPRISKL-AAY-Mal-C-PEG_{B}-RQIKIWFQNRRMKWKK | 83 |
| E6-11 | PPSNATAETQPIPQK-AAY-TzF-TCOC-LEAIFLRDWDSLYSHDLDTSADS | 84 |
| E6-12 | MLFSNITPK-PEG₂-TzF-TCOC-AAY-SPLTGGNMAF-DBCOam-AzA-AAY-RQIKIWFQNRRMKWKK | 23 |
| E6-13 | RQRLALEAEK-GPGPG-Mal-C-AAY-EERGAAEALSVVRQIKIWFQNRRMKWKK | 25 |
| E6-14 | QSYPGSASL-Mal-C-AAY-SGMPRISKLRQIKIWFQNRRMKWKK | 83 |
| E6-15 | PPSNATAETQPIPQ K-AAY-TzF-TCOC-AAY-LEAIFLRDWDSLYSHDLDTSADS | 84 |
| E6-16 | QSYPGSASLAAYSGMPRISKL-AAY-PrG-AzK-RQIKIWFQNRRMKWK | 31 |
| E6-17 | LANSPDLSHL-AAY-Mal-C-VTKVKEEDAF-DBCOam-AzA-AAY-GlyP-(CO-(CH₂)₁₆CH₃)₂ | 2 |
| E6-18 | (CH₃(CH₂)₂₀-CO)-AAY-C-Mal-NVGLSAVCTYNLSTA-AAY-TzF-TCOC-CIHSINLHNFSNSVL | 6 |
| E6-19 | (CH₃(CH₂)₁₀-CO)-Mal-C-AAY-MLFSNITPK-AAY-TzF-TCOC-SPLTGGNMAF | 7 |

## Claims

1. A **click chemistry HLA tumor antigen polypeptide** corresponding to at least two HLA tumor antigen peptides (HTAP) corresponding to MHC class I and/or class II complexes, arranged on a HLA tumor antigen polypeptide (HTAPP), wherein the HLA tumor antigen peptides (HTAP) are tumor-exclusive or tumor-associated and presented on the cell membrane of the associated tumor cell and correspond to an amino acid sequence of a transcribed mutant gene, wherein the HLA tumor antigen polypeptide comprises an amino acid sequence,
a) wherein the amino acid sequence comprises, in addition to HLA tumor antigen peptide(s), up to 1 to 30 amino acids (long HLA tumor antigen polypeptide); and/or
b) wherein the amino acid sequence comprises an HLA tumor antigen peptide(s) with at least 90 % sequence identity similarity to the native HLA tumor antigen peptide (similarity HLA tumor antigen polypeptide); and/or
c) wherein the amino acid sequence comprises an HLA tumor antigen peptide having an amino acid sequence comprising of only one amino acid substitution relative to the amino acid sequence of the native HLA tumor antigen peptide (substitution HLA tumor antigen polypeptide),
wherein
i) an HLA tumor antigen polypeptide is a tandem polypeptide comprising at least 2 HLA tumor antigen peptides corresponding to MHC class I and/or class II complexes; and/or
ii) an HLA tumor antigen polypeptide is an overlapping tandem polypeptide comprising at least 2 HLA tumor antigen peptides corresponding to MHC class I and/or class II complexes which overlap in their amino acid sequence,
wherein the HTAPP is provided as a first and a second segment wherein
- the first segment comprises a first click chemistry group bound to the first segment at the C-terminus, and
- the second segment comprises a second click chemistry group bound to the second segment at the N-terminus,
**characterized in that**
the click chemistry HLA tumor antigen polypeptide is provided by a click chemistry reaction.

2. The click chemistry HLA tumor antigen polypeptide according to claim 1, wherein the click chemistry HLA tumor antigen polypeptide corresponds to a HLA tumor antigen polypeptide comprising a delivery aiding capping peptide (DACP), wherein the delivery aiding capping peptide comprises a positively charged and amphipathic sequence with an total percentage of 33 % to 89 % of arginine (R) and lysine (K) residues, wherein the HTAPP-DACP is provided as a first, a second segment, each independently preferably corresponding to HTAPs, and a third segment, corresponding to a DACP, wherein
- the first segment comprises a first click chemistry group bound to the first segment, preferably via a linker group, at the C-terminus, also denoted as R¹, and
- the second segment comprises a second click chemistry group bound to the second segment, preferably via a linker group, at the N-terminus, also denoted as R², as well as a third click chemistry group bound to the second segment, preferably via a linker group, at the C-terminus, also denoted as R³, and
- the third segment comprises a fourth click chemistry group bound to the third segment, preferably via a linker group, at the N-terminus, referred also denoted as R⁴,
wherein the first and the second click chemistry group are antagonistic to each other and the third and the fourth click chemistry group are antagonistic to each other and orthogonal to the first and second click chemistry group, and wherein the click chemistry HLA tumor antigen polypeptide is provided by a click chemistry reaction, and
wherein the DCAP is a cell-penetrating peptide (CPP).

3. The click chemistry HLA tumor antigen polypeptide according to claim 1, wherein the click chemistry HLA tumor antigen polypeptide comprises a chemically bound terminal group, wherein the HTAPP is provided as a first, a second segment, each independently preferably corresponding to HTAPs, and a third segment, corresponding to the terminal group, wherein
- the first segment comprises a first click chemistry group bound to the first segment, preferably via a linker group, at the C-terminus, also denoted as R¹, and
- the second segment comprises a second click chemistry group bound to the second segment, preferably via a linker group, at the N-terminus, also denoted as R², as well as a third click chemistry group bound to the second segment, preferably via a linker group, at the *C*-terminus, also denoted as R³, and
- the third segment comprises a fourth click chemistry group bound to the third segment, preferably via a linker group, at the *N*-terminus, referred also denoted as R⁴,
wherein the first and the second click chemistry group are antagonistic to each other and the third and the fourth click chemistry group are antagonistic to each other and orthogonal to the first and second click chemistry group, and wherein the click chemistry HLA tumor antigen polypeptide is provided by a click chemistry reaction, and wherein the terminal group is a lipide, preferably selected from the group comprising or consisting of saturated fatty acids, modified fatty acids, unsaturated fatty acids and their diacylglycerides.

4. The click chemistry HLA tumor antigen polypeptide according to any claims 1 to 3, wherein the first and second click chemistry group and/or the third and fourth click chemistry group are selected in pairs from the list comprising N-maleimide and cysteine group, azide and alkyne group, tetrazine and alkene group.

5. The click chemistry HLA tumor antigen polypeptide according to any claims 1 to 4, wherein the linker group comprised by the first and/or second segment is selected independently from the list comprising PEGₙ (2 ≤ n ≤ 10), AAY, AYY, GPGPG.

6. A **pharmaceutical composition** as a medicament in the therapeutic and/or prophylactic treatment of a carcinoma, comprising a pharmacologically effective amount comprising 2 to 25 HLA tumor antigen peptides (HTAP) corresponding to MHC class I and/or class II complexes, arranged on at least one click chemistry HLA tumor antigen polypeptide(s) (HTAPP) according to claim 1 to 5.

7. The pharmaceutical composition as a medicament according to claim 6, wherein the tandem polypeptide comprises at least one HLA tumor antigen peptide corresponding to MHC class I complexes and at least one HLA tumor antigen peptide corresponding to MHC class II complexes, and/or wherein the overlapping tandem polypeptide comprises at least one HLA tumor antigen peptide corresponding to MHC class I complexes and at least one HLA tumor antigen peptide corresponding to MHC class II complexes.

8. The pharmaceutical composition as a medicament according to claim 6 or 7, wherein the HLA tumor antigen polypeptides corresponding to HLA tumor antigen peptides corresponding to MHC class I and class II complexes are selected from the group consisting of the amino acid sequences set forth in SEQ-ID-Nos.: 1 to 17, 69 and 76.

9. The pharmaceutical composition as a medicament according to any claims 6 to 8, wherein the HLA tumor antigen polypeptide comprises, in addition to the HLA tumor antigen peptides, an delivery aiding capping peptide (DACP), wherein the DACP is a cell-penetrating peptide (CPP), attached to a terminal part of its amino acid sequence.

10. The pharmaceutical composition as a medicament according to claim 9, wherein the HLA tumor antigen polypeptide (HTAPP) comprising a delivery aiding capping peptide (DACP), wherein the DACP is a cell-penetrating peptide (CPP), has an amino acid sequence length between 30 and 60 amino acids.

11. The pharmaceutical composition as a medicament according to any one of claims 9 or 10, wherein the HLA tumor antigen polypeptide comprises a amphipathic delivery aiding capping peptide (DACP), wherein the DACP is a cell-penetrating peptide (CPP), wherein the delivery aiding capping peptide comprises a positively charged and amphipathic sequence with an total percentage of 33 % to 89 % of arginine (R) and lysine (K) residues.

12. The pharmaceutical composition as a medicament according to any one of claims 6 to 11, wherein the pharmacologically effective amount of each individual HLA tumor antigen polypeptide in the composition in an absolute concentration (i.e., administration dose) ranges from 100 to 1000 µg.

13. The pharmaceutical composition as a medicament according to any one of claims 6 to 12, wherein the pharmaceutical composition is used for the treatment of carcinoma as monotherapy or in combination with other known therapies and/or compounds for the treatment of carcinoma.

14. The pharmaceutical composition as a medicament according to any one of claims 6 to 13, wherein subjects to be treated with the pharmaceutical composition have received a standard therapy procedure (e.g., at least one of surgery, radiation, chemotherapy, and/or hormone therapy).

15. The pharmaceutical composition as a medicament according to any one of claims 6 to 14, wherein the pharmaceutical composition, wherein the pharmaceutical composition is administered to a group of subjects having at least one identical allele, wherein the allele is selected from the list comprising of A*01:01, A*02:01, A*02:03, A*02:06, A*02:786, A*03:01, A*11:01, A*24:02, A*30:01, A*30:02, A*31:01, A*32:01, A*33:01, A*68:01, A*68:02, B*07:02, B*08:01, B*15:01, B*35:01, B*40:01, B*44:02, B*57:01, B*57:37, B*58:01, C*03:04, C*04:01, C*06:02, C*07:02, DQA1*01:01, DQB*102:01, DQB1*05:01, DQB1*06:02, DRB1*01:01, DRB1*15:01, E*01:01, and E*01:03.
